(19) Europäisches Patentamt
European Patent Office
Office européen des brevets

(11) Veröffentlichungsnummer: **0 287 959 B1**

(12) **EUROPÄISCHE PATENTSCHRIFT**

(45) Veröffentlichungstag der Patentschrift: **28.04.93**

(51) Int. Cl.5: **C07D 333/16**, C07D 333/28, C07D 333/32, C07D 307/42, C07D 307/56, C07D 207/333, C07D 207/34, C07D 277/24, C07D 277/32, C07D 277/34, C07D 233/64

(21) Anmeldenummer: **88105990.1**

(22) Anmeldetag: **14.04.88**

(54) **p-Phenoxy-phenoxymethyl-fünfring-heteroaromaten.**

(30) Priorität: **21.04.87 DE 3713337**
**08.02.88 DE 3803703**

(43) Veröffentlichungstag der Anmeldung:
**26.10.88 Patentblatt 88/43**

(45) Bekanntmachung des Hinweises auf die Patenterteilung:
**28.04.93 Patentblatt 93/17**

(84) Benannte Vertragsstaaten:
**AT BE CH DE ES FR GB GR IT LI NL**

(56) Entgegenhaltungen:
**EP-A- 0 086 043**
**EP-A- 0 239 047**
**DE-A- 2 516 331**
**GB-A- 2 124 227**
**JP-A-57 175 177**

(73) Patentinhaber: **BASF Aktiengesellschaft**
**Carl-Bosch-Strasse 38**
**W-6700 Ludwigshafen(DE)**

(72) Erfinder: **Theobald, Hans, Dr.**
**Oueichstrasse 6**
**W-6703 Limburgerhof(DE)**
Erfinder: **Kuenast, Christoph, Dr.**
**Salierstrasse 2**
**W-6701 Otterstadt(DE)**
Erfinder: **Hofmeister, Peter, Dr.**
**Bernard-Humblot-Strasse 12**
**W-6730 Neustadt(DE)**
Erfinder: **Neubauer, Hans-Juergen, Dr.**
**B 4,2**
**W-6800 Mannheim 1(DE)**
Erfinder: **Kuekenhoehner, Thomas, Dr.**
**Seidelstrasse 2**
**W-6710 Frankenthal(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 287 959 B1

**PATENT ABSTRACTS OF JAPAN, Band 7, Nr. 19 (C147)(1164), 25 Januar 1983; & JP-A-57175177 (MITSUI TOATSU KAGUKU K.K.) 28.10.1982**

**PATENT ABSTRACTS OF JAPAN, Band 10, Nr. 230 (C-365)(2286), 9 August 1986; & JP-A-616668 (KAVESHO K.K.) 01.04.1986**

Erfinder: **Krieg, Wolfgang, Dr.**
**Saarstrasse 17**
**W-6721 Weingarten(DE)**
Erfinder: **Leyendecker, Joachim, Dr.**
**Scharnhorststrasse 12**
**W-6800 Mannheim 51(DE)**
Erfinder: **Kardorff, Uwe, Dr.**
**Frankenthaler Strasse 42**
**W-6710 Frankenthal 6(DE)**

**Beschreibung**

Die vorliegende Erfindung betrifft neue (p-Phenoxy-phenoxy)-methyl-fünfringheteroaromaten der allgemeinen Formel I

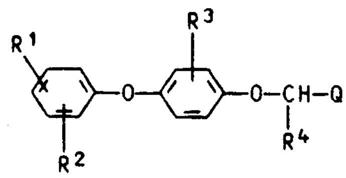

(I),

in der die Substituenten folgende Bedeutung haben:

| | |
|---|---|
| $R^1$, $R^2$, $R^3$ | Wasserstoff, Halogen, $C_1$-$C_8$-Alkyl, $C_1$-$C_8$-Alkoxy, $C_1$-$C_4$-Halogenalkyl, $C_1$-$C_4$-Halogenalkoxy, $C_3$-$C_{10}$-Cycloalkyl, Nitro oder Cyano, |
| $R^4$ | Wasserstoff oder $C_1$-$C_4$-Alkyl, |
| Q | ein Fünfringhetarylrest der Formeln II.1 bis II.33 |

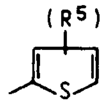 (II.1) 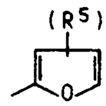 (II.2) 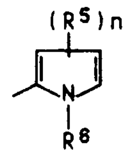 (II.3)

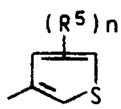 (II.4) 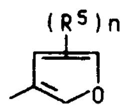 (II.5) 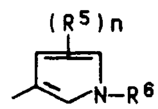 (II.6)

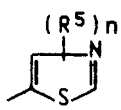 (II.7) 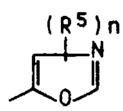 (II.8) 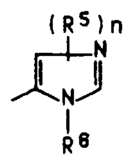 (II.9)

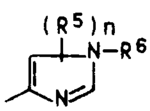 (II.10) 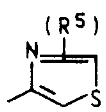 (II.11) 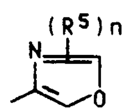 (II.12)

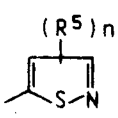 (II.13) 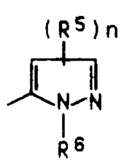 (II.14) 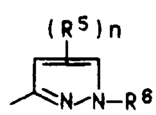 (II.15)

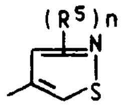 (II.16) 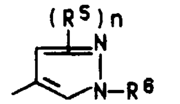 (II.17) 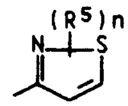 (II.18)

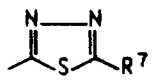 (II.19) 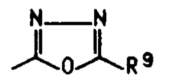 (II.20) 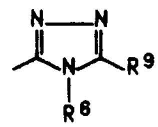 (II.2)

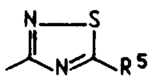 (II.22) 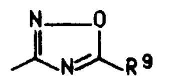 (II.23) 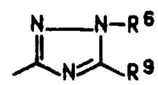 (II.24)

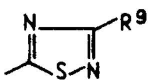 (II.25) 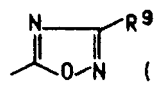 (II.26) 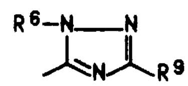 (II.27)

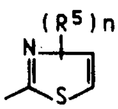 (II.28) 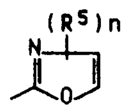 (II.29) 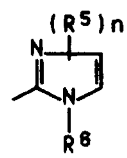 (II.30)

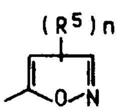 (II.31) 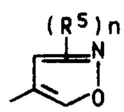 (II.32) 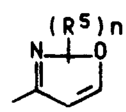 (II.33)

in denen

- $R^5$ Halogen, $C_1$-$C_8$-Alkyl, $C_2$-$C_8$-Alkenyl, $C_1$-$C_4$-Halogenalkyl, $C_1$-$C_8$-Alkoxy, $C_2$-$C_8$-Alkoxyalkyl, $C_3$-$C_{10}$-Cycloalkyl, Aryl, $C_7$-$C_{20}$-Arylalkyl oder im Arylteil ein- bis dreifach durch Halogen, $C_1$-$C_8$-Alkyl, $C_1$-$C_8$-Alkoxy, $C_1$-$C_4$-Halogenalkyl oder $C_1$-$C_4$-Halogenalkoxy oder Cyano oder ein- bis zweifach durch Nitro substituiertes Aryl oder $C_7$-$C_{20}$-Arylalkyl,
- $R^6$ Wasserstoff, $C_1$-$C_8$-Alkyl, $C_3$-$C_{10}$-Cycloalkyl oder Aryl, welches gegebenenfalls ein- bis dreifach durch Halogen, $C_1$-$C_6$-Alkyl, $C_1$-$C_8$-Alkoxy, $C_1$-$C_4$-Halogenalkyl oder $C_1$-$C_3$-Halogenalkoxy substituiert ist,
- $R^7$ Halogen oder $C_3$-$C_{10}$-Cycloalkyl,
- $R^9$ Wasserstoff oder $R^5$ und
- n 0 (unsubstituierter Heterocyclus), 1, gegebenenfalls 2 oder 3

bedeuten, mit der Maßgabe, daß $R^4$ nicht für Methyl steht, wenn Q der Fünfringhetarylrest II.23 oder II.26 ist.

Außerdem betrifft die Erfindung die Herstellung der Verbindungen I, Schädlingsbekämpfungsmittel, die die Verbindungen I als Wirkstoffe enthalten, sowie ein Verfahren zur Bekämpfung von Schädlingen.

Aus DE-C-23 04 962, DE-B-23 22 853, DE-C-24 18 295, DE-A-29 42 989 und JP 80/124 460 sind zahlreiche p-Phenoxy-phenoxyverbindungen bekannt, jedoch enthalten diese Verbindungen keinen Alkylisoxazolrest. Die Wirkung dieser Verbindungen läßt zu wünschen übrig.

(p-Phenoxy-phenoxy)-methylpyridinderivate und (p-Phenoxy-phenoxy)-methylthiadiazolylderivate mit insektizider Wirkung sind aus DE-A-25 16 331 und EP-A-239 047 bekannt. Ferner werden in JP 57/175 179, in JP 57/175 177 und in JP 61/63 668 (p-Phenoxy-phenoxy)-methyl-1,2,4-oxadiazol-5-yl- und -3-yl-Derivate als herbizide Wirkstoffe beschrieben.

Der Erfindung lag daher die Aufgabe zugrunde, neue (p-Phenoxy-phenoxy)-methyl-heteroaromaten mit verbesserter insektizider Wirkung bereitzustellen.

Demgemäß wurden die eingangs definierten neuen (p-Phenoxy-phenoxy)-methyl-fünfringheteroaromaten sowie Verfahren zu deren Herstellung gefunden. Ferner wurde gefunden, daß sich die Verbindungen I hervorragend zur Bekämpfung von Schädlingen eignen.

Die Verbindungen I sind nach folgenden Methoden erhältlich:

a) Die Umsetzung erfolgt zwischen einem (p-Phenoxy)-phenol III und einem Fünfringhetarylderivat IV in Gegenwart einer Base im Temperaturbereich von (-20) bis 250 °C, vorzugsweise von 20 bis 120 °C nach folgender Reaktionsgleichung:

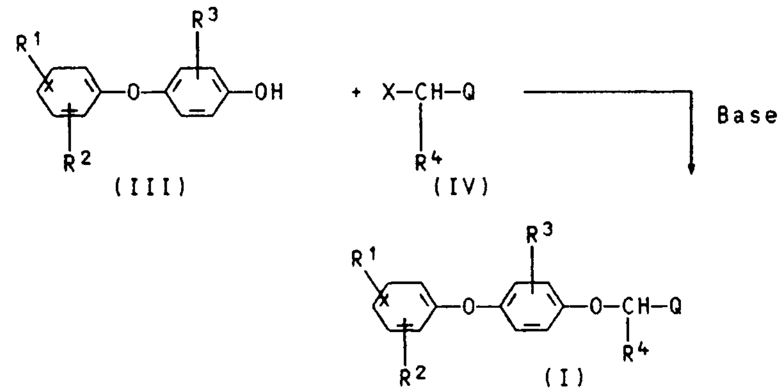


(III) (IV) (I)

Die (p-Phenoxy)-phenole III sind zum Teil aus Houben/Weyl, Bd. VI, 3, Methoden der org. Chemie, Thieme Verlag, 1965, 585 ff bekannt oder können nach den dort beschriebenen Methoden hergestellt werden.

Die Hetarylderivate IV sind entweder bekannt und teilweise kommerziell erhältlich oder lassen sich nach allgemein bekannten chemischen Verfahren herstellen. Verfahren zur Herstellung von Thiophenderivaten (Q = II.1, II.4) finden sich beispielsweise in: Comprehensive Heterocyclic Chemistry, R. Katritzky und W. Rees, Vol. 4, S. 863 ff, Pergamon Press 1984; Furanderivate (Q = II.2, II.5) z.B. DE-A-3 514 384, DE-A-3 546 371 oder Advances in Heterocyclic Chemistry, Vol. 30 S. 167 ff, 1982; Pyrrolderivate (Q = II.3, II.6) z.B. Comprehensive Heterocyclic Chemistry, R. Katritzky und W. Rees, Vol. 4, S. 201 ff, Pergamon Press, 1984; Thiazolderivate (Q = II.7, II.11, II,28), Oxazolderivate (Q = II.8, II.12, II.29), Isothiazolderivate (Q = II.13, II.16, II.18), Thiazolderivate (Q = II.19, II.22, II.25) und Oxadiazolderivate (Q = II.20, II.23, II.26) z.B. Comprehensive Heterocyclic Chemistry, R. Katritzky und W. Rees, Vol. 6, S. 166, 177, 235, 386, 425 ff, Pergamon Press, 1984; Imidazolderivate (Q = II.9, II.10, II.30) z.B. Advances in Heterocyclic Chemistry, Vol. 27, S. 242 ff, 1980; Pyrazolderivate (Q = II.14, II.15, II.17) z.B. Heteroaromatic Nitrogen Compounds, The Azoles, S. 31 ff, Cambridge University Press, 1976; Triazolderivate (Q = II.21, II.24, II.27) z.B. Comprehensive Heterocyclic Chemistry, R. Katritzky und W. Rees, Vol. 5, S. 733 ff, Pergamon Press, 1984; Isoxazolderivate (Q = II.31, II.32, II.33) z.B. DE-A-2 549 962 und DE-A-2 754 832.

Man setzt normalerweise mindestens äquivalente Mengen einer Base, bezogen auf III, zu III und/oder IV zu, kann aber diese auch im Überschuß oder gegebenenfalls auch als Lösungsmittel verwenden. Als Basen eignen sich beispielsweise Hydroxide von Alkali- und Erdalkalimetallen wie Natriumhydroxid, Kaliumhydroxid und Calciumhydroxid. Alkoholate von Alkali- und Erdalkalimetallen wie Natriummethylat, Natriumethylat, Calciummethanolat oder Kalium-tert.-butylat; Alkali- oder Erdalkalimetallhydride wie Natriumhydrid, Kaliumhydrid oder Calciumhydrid; Alkali- oder Erdalkalicarbonate wie Natriumcarbonat, Kaliumcarbonat oder Calciumcarbonat; aliphatische Amine wie Dimethylamin, Triethylamin oder Diisopropylamin; heterocyclische Amine wie Piperidin, Piperazin oder Pyrrolidin; aromatische Amine wie Pyridin oder Pyrrol und gegebenenfalls auch Alkyllithium-Verbindungen wie n-Butyllithium.

Die Umsetzung wird zweckmäßigerweise in einem Lösungsmittel oder Verdünngungsmittel durchgeführt. Hierzu eignen sich beispielsweise aliphatische Kohlenwasserstoffe wie n-Pentan, n-Hexan, das Hexan-Isomerengemisch und Petrolether; aromatische Kohlenwasserstoffe wie Benzol, Toluol, die Xylole und deren Isomerengemische, Benzin; Alkohole wie Methanol, Ethanol, n-Propanol und Isopropanol; Ether wie Diethyl-, Di-n-butylether, Methyl-tert.-butylether, Tetrahydrofuran und Dioxan; Ketone wie Aceton, Methylethylketon und Methylisopropylketon; Nitrile wie Acetonitrile und Propionitril; aprotische dipolare Lösungsmittel wie Dimethylformamid, Dimethylsulfoxid oder Pyridin. Auch Gemische dieser Stoffe können als Lösungs- und Verdünnungsmittel verwendet werden.

b) Die Umsetzung erfolgt zwischen einem Phenolatanion von III und einem Fünfringhetarylderivat IV in einem Temperaturbereich von (-20) bis 120 °C, vorzugsweise von (-20) bis 80 °C nach folgender Reaktionsgleichung:

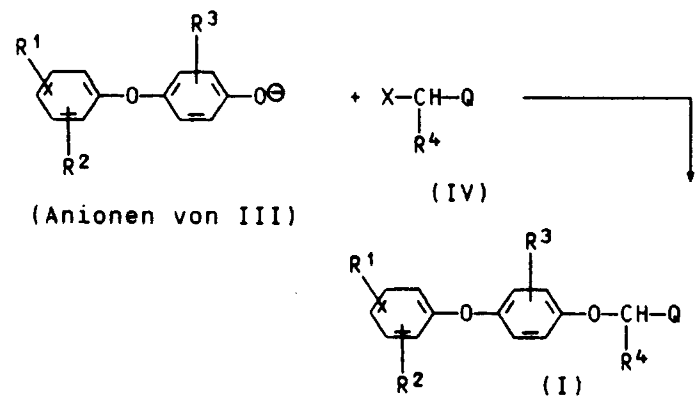

Man arbeitet normalerweise mit äquimolaren Mengen III und IV, kann aber auch im Überschuß der einen oder anderen Komponente arbeiten.

Die Anionen der (p-Phenoxy)-phenole III sind in Form ihrer Metallsalze, wie dem Natrium oder Kaliumsalz bekannt oder lassen sich in situ aus den in Houben/Weyl (s.o.) bekannten oder aus den dort beschriebenen Methoden herstellbaren Phenolen III durch Umsetzung mit gängigen Metallierungsreagentien wie Natriummethylat, Natriumethylat, Kalium-tert.-butylat, Natrium-bzw. Kaliumhydrid oder n-Butyllithium generieren.

Die Umsetzung wird zweckmäßigerweise in einem Lösungsmittel oder Verdünnungsmittel durchgeführt. Hierzu eignen sich beispielsweise aliphatische Kohlenwasserstoffe wie n-Pentan, n-Hexan, das Hexan-Isomerengemisch und Petrolether; halogenierte Kohlenwasserstoffe wie Chlorbenzol, Methylenchlorid, Ethylenchlorid, Chloroform, Tetrachlorethylen; aromatische Kohlenwasserstoffe, wie Benzol, Toluol, die Xylole und deren Isomerengemische und Benzin, Ether wie Diethyl-, Di-n-butylether, Methyl-tert.-butylether, Tetrahydrofuran und Dioxan; Ketone wie Aceton, Methylethylketon und Methylisopropylketon; aprotische dipolare Lösungsmittel wie Dimethylformamid, Dimethylsulfoxid oder Pyridin. Auch Gemische dieser Stoffe können als Lösungs- und Verdünnungsmittel verwendet werden.

In beiden Fallgestaltungen bedeutet der Rest X eine Abgangsgruppe, wie beispielsweise einen Sulfonsäurerest oder ein Halogen. Unter den Sulfonsäureresten sind Methansulfonyl, Trifluormethansulfonyl und p-Toluolsulfonyl, unter den Halogenen sind Chlor und Brom bevorzugt: besonders bevorzugt wird Chlor.

Zur Herstellung der erfindungsgemäßen Verbindungen I nach den vorstehend beschriebenen Methoden setzt man die Ausgangsstoffe üblicherweise in stöchiometrischem Verhältnis ein. Ein Überschuß des einen oder anderen kann in Einzelfällen aber durchaus vorteilhaft sein.

Die Umsetzungen verlaufen gewöhnlich oberhalb von -20°C mit ausreichenden Geschwindigkeiten. 120°C müssen im allgemeinen nicht überschritten werden. Da sie in einigen Fällen unter Wärmeentwicklung verlaufen, kann es von Vorteil sein, Kühlmöglichkeiten vorzusehen.

Die Reaktionsgemische werden in üblicher Weise aufgearbeitet, z.B. durch Versetzen mit Wasser, Trennen der Phasen und Säulenchromatographie. Die neuen Verbindungen der Formel I fallen teilweise in Form farbloser oder schwach bräunlich gefärbter, zäher Öle an, die sich durch längeres Erwärmen unter vermindertem Druck auf mäßig erhöhte Temperatur ("Andestillieren") von den letzten flüchtigen Anteilen befreien und auf diese Weise reinigen lassen. Erhält man die Verbindungen der Formel I in kristalliner Form, so kann ihre Reinigung durch Umkristallisieren erfolgen.

Die Substituenten und der Index in Formel I haben im einzelnen folgende Bedeutungen:

$R^1$

- Wasserstoff,
- Halogen, bevorzugt Fluor, Chlor und Brom, besonders bevorzugt Fluor, Chlor und Brom in meta-Stellung,
- unverzweigtes oder verzweigtes $C_1$-$C_8$-Alkyl, bevorzugt unverzweigtes oder verzweigtes $C_1$-$C_6$-Alkyl, besonders bevorzugt unverzweigtes oder verzweigtes $C_1$-$C_4$-Alkyl in meta-Stellung, wie m-Methyl, M-Ethyl, m-(n-Propyl), m-(iso-Propyl), m-(n-Butyl), m-(iso-Butyl), m-(sec.Butyl) und m-(tert.-Butyl),
- unverzweigtes oder verzweigtes $C_1$-$C_8$-Alkoxy, bevorzugt unverzweigtes oder verzweigtes $C_1$-$C_4$-Alkoxy, besonders bevorzugt $C_1$-$C_2$-Alkoxy in meta-Stellung, wie m-Methoxy und m-Ethoxy,

- unverzweigtes oder verzweigtes $C_1$-$C_4$-Halogenalkyl, bevorzugt $C_1$-$C_2$-Fluor- und Chloralkyl, besonders bevorzugt m-Trifluormethyl und m-Trichlormethyl,
- unverzweigtes oder verzweigtes $C_1$-$C_4$-Halogenalkoxy, bevorzugt $C_1$-$C_2$-Fluor- und Chloralkoxy, besonders bevorzugt m-Trifluormethoxy und m-Trichlormethoxy,
- $C_3$-$C_{10}$-Cycloalkyl, bevorzugt $C_3$-$C_6$-Cycloalkyl, besonders bevorzugt m-Cyclopropyl, m-Cyclobutyl, m-Cyclopentyl und m-Cyclohexyl,
- Nitro
- Cyano,

$R^2$, $R^3$ unabhängig voneinander

- Wasserstoff,
- Halogen, bevorzugt Fluor und Chlor,
- unverzweigtes oder verzweigtes $C_1$-$C_8$-Alkyl, bevorzugt unverzweigtes oder verzweigtes $C_1$-$C_4$-Alkyl, besonders bevorzugt $C_1$-$C_2$-Alkyl, wie Methyl und Ethyl,
- unverzweigtes oder verzweigtes $C_1$-$C_8$-Alkoxy, bevorzugt unverzweigtes oder verzweigtes $C_1$-$C_4$-Alkoxy, besonders bevorzugt $C_1$-$C_2$-Alkoxy, wie Methoxy und Ethoxy,
- unverzweigtes oder verzweigtes $C_1$-$C_4$-Halogenalkyl, bevorzugt $C_1$-$C_2$-Fluor- und Chloralkyl, besonders bevorzugt Trifluormethyl und Trichlormethyl,
- unverzweigtes oder verzweigtes $C_1$-$C_4$-Halogenalkoxy, bevorzugt $C_1$-$C_2$-Fluor- und Chloralkoxy, besonders bevorzugt Trifluormethoxy und Trichormethoxy,
- $C_3$-$C_{10}$-Cycloalkyl, bevorzugt $C_3$-$C_6$-Cycloalkyl, wie Cyclopropyl, Cyclobutyl, Cyclopentyl und Cyclohexyl, besonders bevorzugt Cyclopropyl,
- Nitro,
- Cyano,

$R^4$

- Wasserstoff, $C_1$-$C_4$-Alkyl, wie Methyl, Ethyl, n-Propyl, iso-Propyl, n-Butyl, iso-Butyl, sec.-Butyl und tert.-Butyl, bevorzugt Methyl und Ethyl, besonders bevorzugt Methyl,

$R^5$

- Halogen, bevorzugt Fluor, Chlor und Brom
- unverzweigtes oder verzweigtes $C_1$-$C_8$-Alkyl, bevorzugt unverzweigtes oder verzweigtes $C_1$-$C_4$-Alkyl, wie Methyl, Ethyl, n-Propyl, iso-Butyl, sec.-Butyl und tert.-Butyl, bevorzugt Methyl und Ethyl, n-Propyl, iso-Propyl und tert.-Butyl, besonders bevorzugt Methyl, Ethyl, iso-Propyl und tert.-Butyl,
- $C_2$-$C_8$-Alkenyl, bevorzugt $C_2$-$C_4$-Alkenyl, besonders bevorzugt Vinyl, Methylvinyl und 2,2-Dimethylvinyl,
- unverzweigtes oder verzweigtes $C_1$-$C_4$-Halogenalkyl, bevorzugt $C_1$-$C_2$-Fluor- und Chloralkyl, besonders bevorzugt Trifluormethyl und Trichlormethyl,
- unverzweigtes oder verzweigtes $C_1$-$C_8$-Alkoxy, bevorzugt unverzweigtes oder verzweigtes $C_1$-$C_4$-Alkoxy, besonders bevorzugt unverzweigtes oder verzweigtes $C_1$-$C_3$-Alkoxy, wie Methoxy, Ethoxy, n-Propoxy und iso-Propoxy,
- $C_2$-$C_8$-Alkoxy-alkyl, bevorzugt $C_2$-$C_4$-Alkoxy-alkyl, wie 3-(Methoxy-methyl), 3-(Methoxy-2-ethyl), 3-(Methoxy-methyl) und iso-Propoxy-methyl,
- $C_3$-$C_{10}$-Cycloalkyl, bevorzugt $C_3$-$C_6$-Cycloalkyl, wie Cyclopropyl, Cyclobutyl, Cyclopentyl und Cyclohexyl, besonders bevorzugt Cyclopropyl,
- Aryl, bevorzugt Phenyl, 1-Naphthyl und 2-Naphthyl, besonders bevorzugt Phenyl,
- $C_7$-$C_{20}$-Aryl-alkyl, bevorzugt $C_7$-$C_{10}$-Phenyl-alkyl, wie Benzyl und Phenethyl,
- ein- bis dreifach durch Halogen substituiertes Aryl, bevorzugt durch Fluor oder Chlor monosubstituiertes Phenyl, wie 2-Fluorphenyl, 3-Fluorphenyl, 4-Fluorphenyl, 2-Chlorphenyl, 3-Chlorphenyl und 4-Chlorphenyl,
- ein- bis dreifach durch unverzweigtes oder verzweigtes $C_1$-$C_8$-Alkyl substituiertes Aryl, bevorzugt durch unverzweigtes oder verzweigtes $C_1$-$C_4$-Alkyl monosubstituiertes Phenyl, besonders bevorzugt durch $C_1$-$C_2$-Alkyl monosubstituiertes Phenyl, wie 4-Methylphenyl und 4-Ethylphenyl,
- ein- bis dreifach durch unverzweigtes oder verzweigtes $C_1$-$C_8$-Alkoxy substituiertes Aryl, bevorzugt durch unverzweigtes oder verzweigtes $C_1$-$C_4$-Alkoxy monosubstituiertes Phenyl, besonders bevorzugt durch $C_1$-$C_2$-Alkoxy monosubstituiertes Phenyl, wie 4-Methoxyphenyl und 4-Ethoxyphenyl,
- ein- bis dreifach durch unverzweigtes oder verzweigtes $C_1$-$C_4$-Halogenalkyl substituiertes Aryl, bevorzugt durch $C_1$-$C_2$-Fluor- und Chloralkyl monosubstituiertes Phenyl, besonders bevorzugt durch Trifluoromethyl und Trichlormethyl monosubstituiertes Phenyl, wie 4-Trifluormethylphenyl und 4-Trichlormethylphenyl,

- ein- bis dreifach durch unverzweigtes oder verzweigtes $C_1$-$C_4$-Halogenalkoxy substituiertes Aryl, bevorzugt durch $C_1$-$C_2$-Fluor- und Chloralkoxy monosubstituiertes Phenyl, besonders bevorzugt durch Trifluormethoxy und Trichlormethoxy monosubstituiertes Phenyl, wie Trifluormethoxyphenyl und 4-Trichlormethoxyphenyl,
- ein- bis dreifach durch Cyano substituiertes Aryl, bevorzugt durch Cyano monosubstituiertes Phenyl, wie 4-Cyanophenyl,
- ein- bis zweifach durch Nitro substituiertes Aryl, bevorzugt durch Nitro monosubstituiertes Phenyl, wie 3-Nitrophenyl,
- im Arylteil ein- bis dreifach durch Halogen substituiertes $C_7$-$C_{20}$-Aryl-alkyl, bevorzugt im Phenylteil durch Fluor oder Chlor monosubstituiertes $C_7$-$C_{10}$-Phenyl-alkyl, wie 4-Fluorbenzyl und 4-Chlorbenzyl,
- im Arylteil ein- bis dreifach durch $C_1$-$C_8$-Alkyl substituiertes $C_7$-$C_{20}$-Aryl-alkyl, bevorzugt im Phenylteil durch $C_1$-$C_4$-Alkyl monosubstituiertes $C_7$-$C_{10}$-Phenyl-alkyl, besonders bevorzugt im Phenylteil durch $C_1$-$C_2$-Alkyl monosubstituiertes $C_7$-$C_{10}$-Phenylalkyl, wie 4-Methylphenyl, 4-Ethylbenzyl und 4-Methylphenethyl,
- im Arylteil ein- bis dreifach durch $C_1$-$C_8$-Alkoxy substituiertes $C_7$-$C_{20}$-Aryl-alkyl, bevorzugt im Phenylteil $C_1$-$C_4$-Alkoxy monosubstituiertes $C_7$-$C_{10}$-Phenylalkyl, besonders bevorzugt im Phenylteil durch $C_1$-$C_2$-Alkoxy monosubstituiertes $C_7$-$C_{10}$-Phenylalkyl, wie 4-Methoxybenzyl, 4-Ethoxybenzyl und 4-Methoxyphenethyl,
- im Arylteil ein- bis dreifach durch $C_1$-$C_4$-Halogenalkyl, substituiertes $C_7$-$C_{20}$-Aryl-alkyl, bevorzugt im Phenylteil $C_1$-$C_2$-Fluor- und Chloralkyl monosubstituiertes $C_7$-$C_{10}$-Phenylalkyl, besonders bevorzugt im Phenylteil durch Trifluormethyl und Trichlormethyl monosubstituiertes $C_7$-$C_{10}$-Phenylalkyl wie 4-Trifluormethylbenzyl und 4-Trichlormethylbenzyl,
- im Arylteil ein- bis dreifach durch $C_1$-$C_4$-Halogenalkoxy substituiertes $C_7$-$C_{20}$-Aryl-alkyl, bevorzugt im Phenylteil durch $C_1$-$C_2$-Halogenalkyl monosubstituiertes $C_7$-$C_{10}$-Phenylalkyl, besonders bevorzugt durch Trifluormethyl und Trichlormethyl monosubstituiertes $C_7$-$C_{10}$-Phenylalkyl wie 4-Trifluormethoxybenzyl und 4-Trichlormethoxybenzyl,
- im Arylteil ein- bis dreifach durch Cyano substituiertes $C_7$-$C_{20}$-Aryl-alkyl, bevorzugt im Phenylteil durch Cyano monosubstituiertes $C_7$-$C_{10}$-Phenyl-alkyl, wie 4-Cyanobenzyl und 4-Cyanophenethyl,
- im Arylteil ein- bis zweifach durch Nitro substituiertes $C_7$-$C_{20}$-Aryl-alkyl, bevorzugt durch Nitro monosubstituiertes $C_7$-$C_{10}$-Phenyl-alkyl, wie 3-Nitrobenzyl.

$R^6$

- ein- bis dreifach durch Halogen substituiertes Aryl, bevorzugt ein- bis dreifach durch Fluor oder Chlor monosubstituiertes Phenyl, wie 2-Fluorphenyl, 3-Fluorphenyl, 4-Fluorphenyl, 2-Chlorphenyl, 3-Chlorphenyl und 4-Chlorphenyl,
- Wasserstoff,
- verzweigtes oder unverzweigtes $C_1$-$C_8$-Alkyl, bevorzugt $C_1$-$C_4$-Alkyl, besonders bevorzugt Methyl, Ethyl, n-Propyl-, iso-Propyl, tert.-Butyl,
- $C_3$-$C_{10}$-Cycloalkyl, bevorzugt $C_3$-$C_8$-Cycloalkyl, besonders bevorzugt $C_3$-$C_6$-Cycloalkyl, wie Cyclopropyl, Cyclobutyl, Cyclopentyl und Cyclohexyl,
- Aryl, bevorzugt Phenyl, 1-Naphthyl und 2-Naphthyl, besonders bevorzugt Phenyl,
- ein- bis dreifach durch Halogen substituiertes Aryl, bevorzugt ein- bis dreifach durch Fluor oder Chlor monosubstituiertes Phenyl, wie 2-Fluorphenyl, 3-Fluorphenyl, 4-Fluorphenyl, 2-Chlorphenyl, 3-Chlorphenyl und 4-Chlorphenyl,
- ein- bis dreifach durch $C_1$-$C_8$-Alkyl substituiertes Aryl, bevorzugt durch $C_1$-$C_4$-Alkyl monosubstituiertes Phenyl, besonders bevorzugt durch $C_1$-$C_2$-monosubstituiertes Phenyl, wie 4-Methylphenyl und 4-Ethylphenyl,
- ein- bis dreifach durch $C_1$-$C_8$-Alkoxy substituiertes Aryl, bevorzugt durch $C_1$-$C_4$-Alkoxy monosubstituiertes Phenyl, besonders bevorzugt durch $C_1$-$C_2$-Alkoxy monosubstituiertes Phenyl, wie 4-Methoxyphenyl und 4-Ethoxyphenyl,
- ein- bis dreifach $C_1$-$C_4$-Halogenalkyl substituiertes Aryl, bevorzugt durch $C_1$-$C_2$-Fluor- und Chloralkyl monosubstituiertes Phenyl, besonders bevorzugt durch Trifluormethyl und Trichlorethyl monosubstituiertes Phenyl, wie 4-Trifluormethylphenyl und 4-Trichlormethylphenyl,

- ein- bis dreifach durch $C_1$-$C_4$-Halogenalkoxy substituiertes Aryl, bevorzugt durch $C_1$-$C_2$-Fluor- und Chloralkoxy monosubstituiertes Phenyl, besonders bevorzugt durch Trifluormethoxy und Trichlormethoxy monosubstituiertes Phenyl, wie Trifluormethoxyphenyl und 4-Trichlormethoxyphenyl,

$R^7$

- Halogen, bevorzugt Fluor, Chlor und Brom, besonders bevorzugt Chlor und Brom,
- $C_3$-$C_8$-Cycloalkyl, bevorzugt $C_3$-$C_7$-Cycloalkyl, besonders bevorzugt $C_3$-$C_6$-Cycloalkyl, wie Cyclopropyl, Cyclobutyl, Cyclopentyl und Cyclohexyl,

$R^9$

- Wasserstoff sowie die für $R^5$ genannte Bedeutung und

n

- null (unsubstituierter Heterocyclus), eins, gegebenenfalls zwei oder drei.

Die (p-Phenoxy-phenoxy)-methyl-fünfringheteroaromaten der allgemeinen Formel I sind geeignet, Schädlinge aus der Klasse der Insekten, Spinnentiere und Nematoden wirksam zu bekämpfen. Sie können im Pflanzenschutz sowie auf dem Hygiene-, Vorratsschutz- und Veterinärsektor als Schädlingsbekämpfungsmittel eingesetzt werden. Die erfindungsgemäßen Verbindungen wirken entwicklungsstörend auf den tierischen Organismus und sind für Wirbeltiere praktisch ungiftig. Die meisten dieser Verbindungen werden überdies leicht zu Stoffen abgebaut, die in der natürlichen Umgebung vorkommen und von Mikroorganismen weiter zerlegt werden.

Zu den schädlichen Insekten gehören aus der Ordnung der Schmetterlinge (Lepidoptera) beispielsweise Plutella maculipennis (Kohlschabe), Leucoptera coffeella (Kaffeemotte), Hyponomeuta malinellus (Apfelbaumgespinstmotte), Argyresthia conjugella (Apfelmotte), Sitotroga cerealella (Getreidemotte), Phthorimaea operculella (Kartoffelmotte), Capua reticulana (Apfelschalenwickler), Sparganothis pilleriana (Springwurm), Cacoecia murinana (Tannentriebwickler), Tortrix viridana (Eichenwickler), Clysia ambiguella (Heu- und Sauerwurm), Evetria buoliana (Kieferntriebwickler), Polychrosis botrana (Bekreuzter Traubenwickler), Cydia pomonella (Obstmade), Laspeyresia molesta (Pfirsichtriebbohrer), Laspeyresia funebrana (Pflaumenwickler), Ostrinia nubilalis (Maiszünsler), Loxostege sticticalis (Rübenzünsler), Ephestia kuehniella (Mehlmotte), Chilo suppressalis (Reisstengelbohrer), Galleria mellonella (Wachsmotte), Malacosoma neustria (Ringelspinner), Dendrolimus pini (Kiefernspinner), Thaumatopoea ptiyocampa (Pinienprozessionsspinner), Phalera bucephala (Mondfleck), Cheimatobia brumata (Kleiner Frostspanner), Hibernia defoliaria (Großer Frostspanner), Bupalus piniarius (Kiefernspanner), Hyphantria cunea (Weißer Bärenspinner), Agrotis segetum (Wintersaateule), Agrotis ypsilon (Ypsiloneule), Barathra brassicae (Kohleule), Cirphis unipuncta (Heerwurm), Prodenia litura (Baumwollraupe), Laphygma exigua (Rüben-Heerwurm), Panolis flammea (Forleule), Earias insulana (Baumwollkapselwurm), Plusia gamma (Gammaeule), Alabama argillacea (Baumwollblattwurm), Lymantria dispar (Schwammspinner), Lymantria monacha (Nonne), Pieris brassicae (Kohlweißling), Aporia crataegi (Baumweißling);

aus der Ordnung der Käfer (Coleoptera) beispielsweise Blitophaga undata (Schwarzer Rübenaaskäfer), Melanotus communis (Drahtwurm), Limonius californicus (Drahtwurm), Agriotes lineatus (Saatschnellkäfer), Agriotes obscurus (Humusschnellkäfer), Agrilus sinuatus (Birnbaum-Prachtkäfer), Meligethes aeneus (Rapsglanzkäfer), Atomaria linearis (Moosknopfkäfer), Epilachna varivestis (Mexikanischer Bohnenkäfer), Phyllopertha horticola (Junikäfer), Popillia japonica (Japankäfer), Melolontha melolontha (Feldmaikäfer), Melolontha hippocastani (Waldmaikäfer), Amphimallus solstitialis (Brachkäfer), Crioceris asparagi (Spargelhähnchen), Lema melanopus (Getreidehähnchen), Leptinotarsa decemlineata (Kartoffelkäfer), Phaedon cochleariae (Meerrettich-Blattkäfer), Phyllotreta nemorum (Kohlerdfloh), Chaetocnema tibialis (Rübenflohkäfer), Phylloides chysocephala (Raps-Flohkäfer), Diabrotica 12-punctata (Südlicher Maiswurzelwurm), Cassida nebulosa (Nebliger Schildkäfer), Bruchus lentis (Linsenkäfer), Bruchus rufimanus (Pferdebohnenkäfer), Bruchus pisorum (Erbsenkäfer), Sitona lineatus (Liniierter Blattrandkäfer), Ortiorrhynchus sulcatus (Gefurchter Lappenrüßler), Otiorrhynchus ovatus (Erdbeerwurzelrüßler), Hylobies abietis (Großer Brauner Rüsselkäfer), Byctiscus betulae (Rebenstecher), Anthonomus pomorum (Apfelblütenstecher), Anthonomus grandis (Kapselkäfer), Ceuthorrhynchus assimilis (Kohlschotenrüßler), Ceuthorrynchus napi (Großer Kohltriebrüßler), Sitophilus granaria (Kornkäfer), Anisandrus dispar (Ungleicher Holzborkenkäfer), Ips typographus (Buchdrucker), Blastophagus piniperda (Gefurchter Waldgärtner);

aus der Ordnung der Zweiflügler (Diptera) beispielswiese Lycoria pectoralis, Mayetiola destructor (Hessenfliege), Basineura brassicae (Kohlschoten-Gallmücke), Contarinia tritici (Gelbe Weizen-Gallmücke), Haplodiplosis equestris (Sattelmücke), Tipula paludosa (Wiesenschnake), Tipula oleracea (Kohlschnake), Dacus cucurbitae (Melonenfliege), Dacus oleae (Olivenfliege), Ceratitis capitata (Mittelmeerfruchtfliege), Rhagoletis cerasi (Kirschfruchtfliege), Rhagoletis pomonella (Apfelmade), Anastrepha ludens (Mexikanische Fruchtfliege), Oscinella frit (Fritfliege), Phorbia coarctata (Brachfliege), Phorbia antiqua (Zwiebelfliege),

Phorbia brassicae (Kleine Kohlfliege), Pegomya hysocyami (Rübenfliege), Anopheles maculipennis, Culex pipiens, Aedes aegypti (Gelbfiebermücke), Aedes vexans, Tabanus bovinus (Rinderbremse), Tipula paludosa (Wiesenschnake), Musca domestica (Stubenfliege), Fannia canicularis (Kleine Stubenfliege), Muscina stabulans, Glossina morsitans (Tsetse-Fliege), Oestrus ovis, Chrysomya macellaria, Chrysomya hominivorax, Lucilia cuprina, Lucilia sericata, Hypoderma lineata;
aus der Ordnung der Hautflügler (Hymenoptera) beispielsweise Athalia rose (Rübenblattwespe), Hoplocampa minuta (Pflaumensägewespe), Monomorium pharaonis (Pharaoameise), Solenopsis geminata (Feuerameise), Atta sexdens (Blattschneiderameise);
aus der Ordnung der Wanzen (Heteroptera) beispielsweise Nezara viridula (Grüne Reiswanze), Eurygaster integriceps (Asiatische Getreidewande), Blissus leucopterus (Chinch bug), Dysdercus cingulatus (Kapok-Wanze), Dysdercus intermedius (Baumwollwanze), Piesma quadrata (Rübenwanze), Lygus pratensis (Gemeine Wiesenwanze);
aus der Ordnung der Pflanzensauger (Homoptera) beispielsweise Perkinsiella saccharicida (Zuckerrohrzikade), Nilaparvata lugens (Braune Zikade), Empoasca fabae (Kartoffelzikade), Psylla mali (Apfelblattsauger), Psylla piri (Birnblattsauger), Trialeurodes vaporariorum (Weiße Fliege), Aphis fabae (Schwarze Bohnenlaus), Aphis pomi (Grüne Apfellaus), Aphis sambuci (Holunderblattlaus), Aphidula nasturtii (Kreuzdornblattlaus), Cerosipha gossypii (Gurkenblattlaus), Sappaphis mali (Rosige Apfellaus), Sappaphis mala (Mehlige Birnblattlaus), Dyasphis radicola (Mehlige Apfelfaltenlaus), Brachycaudus cardui (Große Pflaumenblattlaus), Brevicoryne brassicae (Kohlblattlaus), Phorodon humuli (Hopfenblattlaus), Rhopalomyzus ascalonicus (Zwiebellaus), Myzodes persicae (Grüne Pfirsichlaus), Myzus cerasi (Schwarze Sauerkrischenlaus), Dysaulacorthum pseudosolani (Gefleckte Kartoffellaus), Acyrthosiphon onobrychis (Grüne Erbsenlaus), Macrosiphon rosae (Große Rosenblattlaus), Megoura viciae (Wickenlaus), Schizoneura lanuginosa (Birnenblattlaus), Pemphigus bursarius (Salatwurzellaus), Dreyfusia nordmannianae (Tannentrieblaus), Dreyfusia piceae (Weißtannenstammlaus), Adelges laricis (Rote Fichtengallenlaus), Viteus vitifolii (Reblaus);
aus der Ordnung der Termiten (Isoptera) beispielsweise Reticulitermes lucifugus, Calotermes flavicollis, Leucotermes flavipes, Termes natalensis;
aus der Ordnung der Geradflügler (Orthoptera) beispielsweise Forficula auricularia (Gemeiner Ohrwurm), Acheta domestica (Heimchen), Gryllotalpa gryllotalpa (Maulwurfsgrille), Tachycines asynamorus (Gewächshausschrecke), Locusta migratoria (Wanderheuschrecke), Stauronotus maroccanus (Marokkanische Wanderheuschrecke), Schistocerca peregrina (Wanderheuschrecke), Nomadacris septemfasciata (Wanderheuschrecke), Melanoplus spretus (Felsengebirgsheuschrecke), Melanoplus femur-rubrum (Rotbeinige Heuschrecke), Blatta orientalis (Küchenschabe), Blattella germanica (Deutsche Schabe), Periplaneta americana (Amerikanische Schabe), Blabera gigantes (Riesenschabe).

Zur Klasse der Arachnoidea gehören Spinntentiere (Acarina) beispielsweise Ixodes ricinus (Holzbock), Ornithodorus moubata, Amblyomma americanum, Dermacentor silvarum, Boophilus microplus, Tetranychus telarius, Tetranychus pacificus, Paratetranychus pilosus, Bryobia praetiosa.

Zur Klasse der Nematoden zählen beispielsweise Wurzelgallennematoden, z.B. Meloidogyne incognita, Meloidogyne hapla, Meloidogyne javanica, Zysten bildende Nematoden, z.B. Globodera rostochiensis, Heterodera schacutii, Heterodera avenae, Heterodera glycinae, Heterodera triflolii, Stock- und Blattälchen, z.B. Ditylenchus dipsaci, Ditylenchus destructor, Pratylenchus neglectus, Pratylenchus penetrans, Paratylenchus goodeyi, Paratylenchus curvitatus sowie Tylenchorhynchus dubius, Tylenchorhynchus claytoni, Rotylenchus robustus, Heliocotylenchus multicinctus, Radopholus similis, Belonolaimus longicaudatus, Longidorus elongatus, Trichodorus primitivus.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Wäßrige Anwendungsformen können als Emulsionskonzentrate, Pasten oder netzbare Pulver (Spritzpulver, Öldispersionen) durch Zusatz von Wasser bereitet werden. Zur Herstellung von Emulsionen,

Pasten oder Öldispersionen können die Substanzen als solche oder in einem Öl oder Lösungsmittel gelöst, mittel Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkali, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol, Octylphenol, Nonylphenon, Alkylphenolpolyglykolether, Tributylphenylpolyglykolether, Aklylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten im allgemeinen zwischen 0,1 und 95 Gew.-% Wirkstoff, vorzugsweise zwischen 0,5 und 90 Gew.-%.

Beispiele für Formulierungen sind:

I. 5 Gew.-Teile der Verbindung Nr. 31.25 werden mit 95 Gew.-Teilen feinteiligem Kaolin innig vermischt. Man erhält auf diese Weise ein Stäubemittel, das 3 Gew.-% des Wirkstoffs enthält.

II. 30 Gew.-Teile der Verbindung Nr. 31.25 werden mit einer Mischung aus 92 Gew.-Teilen pulverförmigen Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

III. 10 Gew.-Teile der Verbindung Nr. 31.25 werden in einer Mischung gelöst, die aus 90 Gew.-Teilen Xylol, 6 Gew.-Teilen des Anlagerungsproduktes von 8 bis 10 Mol Ethylenoxid an 1 Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht.

IV. 20 Gew.-Teile der Verbindung Nr. 31.25 werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5 Gew.-Teilen des Anlagerungsproduktes von 40 Mol Ethylenoxid an 1 Mol Ricinusöl besteht.

V. 80 Gew.-Teile der Verbindung Nr. 31.25 werden mit 3 Gew.-Teilen des Natriumsalzes der Diisobutylnaphthalin-alpha-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen.

Die Wirkstoffkonzentrationen in den anwendungsfertigen Zubereitungen können in größeren Bereichen variiert werden.

Im allgemeinen liegen sie zwischen 0,0001 und 10 %, vorzugsweise zwischen 0,01 und 1 %. Die Wirkstoffe können auch mit gutem Erfolg im Ultra-Low-Volume-Verfahren (ULV) verwendet werden, wobei es möglich ist, Formulierungen mit mehr als 95 Gew.-% Wirkstoff oder sogar den Wirkstoff ohne Zusätze auszubringen.

Die Aufwandmenge an Wirkstoff beträgt unter Freilandbedingungen 0,05 bis 10, vorzugsweise 0,1 bis 2 kg/ha, im Fall der Isoazolverbindungen (II.31 und II.32) bevorzugt 0,1 bis 0,5 kg/ha.

Zu den Wirkstoffen können Öle verschiedenen Typs, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

Beispielsweise können folgende Mittel zugemischt werden: 1,2-Dibrom-3-chlorpropan, 1,3-Dichlorpropen, 1,3-Dichlorpropen + 1,2-Dichlorpropan, 1,2-Dibromethan, 2-sec.-Butyl-phenyl-N-methylcarbamat, o-Chlorphenyl-N-methylcarbamat, 3-Isopropyl-5-methylphenyl-N-methylcarbamat, o-Isopropoxyphenyl-N-methylcarbamat, 3,5-Dimethyl-4-methylmercapto-phenyl-N-methylcarbamat, 4-Dimethylamino-3,5-xylyl-N-methylcarbamat, 2-(1,3-Dioxolan-2-yl)-phenyl-N-methylcarbamat, 1-Naphthyl-N-methylcarbamat, 2,3-Dihydro-2,2-dimethyl-benzofuran-7-yl-N-methylcarbamat, 2,2-Dimethyl-1,3-benzodioxol-4-yl-N-methylcarbamat, 2-Dimethylamino-5,6-dimethyl-4-pyrimidinyl-dimethylcarbamat, 2-Methyl-2-(methylthio)-propionaldehyd-O-(methylcarbamoyl)-oxim, S-Methyl-N[(methylcarbamoyl)-oxyl]-thio-acetimidat, Methyl-N′,N′-dimethyl-N-[-(methylcarbamoyl)oxy]-1-thiooxamidat, N-(2-Methylchlorphenyl)-N′,N′-dimethylformamidin, Tetrachlorthiophen, 1-(2,6-Difluor-benzoyl)-3-(4-chlorphenyl)-harnstoff, O,O-Dimethyl-O-(p-nitrophenyl)-phosphorthioat, O,O-Diethyl-O-(p-nitrophenyl)-phosphorthioat, O-Ethyl-O(p-nitrophenyl)-phenylphosphonothioat, O,O-Dimethyl-O-(3-methyl-4-nitrophenyl)-phosphorthioat, O,O-Diethyl-O-(2,4-dichlorphenyl)-phosphorthioat, O-Ethyl-O-(2,4-dichlorphenyl)-phenyl-phosphonothioat, O,O-Dimethyl-O-(2,4,5-trichlorphenyl)-phosphorthioat, O-Ethyl-O-(2,4,5-trichlorphenyl)-ethyl-phosphorthioat, O,O-Dimethyl-O-(4-brom-2,5-dichlorphenyl)-phosphorthioat, O,O-Dimethyl-O-(2,5-dichlor-4-jodphenyl)-phosphorthioat, O,O-Dimethyl-O-(3-methyl-4-methylthiophenyl)-phosphorthioat, O-Ethyl-O-(3-methyl-4-methylthiophenyl)-isopropyl-phosphoramidat, O,O-Diethyl-O-(p-methylsulfinyl-phenyl)-phosphorthioat, O-Ethyl-S-phenyl-ethyl-phosphonodithioat, O,O-Diethyl-[2-chlor-1-(2,4-dichlorphenyl)-vinyl]-phosphat, O,O-Dimethyl-[2-chlor-1-(2,4,5-trichlorphenyl)]-vinylphosphat, O,O-Dimethyl-S-(1′-phenyl)-ethylacetat-phosphordithioat, Bis-(dimethylamino)-fluorphosphinoxid, Octamethyl-pyrophosphoramid, O,O,O,O-Tetraethyldithio-pyrophosphat, S-Chlormethyl-O,O-diethyl-phosphordithioat, O-Ethyl-S,S-dipropyl-phosphordithioat, O,O-Dimethyl-O-2,2-dichlorvinyl-phosphat, O,O-Dimethyl-1,2-dibrom-2,2-dichlorethylphosphat, O,O-Dimethyl-2,2,2-trichlor-1-hydroxy-ethylphosphonat, O,O-Dimethyl-S-[1,2-biscarbethoxy-ethyl-(1)]-phosphordithioat, O,O-dimethyl-O-(1-methyl-2-carbmethoxy-vinyl)-phosphat, O,O-Dimethyl-S-(N-methyl-carbamoyl-methyl)-phosphordithioat, O,O-dimethyl-S-(N-methylcarbamoyl-methyl)-phosphorthioat, O,O-Dimethyl-S-(N-methoxyethyl-carbamoyl-methyl)-phosphordithioat, O,O-Dimethyl-S-(N-formyl-N-methyl-carbamoyl-methyl)-phosphordithioat, O,O-Dimethyl-O-[1-methyl-2-(methyl-carbamoyl-vinyl]-phosphat, O,O-Dimethyl-O-[(1-methyl-2-dimethylcarbamoyl)-vinyl]-phosphat, O,O-Dimethyl-O-[(1-methyl-2-chlor-2-diethylcarbamoyl)-vinyl]-phosphat, O,O-Diethyl-S-(ethylthiomethyl)-phosphordithioat, O,O-Diethyl-S-[-(p-chlorphenylthio)-methyl]-phosphordithioat, O,O-Dimethyl-S-(2-ethylthioethyl)-phosphorthioat, O,O-Dimethyl-S-(2-ethylthioethyl)-phosphordithioat, O,O-Dimethyl-S-(2-ethylsulfinyl-ethyl)-phosphorthioat, O,O-Diethyl-S-(2-ethylthio-ethyl)-phosphordithioat, O,O-Diethyl-S-(2-ethylsulfinyl-ethyl)-phosphorthioat, O,O-Diethyl-thiophosphoryliminophenyl)-acetonitril, O,O-diethyl-S-(2-chlor-1-phthalimidoethyl)-phosphordithioat, O,O-Diethyl-S-[6-chlor-benzoxazolon-(2)-yl(3)]-methyldithiophosphat, O,O-Dimethyl-S-[2-methoxy-1,3-4-thiadiazol-5-[4H]-onyl-(4)-methyl]-phosphordithioat, O,O-Diethyl-O-[3,5,6-trichlor-pyridyl-(2)]-phosphorthioat, O,O-Diethyl-O-(2-pyrazinyl)-phosphorthioat, O,O-Diethyl-O-[2-isopropyl-4-methyl-pyrimidinyl(6)]-phosphorthioat, O,O-Diethyl-O-[2-(diethylamino)-6-methyl-4-pyrimidinyl]-thionophosphat, O,O-Dimethyl-S-(4-oxo-1,2,3-benzotriazin-3-[4H]-yl-methyl)-phosphordithioat, O,O-Dimethyl-S-[(4,6-diamino-1,3,5-triazin-2-yl)-methyl]-phosphordithioat, O,O-Diethyl-(1-phenyl-1,2,4-triazol-3-yl)-thionophosphat, O,S-Dimethyl-phosphor-amidothioat, O,S-Dimethyl-N-acetyl-phosphoramidothioat, alpha-Hexachlorcyclohexan, 1,1-Di-(p-methoxyphenyl)-2,2,2-trichlor-ethan, 6,7,8,9,10,10-Hexachloro-1,5,5a,6,9,9a-hexahydro-6,9-methano-2,4,3-benzodioxa-thiepin-3-oxid, Pyrethrine, DL-2-Allyl-3-methyl-cyclopenten-(2)-on-(1)-yl-(4)-DL-cis,-trans-chysanthemat, 5-Benzyl-furyl-(3)-methyl-DL-cis,-trans-chrysanthemat, 3-Phenoxybenzyl(±)-cis,-trans-2,2-dimethyl-3-(2,2-dichlorvinyl)-cyclopropancarboxylat, alpha-Cyano-3-phenoxybenzyl(±)-cis,-trans-2,2-dimethyl-3-(2,2-dichlorvinyl)-cyclopropancarboxylat, (s)-alpha-Cyano-3-phenoxybenzyl-cis(1R,3R)-2,2-dimethyl-3-(2,2-dibromvinyl)-cyclopropancarboxylat, 3,4,5,6-Tetrahydrophthalimidoethyl-DL-cis,-trans-chrysanthemat, 2-Methyl-5-(2-propinyl)-3-furylmethyl-chrysanthemat, (alpha-Cyano-3-phenoxybenzyl)-alpha-isopropyl-4-chlorphenylacetat.

Herstellungsbeispiele

Beispiel 1

2-Chlor-5-[(4-phenoxyphenoxy)-methyl]-thiophen (Verbindung Nr. 1.63)

5,57 g 4-Phenoxyphenol und 4,14 g Kaliumcarbonat werden in 50 ml trockenem Dimethylformamid 1 Stunde bei 70 °C gerührt. Anschließend werden 5 g 2-Chlor-5-chlormethylthiophen zugetropft, weitere 12 Stunden bei 80 °C und über Nacht bei Raumtemperatur (ca. 20 °C) nachgerührt. Zur Aufarbeitung wird in 500 ml Essigester eingerührt und anschließend 3mal mit Wasser gewaschen. Nach Trocknung über Natriumsulfat und Entfernen des Lösungsmittels wird der Rückstand aus n-Hexan umkristallisiert. Man erhält 3,7 g 2-Chlor-5-[(4-phenoxyphenoxy)-methyl-thiophen; farbloses Pulver; Fp.: 48-49 °C.

Beispiel 2

2-Brom-4-[(4-phenoxy-phenoxy)-methyl]-thiophen (Verbindung Nr. 4.54)

7,44 g 4-Phenoxyphenol und 5,52 g Kaliumcarbonat werden in 50 ml trockenem Dimethylformamid 1 Stunde bei 70°C gerührt. Anschließend werden 5,52 g 2-Brom-4-chlormethylthiophen zugetropft, weitere 12 Stunden bei 80°C und über Nacht bei Raumtemperatur (ca. 20°C) nachgerührt. Zur Aufarbeitung wird in 500 ml Essigester eingerührt und anschließend 3 mal mit Wasser gewaschen. Nach Trocknung über Natriumsulfat und Entfernen des Lösungsmittels wird der Rückstand aus n-Hexan umkristallisiert. Man erhält 10,7 g 2-Brom-4[(4-phenoxyphenoxy)-methyl]-thiophen; farbloses Pulver; Fp.: 90°C.

Beispiel 3

5-Methyl-2-[(4-phenoxyphenoxy)-methyl]-1,3,4-oxadiazol (Verbindung Nr. 20.24)

9,3 g 4-Phenoxyphenol, 9 g Kaliumcarbonat werden in 75 ml trockenem Dimethylformamid 1 Stunde bei 80°C gerührt. Anschließend werden 6,6 g 2-Chlormethyl-5-methyl-1,3,4-oxadiazol in 20 ml trockenem Dimethylformamid zugetropft. Man rührt 10 Stunden bei 80°C und über Nacht bei Raumtemperatur (ca. 20°C) nach. Zur Aufarbeitung wird in 500 ml Eiswasser eingerührt und 3 mal mit Essigester extrahiert. Nach Trocknung über Natriumsulfat und Einengen des Lösungsmittels wird der Rückstand durch Chromatographie an Kieselgel mit n-Hexan/Essigester = 4/1 als Eluent gereingt. Man erhält 12 g 5-Methyl-2-[(4-phenoxyphenoxy)-methyl]-1,3,4-oxadiazol; farblose Kristalle; Fp.: 78-79°C.

Beispiel 4

3-Cyclopropyl-5-[(4-phenoxyphenoxy)-methyl]-1,2,4-oxadiazol (Verbindung Nr. 26.20)

1 g 80 %iges Natriumhydrid wird in 25 ml absolutem Dimethylformamid vorgelegt. Hierzu tropft man unter Kühlen 5,58 g 4-Phenoxy-phenol als Lösung in 30 ml absolutem Dimethylformamid. Nach Beendigung der Wasserstoffentwicklung werden 4,76 g 5-Chlormethyl-3-cyclopropyl-1,2,4-oxadiazol zugetropft und anschließend 8 Stunden bei 80°C gerührt. Nach Entfernen des Lösungsmittels wird der Rückstand in 250 ml Wasser aufgeschlämmt, dreimal mit je 100 ml Ether extrahiert und die vereinigten Etherphasen über Natriumsulfat getrocknet. Nach Entfernen des Lösungsmittels i. Vak. und Umkristallisation des Rohproduktes aus Methanol/Wasser = 1:1 erhält man 8,0 g 3-Cyclopropyl-5-[(4-phenoxyphenoxy)-methyl]-1,2,4-oxadiazol; Fp.: 99°C.

Beispiel 5

2-Methyl-4-[(4-phenoxyphenoxy)-methyl]-thiazol (Verbindung Nr. 11.11)

1,5 g 80 %iges Natriumhydrid werden in 15 ml absolutem Dimethylformamid vorgelegt. Hierzu wird die Lösung von 8,83 g 4-Phenoxyphenol in 15 ml absolutem Dimethylformamid zugetropft. Nach Beendigung der Wasserstoffentwicklung werden 7,0 g 4-Chlormethyl-2-methyl-thiazol zugetropft und noch 3 Stunden bei 80°C nachgerührt. Nach Entfernung des Solvens wird der Rückstand mit 200 ml Wasser aufgeschlämmt und dreimal mit je 50 ml Ether extrahiert. Nach Trocknen über Natriumsulfat und Abziehen des Solvens wird das Rohprodukt aus Cyclohexan/Methyl-tert.-butylether = 20/1 umkristallisiert. Man erhält 6,5 g 2-Methyl-4-[(4-phenoxyphenoxy)-methyl]-thiazol; Fp.: 70-73°C.

Beispiel 6

5-[(p-Phenoxy)-phenoxy-methyl]-3-methyl-isoxazol (Verbindung Nr. 31.25)

Zu 3,1 g Natriumhydrid (85 %ig in Paraffin) in 100 ml Dimethylsulfoxid wird bei 10 bis 20°C 18,6g p-Phenoxyphenol in 50 ml Dimethylsulfoxid getropft, 30 Minuten bei 50°C gerührt und anschließend bei 15 bis 20°C 3-Methyl-5-chlormethylisoxazol zugetropft. Nach 8 Stunden bei 70°C wird wie üblich aufgearbeitet und man erhält 26,7 g Verbindung 31.25; Fp.: 48 bis 51°C.

| Analyse: $C_{17}H_{15}O_3N$ (281) | | | |
|---|---|---|---|
| Ber.: | C 72,6 | H 5,4 | N 5,0 |
| Gef.: | C 73,0 | H 5,6 | N 4,8 |

Entsprechend dieser Herstellvorschriften können die in den folgenden Tabellen 1 bis 33 beschriebenen Verbindungen I hergestellt werden. Im Falle von n = 0 liegt der unsubstituierte Heterocyclus vor.

Tabelle 1

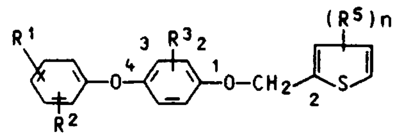

| Verbindung Nr. | $R^1$ | $R^2$ | $R^3$ | $R^5$ | n | phys. Daten (Fp. °C) |
|---|---|---|---|---|---|---|
| 1.1 | H | H | H | - | 0 | 55 |
| 1.2 | 3-Cl | H | H | - | 0 | |
| 1.3 | 3-Br | H | H | - | 0 | |
| 1.4 | 3-F | H | H | - | 0 | |
| 1.5 | $3-CF_3$ | H | H | - | 0 | |
| 1.6 | $3-OCH_3$ | H | H | - | 0 | |
| 1.7 | $3-CH_3$ | H | H | - | 0 | |
| 1.8 | $3-C_2H_5$ | H | H | - | 0 | |
| 1.9 | H | 4-F | H | - | 0 | |
| 1.10 | 3-Cl | 4-F | H | - | 0 | |
| 1.11 | H | H | 3-Cl | - | 0 | |
| 1.12 | H | H | 3-F | - | 0 | |
| 1.13 | $3-CH_3$ | H | 3-F | - | 0 | |
| 1.14 | $3-C_2H_5$ | H | 3-F | - | 0 | |
| 1.15 | 3-Cl | H | 3-F | - | 0 | |
| 1.16 | 3-F | H | 3-F | - | 0 | |
| 1.17 | $3-CF_3$ | H | 3-F | - | 0 | |
| 1.18 | 3-Br | H | 3-F | - | 0 | |
| 1.19 | $3-OCH_3$ | H | 3-F | - | 0 | |
| 1.20 | $3-CH_3$ | H | 3-Cl | - | 0 | |
| 1.21 | $3-C_2H_5$ | H | 3-Cl | - | 0 | |
| 1.22 | 3-Cl | H | 3-Cl | - | 0 | |
| 1.23 | 3-F | H | 3-Cl | - | 0 | |
| 1.24 | $3-CF_3$ | H | 3-Cl | - | 0 | |
| 1.25 | 3-Br | H | 3-Cl | - | 0 | |
| 1.26 | $3-OCH_3$ | H | 3-Cl | - | 0 | |
| 1.27 | 3-Cl | 4-F | 3-Cl | - | 0 | |
| 1.28 | 3-Cl | 4-F | 3-F | - | 0 | |
| 1.29 | H | 4-F | 3-F | - | 0 | |
| 1.30 | H | 4-F | 3-Cl | - | 0 | |
| 1.31 | H | H | H | $5-CH_3$ | 1 | 52 |
| 1.32 | H | H | H | 5-Cyclopropyl | 1 | |
| 1.33 | H | H | 3-F | 5-Cyclopropyl | 1 | |
| 1.34 | H | H | 3-Cl | 5-Cyclopropyl | 1 | |

Tabelle 1 (Fortsetzung)

| Verbindung Nr. | $R^1$ | $R^2$ | $R^3$ | $R^5$ | n | phys. Daten (Fp. °C) |
|---|---|---|---|---|---|---|
| 1.35 | 3-F | H | H | 5-$CH_3$ | 1 | |
| 1.36 | 3-Cl | H | H | 5-$CH_3$ | 1 | |
| 1.37 | 3-Br | H | H | 5-$CH_3$ | 1 | |
| 1.38 | 3-$CF_3$ | H | H | 5-$CH_3$ | 1 | |
| 1.39 | 3-$CH_3$ | H | H | 5-$CH_3$ | 1 | |
| 1.40 | 3-$C_2H_5$ | H | H | 5-$CH_3$ | 1 | |
| 1.41 | 3-$OCH_3$ | H | H | 5-$CH_3$ | 1 | |
| 1.42 | 3-Cl | 4-F | H | 5-$CH_3$ | 1 | |
| 1.43 | 3-F | H | H | 5-Cyclopropyl | 1 | |
| 1.44 | 3-Cl | H | H | 5-Cyclopropyl | 1 | |
| 1.45 | 3-Br | H | H | 5-Cyclopropyl | 1 | |
| 1.46 | 3-$CF_3$ | H | H | 5-Cyclopropyl | 1 | |
| 1.47 | 3-$CH_3$ | H | H | 5-Cyclopropyl | 1 | |
| 1.48 | 3-$C_2H_5$ | H | H | 5-Cyclopropyl | 1 | |
| 1.49 | 3-$OCH_3$ | H | H | 5-Cyclopropyl | 1 | |
| 1.50 | 3-Cl | 4-F | H | 5-Cyclopropyl | 1 | |
| 1.51 | H | H | H | 4-$CH_3$ | 1 | |
| 1.52 | 3-F | H | H | 4-$CH_3$ | 1 | |
| 1.53 | 3-Cl | H | H | 4-$CH_3$ | 1 | |
| 1.54 | 3-Br | H | H | 4-$CH_3$ | 1 | |
| 1.55 | 3-$CF_3$ | H | H | 4-$CH_3$ | 1 | |
| 1.56 | 3-$CH_3$ | H | H | 4-$CH_3$ | 1 | |
| 1.57 | 3-$C_2H_5$ | H | H | 4-$CH_3$ | 1 | |
| 1.58 | 3-$OCH_3$ | H | H | 4-$CH_3$ | 1 | |
| 1.59 | 3-Cl | 4-F | H | 4-$CH_3$ | 1 | |
| 1.60 | H | H | H | 4-Cyclopropyl | 1 | |
| 1.61 | H | H | H | 3-$CH_3$ | 1 | 45 |
| 1.62 | H | H | H | 3-Cyclopropyl | 1 | |
| 1.63 | H | H | H | 5-Cl | 1 | 48-49 |
| 1.64 | H | H | H | 5-Br | 1 | 56-57 |
| 1.65 | 3-F | H | H | 5-Cl | 1 | |
| 1.66 | 3-Cl | H | H | 5-Cl | 1 | |
| 1.67 | 3-Br | H | H | 5-Cl | 1 | |
| 1.68 | 3-$CF_3$ | H | H | 5-Cl | 1 | |
| 1.69 | 3-$CH_3$ | H | H | 5-Cl | 1 | |
| 1.70 | 3-$C_2H_5$ | H | H | 5-Cl | 1 | |
| 1.71 | 3-$OCH_3$ | H | H | 5-Cl | 1 | |
| 1.72 | 3-Cl | 4-F | H | 5-Cl | 1 | |

Tabelle 1 (Fortsetzung)

| Verbindung Nr. | $R^1$ | $R^2$ | $R^3$ | $R^5$ | n | phys. Daten (Fp. °C) |
|---|---|---|---|---|---|---|
| 1.73 | H | H | H | 4-Cl | 1 | |
| 1.74 | H | H | H | 4-Br | 1 | 60-61 |
| 1.75 | 3-F | H | H | 4-Br | 1 | |
| 1.76 | 3-Cl | H | H | 4-Br | 1 | |
| 1.77 | 3-Br | H | H | 4-Br | 1 | |
| 1.78 | $3-CF_3$ | H | H | 4-Br | 1 | |
| 1.79 | $3-CH_3$ | H | H | 4-Br | 1 | |
| 1.80 | $3-C_2H_5$ | H | H | 4-Br | 1 | |
| 1.81 | $3-OCH_3$ | H | H | 4-Br | 1 | |
| 1.82 | 3-Cl | 4-F | H | 4-Br | 1 | |
| 1.83 | H | H | H | 4,5-Dichlor | 2 | |
| 1.84 | $3-CH_3$ | H | H | 4,5-Dichlor | 2 | |
| 1.85 | H | H | H | $4-OCH_3$ | 1 | |
| 1.86 | H | H | H | $4-OC_2H_5$ | 1 | |
| 1.87 | H | H | H | $4-OCH_3$ | 1 | |
| 1.88 | H | H | H | $4-OC_2H_5$ | 1 | |
| 1.89 | 3-F | H | H | $4-OC_2H_5$ | 1 | |
| 1.90 | 3-Cl | H | H | $4-OC_2H_5$ | 1 | |
| 1.91 | 3-Br | H | H | $4-OC_2H_5$ | 1 | |
| 1.92 | $3-CF_3$ | H | H | $4-OC_2H_5$ | 1 | |
| 1.93 | $3-CH_3$ | H | H | $4-OC_2H_5$ | 1 | |
| 1.94 | $3-C_2H_5$ | H | H | $4-OC_2H_5$ | 1 | |
| 1.95 | $3-OCH_3$ | H | H | $4-OC_2H_5$ | 1 | |
| 1.96 | 3-Cl | 4-F | H | $4-OC_2H_5$ | 1 | |
| 1.97 | H | H | 3-F | $4-OC_2H_5$ | 1 | |
| 1.98 | H | H | 3-Cl | $4-OC_2H_5$ | 1 | |
| 1.99 | 3-F | H | H | 5-Br | 1 | 48-50 |
| 1.100 | 3-Cl | H | H | 5-Br | 1 | 44-45 |
| 1.101 | 3-Br | H | H | 5-Br | 1 | 59-61 |
| 1.102 | $3-CF_3$ | H | H | 5-Br | 1 | 38-40 |
| 1.103 | $3-CH_3$ | H | H | 5-Br | 1 | 56-57 |
| 1.104 | $3-C_2H_5$ | H | H | 5-Br | 1 | 48-50 |
| 1.105 | $3-OCH_3$ | H | H | 5-Br | 1 | 58-60 |
| 1.106 | 3-Cl | 4-F | H | 5-Br | 1 | 69-71 |
| 1.107 | H | H | 3-F | 5-Br | 1 | |
| 1.108 | H | H | 3-Cl | 5-Br | 1 | |
| 1.109 | 3-F | H | 3-F | 5-Br | 1 | |
| 1.110 | 3-F | H | 3-Cl | 5-Br | 1 | |

Tabelle 1 (Fortsetzung)

| Verbindung Nr. | $R^1$ | $R^2$ | $R^3$ | $R^5$ | n | phys. Daten (Fp. °C) |
|---|---|---|---|---|---|---|
| 1.111 | 3-Cl | H | 3-F | 5-Br | 1 | |
| 1.112 | 3-Cl | H | 3-Cl | 5-Br | 1 | |
| 1.113 | 3-Br | H | 3-F | 5-Br | 1 | |
| 1.114 | 3-Br | H | 3-Cl | 5-Br | 1 | |
| 1.115 | 3-$CF_3$ | H | 3-F | 5-Br | 1 | 300-MHz-$^1$H-NMR in $CDCl_3$ [ppm]: 5.39 (s) |
| 1.116 | 3-$CF_3$ | H | 3-Cl | 5-Br | 1 | |
| 1.117 | 3-$CH_3$ | H | 3-F | 5-Br | 1 | |
| 1.118 | 3-$CH_3$ | H | 3-Cl | 5-Br | 1 | |
| 1.119 | 3-$C_2H_5$ | H | 3-F | 5-Br | 1 | |
| 1.120 | 3-$C_2H_5$ | H | 3-Cl | 5-Br | 1 | |
| 1.121 | 3-$OCH_3$ | H | 3-F | 5-Br | 1 | |
| 1.122 | 3-Cl | 4-F | 3-F | 5-Br | 1 | |
| 1.123 | 4-F | H | H | 5-Br | 1 | 59-61 |
| 1.124 | 3-t-$C_4H_9$ | H | H | 5-Br | 1 | 300-MHz-$^1$H-NMR zu $CDCl_3$ [ppm]: 1,34(s) |

Tabelle 2

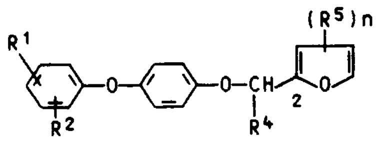

| Verbindung Nr. | $R^1$ | $R^2$ | $R^4$ | $R^5$ | n | phys. Daten (Fp. °C) |
|---|---|---|---|---|---|---|
| 2.1 | H | H | H | - | 0 | |
| 2.2 | H | H | $CH_3$ | - | 0 | |
| 2.3 | 3-F | H | H | - | 0 | |
| 2.4 | 3-Cl | H | H | - | 0 | |
| 2.5 | 3-Br | H | H | - | 0 | |
| 2.6 | 3-$CF_3$ | H | H | - | 0 | |
| 2.7 | 3-$CH_3$ | H | H | - | 0 | |
| 2.8 | 3-$C_2H_5$ | H | H | - | 0 | |
| 2.9 | 3-$OCH_3$ | H | H | - | 0 | |
| 2.10 | 3-Cl | 4-F | H | - | 0 | |
| 2.11 | H | H | H | 4-$CH_3$ | 1 | |
| 2.12 | 3-F | H | H | 4-$CH_3$ | 1 | |
| 2.13 | 3-Cl | H | H | 4-$CH_3$ | 1 | |
| 2.14 | 3-Br | H | H | 4-$CH_3$ | 1 | |
| 2.15 | 3-$CF_3$ | H | H | 4-$CH_3$ | 1 | |
| 2.16 | 3-$CH_3$ | H | H | 4-$CH_3$ | 1 | |
| 2.17 | 3-$C_2H_5$ | H | H | 4-$CH_3$ | 1 | |
| 2.18 | 3-$OCH_3$ | H | H | 4-$CH_3$ | 1 | |
| 2.19 | 3-Cl | 4-F | H | 4-$CH_3$ | 1 | |
| 2.20 | H | H | H | 5-$CH_3$ | 1 | |
| 2.21 | 3-F | H | H | 5-$CH_3$ | 1 | |
| 2.22 | 3-Cl | H | H | 5-$CH_3$ | 1 | |
| 2.23 | 3-Br | H | H | 5-$CH_3$ | 1 | |
| 2.24 | 3-$CF_3$ | H | H | 5-$CH_3$ | 1 | |
| 2.25 | 3-$CH_3$ | H | H | 5-$CH_3$ | 1 | |
| 2.26 | 3-$C_2H_5$ | H | H | 5-$CH_3$ | 1 | |
| 2.27 | 3-$OCH_3$ | H | H | 5-$CH_3$ | 1 | |
| 2.28 | 3-Cl | 4-F | H | 5-$CH_3$ | 1 | |
| 2.29 | H | H | H | 5-Cyclopropyl | 1 | |
| 2.30 | 3-F | H | H | 5-Cyclopropyl | 1 | |
| 2.31 | 3-Cl | H | H | 5-Cyclopropyl | 1 | |
| 2.32 | 3-Br | H | H | 5-Cyclopropyl | 1 | |

Tabelle 2 (Fortsetzung)

| Verbindung Nr. | $R^1$ | $R^2$ | $R^4$ | $R^5$ | n | phys. Daten (Fp. °C) |
|---|---|---|---|---|---|---|
| 2.33 | 3-$CF_3$ | H | H | 5-Cyclopropyl | 1 | |
| 2.34 | 3-$CH_3$ | H | H | 5-Cyclopropyl | 1 | |
| 2.35 | 3-$C_2H_5$ | H | H | 5-Cyclopropyl | 1 | |
| 2.36 | 3-$OCH_3$ | H | H | 5-Cyclopropyl | 1 | |
| 2.37 | 3-Cl | 4-F | H | 5-Cyclopropyl | 1 | |
| 2.38 | 3-F | H | H | 4-Cyclopropyl | 1 | |
| 2.39 | 3-Cl | H | H | 4-Cyclopropyl | 1 | |
| 2.40 | 3-Br | H | H | 4-Cyclopropyl | 1 | |
| 2.41 | 3-$CF_3$ | H | H | 4-Cyclopropyl | 1 | |
| 2.42 | 3-$CH_3$ | H | H | 4-Cyclopropyl | 1 | |
| 2.43 | 3-$C_2H_5$ | H | H | 4-Cyclopropyl | 1 | |
| 2.44 | 3-$OCH_3$ | H | H | 4-Cyclopropyl | 1 | |
| 2.45 | 3-Cl | H | H | 4-Cyclopropyl | 1 | |

Tabelle 3

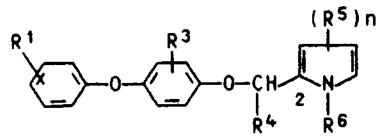

| Verbindung Nr. | $R^1$ | $R^3$ | $R^4$ | $R^5$ | $R^6$ | n | phys. Daten (Fp. °C) |
|---|---|---|---|---|---|---|---|
| 3.1 | H | H | H | - | $CH_3$ | 0 | |
| 3.2 | 3-F | H | H | - | $CH_3$ | 0 | |
| 3.3 | 3-Cl | H | H | - | $CH_3$ | 0 | |
| 3.4 | 3-Br | H | H | - | $CH_3$ | 0 | |
| 3.5 | $3-CF_3$ | H | H | - | $CH_3$ | 0 | |
| 3.6 | $3-CH_3$ | H | H | - | $CH_3$ | 0 | |
| 3.7 | 4-F | H | H | - | $CH_3$ | 0 | |
| 3.8 | $3-C_2H_5$ | H | H | - | $CH_3$ | 0 | |
| 3.9 | 3-F | H | H | 4-Cl | $CH_3$ | 1 | |
| 3.10 | 3-Cl | H | H | 4-Cl | $CH_3$ | 1 | |
| 3.11 | 3-Br | H | H | 4-Cl | $CH_3$ | 1 | |
| 3.12 | $3-CF_3$ | H | H | 4-Cl | $CH_3$ | 1 | |
| 3.13 | $3-CH_3$ | H | H | 4-Cl | $CH_3$ | 1 | |
| 3.14 | 4-F | H | H | 4-Cl | $CH_3$ | 1 | |
| 3.15 | $3-C_2H_5$ | H | H | 4-Cl | $CH_3$ | 1 | |
| 3.16 | $3-OC_2H_5$ | H | H | 4-Cl | $CH_3$ | 1 | |
| 3.17 | $3-OCH_3$ | H | H | 4-Cl | $CH_3$ | 1 | |
| 3.18 | 3-F | H | H | $4-i-C_3H_7$ | $CH_3$ | 1 | |
| 3.19 | 3-Cl | H | H | $4-i-C_3H_7$ | $CH_3$ | 1 | |
| 3.20 | 3-Br | H | H | $4-i-C_3H_7$ | $CH_3$ | 1 | |
| 3.21 | $3-CF_3$ | H | H | $4-i-C_3H_7$ | $CH_3$ | 1 | |
| 3.22 | $3-CH_3$ | H | H | $4-i-C_3H_7$ | $CH_3$ | 1 | |
| 3.23 | 4-F | H | H | $4-i-C_3H_7$ | $CH_3$ | 1 | |
| 3.24 | $3-C_2H_5$ | H | H | $4-i-C_3H_7$ | $CH_3$ | 1 | |
| 3.25 | $3-OC_2H_5$ | H | H | $4-i-C_3H_7$ | $CH_3$ | 1 | |
| 3.26 | $3-OCH_3$ | H | H | $4-i-C_3H_7$ | $CH_3$ | 1 | |
| 3.27 | 3-F | H | H | 5-Cl | $CH_3$ | 1 | |
| 3.28 | 3-Cl | H | H | 5-Cl | $CH_3$ | 1 | |
| 3.29 | 3-Br | H | H | 5-Cl | $CH_3$ | 1 | |

Tabelle 3 (Fortsetzung)

| Verbindung Nr. | $R^1$ | $R^3$ | $R^4$ | $R^5$ | $R^6$ | n | phys. Daten (Fp. °C) |
|---|---|---|---|---|---|---|---|
| 3.30 | 3-$CF_3$ | H | H | 5-Cl | $CH_3$ | 1 | |
| 3.31 | 3-$CH_3$ | H | H | 5-Cl | $CH_3$ | 1 | |
| 3.32 | 4-F | H | H | 5-Cl | $CH_3$ | 1 | |
| 3.33 | 3-$C_2H_5$ | H | H | 5-Cl | $CH_3$ | 1 | |
| 3.34 | 3-$OC_2H_5$ | H | H | 5-Cl | $CH_3$ | 1 | |
| 3.35 | 3-$OCH_3$ | H | H | 5-Cl | $CH_3$ | 1 | |
| 3.36 | 3-F | H | $CH_3$ | 5-Cl | $CH_3$ | 1 | |
| 3.37 | 3-Cl | H | $CH_3$ | 5-Cl | $CH_3$ | 1 | |
| 3.38 | 3-Br | H | $CH_3$ | 5-Cl | $CH_3$ | 1 | |
| 3.39 | 3-$CF_3$ | H | $CH_3$ | 5-Cl | $CH_3$ | 1 | |
| 3.40 | 4-F | H | $CH_3$ | 5-Cl | $CH_3$ | 1 | |
| 3.41 | 3-$C_2H_5$ | H | $CH_3$ | 5-Cl | $CH_3$ | 1 | |
| 3.42 | 3-$OC_2H_5$ | H | $CH_3$ | 5-Cl | $CH_3$ | 1 | |
| 3.43 | 3-$OCH_3$ | H | $CH_3$ | 5-Cl | $CH_3$ | 1 | |
| 3.44 | 3-F | 3-F | $CH_3$ | - | $CH_3$ | 0 | |
| 3.45 | 3-Cl | 3-F | H | - | $CH_3$ | 0 | |
| 3.46 | 3-Br | 3-F | H | - | $CH_3$ | 0 | |
| 3.47 | 4-F | 3-F | H | - | $CH_3$ | 0 | |

Tabelle 4

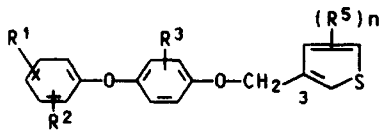

| Verbindung Nr. | $R^1$ | $R^2$ | $R^3$ | $R^5$ | n | phys. Daten (Fp. °C) |
|---|---|---|---|---|---|---|
| 4.1 | H | H | H | - | 0 | 74 |
| 4.2 | H | H | 3-F | - | 0 | |
| 4.3 | H | H | 3-Cl | - | 0 | |
| 4.4 | 3-F | H | H | - | 0 | |
| 4.5 | 3-Cl | H | H | - | 0 | |
| 4.6 | 3-Br | H | H | - | 0 | |
| 4.7 | 3-$CF_3$ | H | H | - | 0 | |
| 4.8 | 3-$CH_3$ | H | H | - | 0 | |
| 4.9 | 3-$C_2H_5$ | H | H | - | 0 | |
| 4.10 | 3-$OCH_3$ | H | H | - | 0 | |
| 4.11 | 3-Cl | 4-F | H | - | 0 | |
| 4.12 | H | H | H | 4-$CH_3$ | 1 | |
| 4.13 | H | H | H | 4-Cyclopropyl | 1 | |
| 4.14 | H | H | H | 5-$CH_3$ | 1 | |
| 4.15 | H | H | H | 5-Cyclopropyl | 1 | |
| 4.16 | H | H | H | 4-$OCH_3$ | 1 | |
| 4.17 | H | H | H | 4-$OC_2H_5$ | 1 | |
| 4.18 | H | H | H | 5-$OCH_3$ | 1 | |
| 4.19 | H | H | H | 5-$OC_2H_5$ | 1 | |
| 4.20 | H | H | H | 4-Cl | 1 | |
| 4.21 | H | H | H | 4-Br | 1 | |
| 4.22 | H | H | H | 5-Cl | 1 | 84-85 |
| 4.23 | 3-F | H | H | 5-Cl | 1 | |
| 4.24 | 3-Cl | H | H | 5-Cl | 1 | |
| 4.25 | 3-Br | H | H | 5-Cl | 1 | |
| 4.26 | 3-$CF_3$ | H | H | 5-Cl | 1 | |
| 4.27 | 3-$CH_3$ | H | H | 5-Cl | 1 | |
| 4.28 | 3-$C_2H_5$ | H | H | 5-Cl | 1 | |
| 4.29 | 3-$OCH_3$ | H | H | 5-Cl | 1 | |
| 4.30 | 3-Cl | 4-F | H | 5-Cl | 1 | |
| 4.40 | H | H | 3-Cl | 5-Cl | 1 | |
| 4.41 | H | H | 3-F | 5-Cl | 1 | |
| 4.42 | 3-F | H | 3-F | 5-Cl | 1 | |

Tabelle 4 (Fortsetzung)

| Verbindung Nr. | $R^1$ | $R^2$ | $R^3$ | $R^5$ | n | phys. Daten (Fp. °C) |
|---|---|---|---|---|---|---|
| 4.43 | 3-Cl | H | 3-F | 5-Cl | 1 | |
| 4.44 | 3-$CF_3$ | H | 3-F | 5-Cl | 1 | |
| 4.45 | 3-$OCH_3$ | H | 3-F | 5-Cl | 1 | |
| 4.46 | 3-Cl | 4-F | 3-F | 5-Cl | 1 | |
| 4.47 | 3-F | H | 3-Cl | 5-Cl | 1 | |
| 4.48 | 3-Cl | H | 3-Cl | 5-Cl | 1 | |
| 4.49 | 3-Br | H | 3-Cl | 5-Cl | 1 | |
| 4.50 | 3-$CF_3$ | H | 3-Cl | 5-Cl | 1 | |
| 4.51 | 3-$OCH_3$ | H | 3-Cl | 5-Cl | 1 | |
| 4.52 | 3-$CH_3$ | H | 3-Cl | 5-Cl | 1 | |
| 4.53 | 3-Cl | 4-F | 3-Cl | 5-Cl | 1 | |
| 4.54 | H | H | H | 5-Br | 1 | 90 |
| 4.55 | 3-F | H | H | 5-Br | 1 | |
| 4.56 | 3-Cl | H | H | 5-Br | 1 | |
| 4.57 | 3-Br | H | H | 5-Br | 1 | |
| 4.58 | 3-$CF_3$ | H | H | 5-Br | 1 | |
| 4.59 | 3-$CH_3$ | H | H | 5-Br | 1 | |
| 4.60 | 3-$C_2H_5$ | H | H | 5-Br | 1 | |
| 4.61 | 3-$OCH_3$ | H | H | 5-Br | 1 | |
| 4.62 | 3-Cl | 4-F | H | 5-Br | 1 | |
| 4.63 | H | H | H | 4,5-Dichlor | 2 | 68-71 |
| 4.64 | 3-F | H | H | 4,5-Dichlor | 2 | 53-54 |
| 4.65 | 3-Cl | H | H | 4,5-Dichlor | 2 | 74-76 |
| 4.66 | 3-Br | H | H | 4,5-Dichlor | 2 | 86-90 |
| 4.67 | 3-$CF_3$ | H | H | 4,5-Dichlor | 2 | 69-72 |
| 4.68 | 3-$CH_3$ | H | H | 4,5-Dichlor | 2 | 67-71 |
| 4.69 | 3-$C_2H_5$ | H | H | 4,5-Dichlor | 2 | 300-MHz-$^1$H-NMR in $CDCl_3$ [ppm]: 4,96(s) |
| 4.70 | 3-$OCH_3$ | H | H | 4,5-Dichlor | 2 | 59-62 |
| 4.71 | 3-Cl | 4-F | H | 4,5-Dichlor | 2 | 300-MHz-$^1$H-NMR in $CDCl_3$ [ppm]: 4,95 (s) |
| 4.72 | H | H | 3-Cl | 4,5-Dichlor | 2 | |
| 4.73 | H | H | 3-F | 4,5-Dichlor | 2 | |
| 4.74 | H | H | H | 4,5-Dibrom | 2 | 95 |
| 4.75 | H | H | 3-Cl | 4,5-Dibrom | 2 | |

Tabelle 4 (Fortsetzung)

| Verbindung Nr. | $R^1$ | $R^2$ | $R^3$ | $R^5$ | n | phys. Daten (Fp. °C) |
|---|---|---|---|---|---|---|
| 4.76 | H | H | 3-F | 4,5-Dibrom | 2 | |
| 4.77 | 3-F | H | H | 4,5-Dibrom | 2 | 65-67 |
| 4.78 | 3-Cl | H | H | 4,5-Dibrom | 2 | 95 |
| 4.79 | 3-Br | H | H | 4,5-Dibrom | 2 | 98 |
| 4.80 | 3-$CF_3$ | H | H | 4,5-Dibrom | 2 | 81 |
| 4.81 | 3-$CH_3$ | H | H | 4,5-Dibrom | 2 | 80 |
| 4.82 | 3-$C_2H_5$ | H | H | 4,5-Dibrom | 2 | 89-91 |
| 4.83 | 3-$OCH_3$ | H | H | 4,5-Dibrom | 2 | 75 |
| 4.84 | 3-Cl | 4-F | H | 4,5-Dibrom | 2 | 300 MHz-$^1$H-NMR in $CDCl_3$ [ppm]: 4,96(s) |
| 4.85 | 3-F | H | 3-Cl | 4,5-Dibrom | 2 | |
| 4.86 | 3-Cl | H | 3-Cl | 4,5-Dibrom | 2 | |
| 4.87 | 3-Br | H | 3-Cl | 4,5-Dibrom | 2 | |
| 4.88 | 3-$CF_3$ | H | 3-Cl | 4,5-Dibrom | 2 | |
| 4.89 | 3-$CH_3$ | H | 3-Cl | 4,5-Dibrom | 2 | 92-95 |
| 4.90 | 3-$C_2H_5$ | H | 3-Cl | 4,5-Dibrom | 2 | |
| 4.91 | 3-$OCH_3$ | H | 3-Cl | 4,5-Dibrom | 2 | |
| 4.92 | 3-Cl | 4-F | 3-Cl | 4,5-Dibrom | 2 | |
| 4.93 | 3-F | H | 3-F | 4,5-Dibrom | 2 | |
| 4.94 | 3-Cl | H | 3-F | 4,5-Dibrom | 2 | |
| 4.95 | 3-Br | H | 3-F | 4,5-Dibrom | 2 | |
| 4.96 | 3-$CF_3$ | H | 3-F | 4,5-Dibrom | 2 | |
| 4.97 | 3-$CH_3$ | H | 3-F | 4,5-Dibrom | 2 | |
| 4.98 | 3-$OCH_3$ | H | 3-F | 4,5-Dibrom | 2 | |
| 4.99 | H | H | H | 2,4,5-Tribrom | 3 | |
| 4.100 | 3-Cl | 4-F | 3-F | 4,5-Dibrom | 2 | |
| 4.101 | H | 4-F | 3-F | 4,5-Dibrom | 2 | |
| 4.102 | H | 4-F | 3-Cl | 4,5-Dibrom | 2 | |
| 4.103 | 4-F | H | H | 4,5-Dichlor | 2 | 57-58 |

Tabelle 5

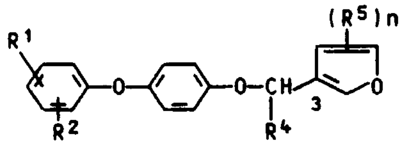

| Verbindung Nr. | $R^1$ | $R^2$ | $R^4$ | $R^5$ | n | phys. Daten (Fp. °C) |
|---|---|---|---|---|---|---|
| 5.1 | H | H | H | - | 0 | |
| 5.2 | H | H | $CH_3$ | - | 0 | |
| 5.3 | 3-F | H | H | - | 0 | |
| 5.4 | 3-Cl | H | H | - | 0 | |
| 5.5 | 3-Br | H | H | - | 0 | |
| 5.6 | 3-$CF_3$ | H | H | - | 0 | |
| 5.7 | 3-$CH_3$ | H | H | - | 0 | |
| 5.8 | 3-$C_2H_5$ | H | H | - | 0 | |
| 5.9 | 3-$OCH_3$ | H | H | - | 0 | |
| 5.10 | 3-Cl | 4-F | H | - | 0 | |
| 5.11 | H | H | H | 4-$CH_3$ | 1 | |
| 5.12 | 3-F | H | H | 4-$CH_3$ | 1 | |
| 5.13 | 3-Cl | H | H | 4-$CH_3$ | 1 | |
| 5.14 | 3-Br | H | H | 4-$CH_3$ | 1 | |
| 5.15 | 3-$CF_3$ | H | H | 4-$CH_3$ | 1 | |
| 5.16 | 3-$CH_3$ | H | H | 4-$CH_3$ | 1 | |
| 5.17 | 3-$C_2H_5$ | H | H | 4-$CH_3$ | 1 | |
| 5.18 | 3-$OCH_3$ | H | H | 4-$CH_3$ | 1 | |
| 5.19 | 3-Cl | 4-F | H | 4-$CH_3$ | 1 | |
| 5.20 | H | H | H | 4-Cyclopropyl | 1 | |
| 5.21 | 3-F | H | H | 4-Cyclopropyl | 1 | |
| 5.22 | 3-Cl | H | H | 4-Cyclopropyl | 1 | |
| 5.23 | 3-Br | H | H | 4-Cyclopropyl | 1 | |
| 5.24 | 3-$CF_3$ | H | H | 4-Cyclopropyl | 1 | |
| 5.25 | 3-$CH_3$ | H | H | 4-Cyclopropyl | 1 | |
| 5.26 | 3-$C_2H_5$ | H | H | 4-Cyclopropyl | 1 | |
| 5.27 | 3-$OCH_3$ | H | H | 4-Cyclopropyl | 1 | |
| 5.28 | 3-Cl | 4-F | H | 4-Cyclopropyl | 1 | |
| 5.29 | H | H | H | 5-$CH_3$ | 1 | |
| 5.30 | H | H | H | 5-iso-$C_3H_7$ | 1 | 300-MHz-$^1$H-NMR in $CDCl_3$ [ppm]: 4,86 (s) |

Tabelle 5 (Fortsetzung)

| Verbindung Nr. | $R^1$ | $R^2$ | $R^4$ | $R^5$ | n | phys. Daten (Fp. °C) |
|---|---|---|---|---|---|---|
| 5.31 | 3-F | H | H | $5-CH_3$ | 1 | |
| 5.32 | 3-Cl | H | H | $5-CH_3$ | 1 | |
| 5.33 | 3-Br | H | H | $5-CH_3$ | 1 | |
| 5.34 | $3-CF_3$ | H | H | $5-CH_3$ | 1 | |
| 5.35 | $3-CH_3$ | H | H | $5-CH_3$ | 1 | |
| 5.36 | $3-C_2H_5$ | H | H | $5-CH_3$ | 1 | |
| 5.37 | $3-OCH_3$ | H | H | $5-CH_3$ | 1 | |
| 5.38 | 3-Cl | 4-F | H | $5-CH_3$ | 1 | |
| 5.39 | H | H | H | 5-Cyclopropyl | 1 | |
| 5.40 | 3-F | H | H | 5-Cyclopropyl | 1 | |
| 5.41 | 3-Cl | H | H | 5-Cyclopropyl | 1 | |
| 5.42 | 3-Br | H | H | 5-Cyclopropyl | 1 | |
| 5.43 | $3-CF_3$ | H | H | 5-Cyclopropyl | 1 | |
| 5.44 | $3-CH_3$ | H | H | 5-Cyclopropyl | 1 | |
| 5.45 | $3-C_2H_5$ | H | H | 5-Cyclopropyl | 1 | |
| 5.46 | $3-OCH_3$ | H | H | 5-Cyclopropyl | 1 | |
| 5.47 | 3-Cl | 4-F | H | 5-Cyclopropyl | 1 | |

Tabelle 6 (Fortsetzung)

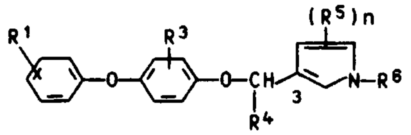

| Verbindung Nr. | $R^1$ | $R^3$ | $R^4$ | $R^5$ | $R^6$ | n | phys. Daten (Fp. °C) |
|---|---|---|---|---|---|---|---|
| 6.1 | H | H | H | - | $CH_3$ | 0 | |
| 6.2 | H | H | $CH_3$ | - | $CH_3$ | 0 | |
| 6.3 | 3-F | H | H | - | $CH_3$ | 0 | |
| 6.4 | 3-Cl | H | H | - | $CH_3$ | 0 | |
| 6.5 | 3-Br | H | H | - | $CH_3$ | 0 | |
| 6.6 | 3-$CF_3$ | H | H | - | $CH_3$ | 0 | |
| 6.7 | 3-$CH_3$ | H | H | - | $CH_3$ | 0 | |
| 6.8 | 3-$C_2H_5$ | H | H | - | $CH_3$ | 0 | |
| 6.9 | 3-$OCH_3$ | H | H | - | $CH_3$ | 0 | |
| 6.10 | 3-$OC_2H_5$ | H | H | - | $CH_3$ | 0 | |
| 6.11 | 4-F | H | H | - | $CH_3$ | 0 | |
| 6.12 | 3-F | H | H | 4-Cl | $CH_3$ | 1 | |
| 6.13 | 3-Cl | H | H | 4-Cl | $CH_3$ | 1 | |
| 6.14 | 3-Br | H | H | 4-Cl | $CH_3$ | 1 | |
| 6.15 | 3-$CF_3$ | H | H | 4-Cl | $CH_3$ | 1 | |
| 6.16 | 3-$CH_3$ | H | H | 4-Cl | $CH_3$ | 1 | |
| 6.17 | 3-$C_2H_5$ | H | H | 4-Cl | $CH_3$ | 1 | |
| 6.18 | 3-$CH_3$ | H | H | 4-Cl | $CH_3$ | 1 | |
| 6.19 | 3-$OC_2H_5$ | H | H | 4-Cl | $CH_3$ | 1 | |
| 6.20 | 4-F | H | H | 4-Cl | $CH_3$ | 1 | |
| 6.21 | 3-F | 3-F | $CH_3$ | 4-Cl | $CH_3$ | 1 | |
| 6.22 | 3-Cl | 3-F | $CH_3$ | 4-Cl | $CH_3$ | 1 | |
| 6.23 | 3-Br | 3-F | $CH_3$ | 4-Cl | $CH_3$ | 1 | |
| 6.24 | 3-$CF_3$ | 3-F | $CH_3$ | 4-Cl | $CH_3$ | 1 | |
| 6.25 | 3-$CH_3$ | 3-F | $CH_3$ | 4-Cl | $CH_3$ | 1 | |
| 6.26 | 3-$C_2H_5$ | 3-F | $CH_3$ | 4-Cl | $CH_3$ | 1 | |
| 6.27 | 3-$OCH_3$ | 3-F | $CH_3$ | 4-Cl | $CH_3$ | 1 | |
| 6.28 | 3-$OC_2H_5$ | 3-F | $CH_3$ | 4-Cl | $CH_3$ | 1 | |
| 6.29 | 4-F | 3-F | $CH_3$ | 4-Cl | $CH_3$ | 1 | |
| 6.30 | 3-F | 3-F | $CH_3$ | 5-Cl | $CH_3$ | 1 | |
| 6.31 | 3-Cl | 3-F | $CH_3$ | 5-Cl | $CH_3$ | 1 | |
| 6.32 | 3-Br | 3-F | $CH_3$ | 5-Cl | $CH_3$ | 1 | |
| 6.33 | 3-$CF_3$ | 3-F | $CH_3$ | 5-Cl | $CH_3$ | 1 | |

Tabelle 6 (Fortsetzung)

| Verbindung Nr. | $R^1$ | $R^3$ | $R^4$ | $R^5$ | $R^6$ | n | phys. Daten (Fp. °C) |
|---|---|---|---|---|---|---|---|
| 6.34 | 3-$CH_3$ | 3-F | $CH_3$ | 5-Cl | $CH_3$ | 1 | |
| 6.35 | 3-$C_2H_5$ | 3-F | $CH_3$ | 5-Cl | $CH_3$ | 1 | |
| 6.36 | 3-$OCH_3$ | 3-F | $CH_3$ | 5-Cl | $CH_3$ | 1 | |
| 6.37 | 3-$OC_2H_5$ | 3-F | $CH_3$ | 5-Cl | $CH_3$ | 1 | |
| 6.38 | 4-F | 3-F | $CH_3$ | 5-Cl | $CH_3$ | 1 | |
| 6.39 | 3-F | H | H | 4-$CH_3$ | $CH_3$ | 1 | |
| 6.40 | 3-Cl | H | H | 4-$CH_3$ | $CH_3$ | 1 | |
| 6.41 | 3-Br | H | H | 4-$CH_3$ | $CH_3$ | 1 | |
| 6.42 | 3-$CF_3$ | H | H | 4-$CH_3$ | $CH_3$ | 1 | |
| 6.43 | 3-$CH_3$ | H | H | 4-$CH_3$ | $CH_3$ | 1 | |
| 6.44 | 3-$C_2H_5$ | H | H | 4-$CH_3$ | $CH_3$ | 1 | |
| 6.45 | 3-$OCH_3$ | H | H | 4-$CH_3$ | $CH_3$ | 1 | |
| 6.46 | 3-$OC_2H_5$ | H | H | 4-$CH_3$ | $CH_3$ | 1 | |
| 6.47 | 4-F | H | H | 4-$CH_3$ | $CH_3$ | 1 | |

Tabelle 7

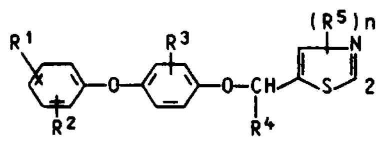

| Verbindung Nr. | $R^1$ | $R^2$ | $R^3$ | $R^4$ | $R^5$ | n | phys. Daten (Fp. °C) |
|---|---|---|---|---|---|---|---|
| 7.1 | H | H | H | H | - | 0 | |
| 7.2 | H | H | H | $CH_3$ | - | 0 | |
| 7.3 | 3-F | H | H | H | - | 0 | |
| 7.4 | 3-Cl | H | H | H | - | 0 | |
| 7.5 | 3-Br | H | H | H | - | 0 | |
| 7.6 | 3-$CF_3$ | H | H | H | - | 0 | |
| 7.7 | 3-$CH_3$ | H | H | H | - | 0 | |
| 7.8 | 3-$C_2H_5$ | H | H | H | - | 0 | |
| 7.9 | 3-$OCH_3$ | H | H | H | - | 0 | |
| 7.10 | 3-Cl | 4-F | H | H | - | 0 | |
| 7.11 | H | H | H | H | 2-$CH_3$ | 1 | |
| 7.12 | 3-F | H | H | H | 2-$CH_3$ | 1 | |
| 7.13 | 3-Cl | H | H | H | 2-$CH_3$ | 1 | |
| 7.14 | 3-Br | H | H | H | 2-$CH_3$ | 1 | |
| 7.15 | 3-$CF_3$ | H | H | H | 2-$CH_3$ | 1 | |
| 7.16 | 3-$CH_3$ | H | H | H | 2-$CH_3$ | 1 | |
| 7.17 | 3-$C_2H_5$ | H | H | H | 2-$CH_3$ | 1 | |
| 7.18 | 3-$OCH_3$ | H | H | H | 2-$CH_3$ | 1 | |
| 7.19 | 3-Cl | 4-F | H | H | 2-$CH_3$ | 1 | |
| 7.20 | H | H | H | H | 2-Cyclopropyl | 1 | |
| 7.21 | 3-F | H | H | H | 2-Cyclopropyl | 1 | |
| 7.22 | 3-Cl | H | H | H | 2-Cyclopropyl | 1 | |
| 7.23 | 3-Br | H | H | H | 2-Cyclopropyl | 1 | |
| 7.24 | 3-$CF_3$ | H | H | H | 2-Cyclopropyl | 1 | |
| 7.25 | 3-$C_2H_5$ | H | H | H | 2-Cyclopropyl | 1 | |
| 7.26 | 3-$CH_3$ | H | H | H | 2-Cyclopropyl | 1 | |
| 7.27 | 3-$OCH_3$ | H | H | H | 2-Cyclopropyl | 1 | |
| 7.28 | 3-Cl | 4-Cl | H | H | 2-Cyclopropyl | 1 | |
| 7.29 | H | H | 3-F | H | 2-Cyclopropyl | 1 | |
| 7.30 | H | H | 3-Cl | H | 2-Cyclopropyl | 1 | |
| 7.31 | 3-F | H | 3-F | H | 2-Cyclopropyl | 1 | |
| 7.32 | 3-Cl | H | 3-F | H | 2-Cyclopropyl | 1 | |

Tabelle 7 (Fortsetzung)

| Verbindung Nr. | $R^1$ | $R^2$ | $R^3$ | $R^4$ | $R^5$ | n | phys. Daten (Fp. °C) |
|---|---|---|---|---|---|---|---|
| 7.33 | 3-Br | H | 3-F | H | 2-Cyclopropyl | 1 | |
| 7.34 | H | H | H | $CH_3$ | 2-Cyclopropyl | 1 | |
| 7.35 | 3-Br | H | H | $CH_3$ | 2-Cyclopropyl | 1 | |
| 7.36 | 3-$C_2H_5$ | H | H | $CH_3$ | 2-Cyclopropyl | 1 | |
| 7.37 | 3-$OCH_3$ | H | H | $CH_3$ | 2-Cyclopropyl | 1 | |
| 7.38 | 3-Cl | 4-F | H | H | 2-Cyclopropyl | 1 | |
| 7.39 | H | H | H | H | 2-$OCH_3$ | 1 | |
| 7.40 | H | H | H | H | 2-$OC_2H_5$ | 1 | |
| 7.41 | 3-F | H | H | $CH_3$ | 2-$OC_2H_5$ | 1 | |
| 7.42 | 3-Cl | H | H | H | 2-$OC_2H_5$ | 1 | |
| 7.43 | 3-Br | H | H | $CH_3$ | 2-$OC_2H_5$ | 1 | |
| 7.44 | 3-$CF_3$ | H | H | H | 2-$OC_2H_5$ | 1 | |
| 7.45 | 3-$CH_3$ | H | H | $CH_3$ | 2-$OC_2H_5$ | 1 | |
| 7.46 | 3-$C_2H_5$ | H | H | H | 2-$OC_2H_5$ | 1 | |
| 7.47 | 3-$OCH_3$ | H | H | $CH_3$ | 2-$OC_2H_5$ | 1 | |
| 7.48 | 3-Cl | 3-F | H | H | 2-$OC_2H_5$ | 1 | |
| 7.49 | H | H | H | H | 2-Cl | 1 | |
| 7.50 | 3-F | H | H | $CH_3$ | 2-Cl | 1 | |
| 7.51 | 3-Cl | H | H | H | 2-Cl | 1 | |
| 7.52 | 3-Br | H | H | $CH_3$ | 2-Cl | 1 | |
| 7.53 | 3-$CF_3$ | H | H | H | 2-Cl | 1 | |
| 7.54 | 3-$CH_3$ | H | H | $CH_3$ | 2-Cl | 1 | |
| 7.55 | 3-$C_2H_5$ | H | H | H | 2-Cl | 1 | |
| 7.56 | 3-$OCH_3$ | H | H | $CH_3$ | 2-Cl | 1 | |
| 7.57 | 3-Cl | 4-F | H | H | 2-Cl | 1 | |
| 7.61 | H | H | H | $CH_3$ | 2-Cyclopropyl | 1 | |
| 7.62 | H | H | H | $CH_3$ | 2-$OCH_3$ | 1 | |
| 7.63 | H | H | H | $CH_3$ | 2-$OC_2H_5$ | 1 | |
| 7.64 | H | H | H | $CH_3$ | 2-Cl | 1 | |

Tabelle 8

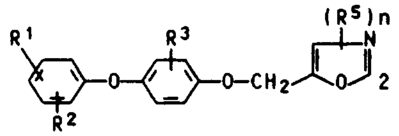

| Verbindung Nr. | $R^1$ | $R^2$ | $R^3$ | $R^5$ | n | phys. Daten (Fp. °C) |
|---|---|---|---|---|---|---|
| 8.1 | H | H | H | - | 0 | |
| 8.2 | 3-F | H | H | - | 0 | |
| 8.3 | 3-Cl | H | H | - | 0 | |
| 8.4 | 3-Br | H | H | - | 0 | |
| 8.5 | 3-$CF_3$ | H | H | - | 0 | |
| 8.6 | 3-$CH_3$ | H | H | - | 0 | |
| 8.7 | 3-$C_2H_5$ | H | H | - | 0 | |
| 8.8 | 3-$OCH_3$ | H | H | - | 0 | |
| 8.9 | 3-Cl | 4-F | H | - | 0 | |
| 8.10 | H | H | H | 2-$CH_3$ | 1 | |
| 8.11 | 3-F | H | H | 2-$CH_3$ | 1 | |
| 8.12 | 3-Cl | H | H | 2-$CH_3$ | 1 | |
| 8.13 | 3-Br | H | H | 2-$CH_3$ | 1 | |
| 8.14 | 3-$CF_3$ | H | H | 2-$CH_3$ | 1 | |
| 8.15 | 3-$CH_3$ | H | H | 2-$CH_3$ | 1 | |
| 8.16 | 3-$C_2H_5$ | H | H | 2-$CH_3$ | 1 | |
| 8.17 | 3-$OCH_3$ | H | H | 2-$CH_3$ | 1 | |
| 8.18 | 3-Cl | 4-F | H | 2-$CH_3$ | 1 | |
| 8.19 | H | H | 3-F | 2-$CH_3$ | 1 | |
| 8.20 | H | H | 3-Cl | 2-$CH_3$ | 1 | |
| 8.21 | 3-F | H | 3-F | 2-$CH_3$ | 1 | |
| 8.22 | 3-F | H | 3-Cl | 2-$CH_3$ | 1 | |
| 8.23 | 3-Cl | H | 3-F | 2-$CH_3$ | 1 | |
| 8.24 | 3-Cl | H | 3-Cl | 2-$CH_3$ | 1 | |
| 8.25 | 3-$CH_3$ | H | 3-F | 2-$CH_3$ | 1 | |
| 8.26 | 3-$CH_3$ | H | 3-Cl | 2-$CH_3$ | 1 | |
| 8.27 | H | H | H | 2-Cyclopropyl | 1 | |
| 8.28 | 3-F | H | H | 2-Cyclopropyl | 1 | |
| 8.29 | 3-Cl | H | H | 2-Cyclopropyl | 1 | |
| 8.30 | 3-Br | H | H | 2-Cyclopropyl | 1 | |
| 8.31 | 3-$CF_3$ | H | H | 2-Cyclopropyl | 1 | |
| 8.32 | 3-$CH_3$ | H | H | 2-Cyclopropyl | 1 | |
| 8.33 | 3-$C_2H_5$ | H | H | 2-Cyclopropyl | 1 | |

Tabelle 8 (Fortsetzung)

| Verbindung Nr. | $R^1$ | $R^2$ | $R^3$ | $R^5$ | n | phys. Daten (Fp. °C) |
|---|---|---|---|---|---|---|
| 8.34 | 3-$OCH_3$ | H | H | 2-Cyclopropyl | 1 | |
| 8.35 | 3-Cl | 4-F | H | 2-Cyclopropyl | 1 | |
| 8.36 | H | H | H | 2-$OCH_3$ | 1 | |
| 8.37 | H | H | H | 2-$OC_2H_5$ | 1 | |
| 8.38 | 3-F | H | H | 2-$OC_2H_5$ | 1 | |
| 8.39 | 3-Cl | H | H | 2-$OC_2H_5$ | 1 | |
| 8.40 | 3-Br | H | H | 2-$OC_2H_5$ | 1 | |
| 8.41 | 3-$CF_3$ | H | H | 2-$OC_2H_5$ | 1 | |
| 8.42 | 3-$CH_3$ | H | H | 2-$OC_2H_5$ | 1 | |
| 8.42 | 3-$C_2H_5$ | H | H | 2-$OC_2H_5$ | 1 | |
| 8.43 | 3-$OCH_3$ | H | H | 2-$OC_2H_5$ | 1 | |
| 8.44 | 3-Cl | 4-F | H | 2-$OC_2H_5$ | 1 | |

Tabelle 9

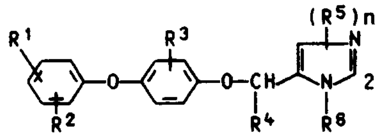

| Verbindung Nr. | $R^1$ | $R^2$ | $R^3$ | $R^4$ | $R^5$ | $R^6$ | n | phys. Daten (Fp. °C) |
|---|---|---|---|---|---|---|---|---|
| 9.1 | H | H | H | H | 4-$CH_3$ | $CH_3$ | 1 | |
| 9.2 | H | H | H | $CH_3$ | 4-$CH_3$ | $CH_3$ | 1 | |
| 9.3 | 3-F | H | H | H | 4-$CH_3$ | $CH_3$ | 1 | |
| 9.4 | 3-Cl | H | H | H | 4-$CH_3$ | $CH_3$ | 1 | |
| 9.5 | 3-Br | H | H | H | 4-$CH_3$ | $CH_3$ | 1 | |
| 9.6 | 3-$CF_3$ | H | H | H | 4-$CH_3$ | $CH_3$ | 1 | |
| 9.7 | 3-$CH_3$ | H | H | H | 4-$CH_3$ | $CH_3$ | 1 | |
| 9.8 | 3-$C_2H_5$ | H | H | H | 4-$CH_3$ | $CH_3$ | 1 | |
| 9.9 | 3-$OCH_3$ | H | H | H | 4-$CH_3$ | $CH_3$ | 1 | |
| 9.10 | 3-$OC_2H_5$ | H | H | H | 4-$CH_3$ | $CH_3$ | 1 | |
| 9.11 | 4-F | H | H | H | 4-$CH_3$ | $CH_3$ | 1 | |
| 9.12 | 3-F | H | H | H | 2,4-Dimethyl | $CH_3$ | 2 | |
| 9.13 | 3-Cl | H | H | H | 2,4-Dimethyl | $CH_3$ | 2 | |
| 9.14 | 3-Br | H | H | H | 2,4-Dimethyl | $CH_3$ | 2 | |
| 9.15 | 3-$CF_3$ | H | H | H | 2,4-Dimethyl | $CH_3$ | 2 | |
| 9.16 | 3-$CH_3$ | H | H | H | 2,4-Dimethyl | $CH_3$ | 2 | |
| 9.17 | 3-$C_2H_5$ | H | H | H | 2,4-Dimethyl | $CH_3$ | 2 | |
| 9.18 | 3-$OCH_3$ | H | H | H | 2,4-Dimethyl | $CH_3$ | 2 | |
| 9.19 | 3-$OC_2H_5$ | H | H | H | 2,4-Dimethyl | $CH_3$ | 2 | |
| 9.20 | 4-F | H | H | H | 2,4-Dimethyl | $CH_3$ | 2 | |
| 9.21 | 3-F | H | H | $CH_3$ | - | $CH_3$ | 0 | |
| 9.22 | 3-Cl | H | H | $CH_3$ | - | $CH_3$ | 0 | |
| 9.23 | 3-Br | H | H | $CH_3$ | - | $CH_3$ | 0 | |
| 9.24 | 3-$CF_3$ | H | H | $CH_3$ | - | $CH_3$ | 0 | |
| 9.25 | 3-$CH_3$ | H | H | $CH_3$ | - | $CH_3$ | 0 | |
| 9.26 | 3-$C_2H_5$ | H | H | $CH_3$ | - | $CH_3$ | 0 | |
| 9.27 | 3-$OCH_3$ | H | H | $CH_3$ | - | $CH_3$ | 0 | |
| 9.28 | 3-$OC_2H_5$ | H | H | $CH_3$ | - | $CH_3$ | 0 | |
| 9.29 | 4-F | H | H | $CH_3$ | - | $CH_3$ | 0 | |
| 9.30 | 3-F | H | H | $CH_3$ | 2-$CH_3$ | $CH_3$ | 1 | |
| 9.31 | 3-Cl | H | H | $CH_3$ | 2-$CH_3$ | $CH_3$ | 1 | |
| 9.32 | 3-Br | H | H | $CH_3$ | 2-$CH_3$ | $CH_3$ | 1 | |
| 9.33 | 3-$CF_3$ | H | H | $CH_3$ | 2-$CH_3$ | $CH_3$ | 1 | |

Tabelle 9 (Fortsetzung)

| Verbindung Nr. | $R^1$ | $R^2$ | $R^3$ | $R^4$ | $R^5$ | $R^6$ | n | phys. Daten (Fp. °C) |
|---|---|---|---|---|---|---|---|---|
| 9.34 | 3-$CH_3$ | H | H | $CH_3$ | 2-$CH_3$ | $CH_3$ | 1 | |
| 9.35 | 3-$C_2H_5$ | H | H | $CH_3$ | 2-$CH_3$ | $CH_3$ | 1 | |
| 9.36 | 3-$OCH_3$ | H | H | $CH_3$ | 2-$CH_3$ | $CH_3$ | 1 | |
| 9.37 | 3-$OC_2H_5$ | H | H | $CH_3$ | 2-$CH_3$ | $CH_3$ | 1 | |
| 9.38 | 4-F | H | H | $CH_3$ | 5-$CH_3$ | $CH_3$ | 1 | |
| 9.39 | 3-F | H | 3-F | H | 4-$CH_3$ | $CH_3$ | 1 | |
| 9.40 | 3-Cl | H | 3-F | H | 4-$CH_3$ | $CH_3$ | 1 | |
| 9.41 | 3-Br | H | 3-F | H | 4-$CH_3$ | $CH_3$ | 1 | |
| 9.42 | 3-$CF_3$ | H | 3-F | H | 4-$CH_3$ | $CH_3$ | 1 | |
| 9.43 | 3-$CH_3$ | H | 3-F | H | 4-$CH_3$ | $CH_3$ | 1 | |
| 9.44 | 3-$C_2H_5$ | H | 3-F | H | 4-$CH_3$ | $CH_3$ | 1 | |
| 9.45 | 3-$OCH_3$ | H | 3-F | H | 4-$CH_3$ | $CH_3$ | 1 | |
| 9.46 | 3-$OC_2H_5$ | H | 3-F | H | 4-$CH_3$ | $CH_3$ | 1 | |
| 9.47 | 4-F | H | 3-F | H | 4-$CH_3$ | $CH_3$ | 1 | |
| 9.48 | H | H | H | H | 2-Cyclopropyl | $CH_3$ | 1 | |
| 9.49 | 3-F | H | H | H | 2-Cyclopropyl | $CH_3$ | 1 | |
| 9.50 | 3-Cl | H | H | H | 2-Cyclopropyl | $CH_3$ | 1 | |
| 9.51 | 3-Br | H | H | H | 2-Cyclopropyl | $CH_3$ | 1 | |
| 9.52 | 3-$CF_3$ | H | 3 | H | 2-Cyclopropyl | $CH_3$ | 1 | |
| 9.53 | 3-$CH_3$ | H | H | H | 2-Cyclopropyl | $CH_3$ | 1 | |
| 9.54 | 3-$C_2H_5$ | H | H | H | 2-Cyclopropyl | $CH_3$ | 1 | |
| 9.55 | 3-$OCH_3$ | H | H | H | 2-Cyclopropyl | $CH_3$ | 1 | |
| 9.56 | 4-F | H | H | H | 2-Cyclopropyl | $CH_3$ | 1 | |
| 9.57 | H | H | 3-F | H | 2-Cyclopropyl | $CH_3$ | 1 | |
| 9.58 | H | H | 3-Cl | H | 2-Cyclopropyl | $CH_3$ | 1 | |

Tabelle 10

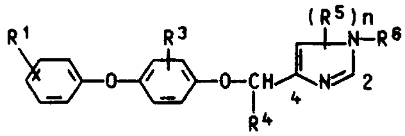

| Verbindung Nr. | $R^1$ | $R^3$ | $R^4$ | $R^5$ | $R^6$ | n | phys. Daten (Fp. °C) |
|---|---|---|---|---|---|---|---|
| 10.1 | H | H | H | - | $CH_3$ | 0 | |
| 10.2 | H | H | $CH_3$ | 5-$CH_3$ | $CH_3$ | 1 | |
| 10.3 | 3-F | H | H | 5-$CH_3$ | $CH_3$ | 1 | |
| 10.4 | 3-Cl | H | H | 5-$CH_3$ | $CH_3$ | 1 | |
| 10.5 | 3-Br | H | H | 5-$CH_3$ | $CH_3$ | 1 | |
| 10.6 | 3-$CF_3$ | H | H | 5-$CH_3$ | $CH_3$ | 1 | |
| 10.7 | 3-$CH_3$ | H | H | 5-$CH_3$ | $CH_3$ | 1 | |
| 10.8 | 3-$C_2H_5$ | H | H | 5-$CH_3$ | $CH_3$ | 1 | |
| 10.9 | 3-$OCH_3$ | H | H | 5-$CH_3$ | $CH_3$ | 1 | |
| 10.10 | 3-$OC_2H_5$ | H | H | 5-$CH_3$ | $CH_3$ | 1 | |
| 10.11 | 4-F | H | H | 5-$CH_3$ | $CH_3$ | 1 | |
| 10.12 | 3-F | H | H | 2-$CH_3$ | $CH_3$ | 1 | |
| 10.13 | 3-Cl | H | H | 2-$CH_3$ | $CH_3$ | 1 | |
| 10.14 | 3-Br | H | H | 2-$CH_3$ | $CH_3$ | 1 | |
| 10.15 | 3-$CF_3$ | H | H | 2-$CH_3$ | $CH_3$ | 1 | |
| 10.16 | 3-$CH_3$ | H | H | 2-$CH_3$ | $CH_3$ | 1 | |
| 10.17 | 3-$C_2H_5$ | H | H | 2-$CH_3$ | $CH_3$ | 1 | |
| 10.18 | 3-$OCH_3$ | H | H | 2-$CH_3$ | $CH_3$ | 1 | |
| 10.19 | 3-$OC_2H_5$ | H | H | 2-$CH_3$ | $CH_3$ | 1 | |
| 10.20 | 4-F | H | H | - | $CH_3$ | 0 | |
| 10.21 | 3-F | H | $CH_3$ | 5-$CH_3$ | $CH_3$ | 1 | |
| 10.22 | 3-Cl | H | $CH_3$ | 5-$CH_3$ | $CH_3$ | 1 | |
| 10.23 | 3-Br | H | $CH_3$ | 5-$CH_3$ | $CH_3$ | 1 | |
| 10.24 | 3-$CF_3$ | H | $CH_3$ | 5-$CH_3$ | $CH_3$ | 1 | |
| 10.25 | 3-$CH_3$ | H | $CH_3$ | 5-$CH_3$ | $CH_3$ | 1 | |
| 10.26 | 3-$C_2H_5$ | H | $CH_3$ | 5-$CH_3$ | $CH_3$ | 1 | |
| 10.27 | 3-$OCH_3$ | H | $CH_3$ | 5-$CH_3$ | $CH_3$ | 1 | |
| 10.28 | 3-$OC_2H_5$ | H | $CH_3$ | 5-$CH_3$ | $CH_3$ | 1 | |
| 10.29 | 4-F | H | $CH_3$ | 5-$CH_3$ | $CH_3$ | 1 | |
| 10.30 | 3-F | H | $CH_3$ | 2-$CH_3$ | $CH_3$ | 1 | |
| 10.31 | 3-Cl | H | $CH_3$ | 2-$CH_3$ | $CH_3$ | 1 | |
| 10.32 | 3-Br | H | $CH_3$ | 2-$CH_3$ | $CH_3$ | 1 | |
| 10.33 | 3-$CF_3$ | H | $CH_3$ | 2-$CH_3$ | $CH_3$ | 1 | |

Tabelle 10 (Fortsetzung)

| Verbindung Nr. | $R^1$ | $R^3$ | $R^4$ | $R^5$ | $R^6$ | n | phys. Daten (Fp. °C) |
|---|---|---|---|---|---|---|---|
| 10.34 | 3-$CH_3$ | H | $CH_3$ | 2-$CH_3$ | $CH_3$ | 1 | |
| 10.35 | 3-$C_2H_5$ | H | $CH_3$ | 2-$CH_3$ | $CH_3$ | 1 | |
| 10.36 | 3-$OCH_3$ | H | $CH_3$ | 2-$CH_3$ | $CH_3$ | 1 | |
| 10.37 | 3-$OC_2H_5$ | H | $CH_3$ | 2-$CH_3$ | $CH_3$ | 1 | |
| 10.38 | 4-F | H | $CH_3$ | 2-$CH_3$ | $CH_3$ | 1 | |
| 10.39 | 3-F | 3-F | H | 5-$CH_3$ | $CH_3$ | 1 | |
| 10.40 | 3-Cl | 3-F | H | 5-$CH_3$ | $CH_3$ | 1 | |
| 10.41 | 3-Br | 3-F | H | 5-$CH_3$ | $CH_3$ | 1 | |
| 10.42 | 3-$CF_3$ | 3-F | H | 5-$CH_3$ | $CH_3$ | 1 | |
| 10.43 | 3-$CH_3$ | 3-F | H | 5-$CH_3$ | $CH_3$ | 1 | |
| 10.44 | 3-$C_2H_5$ | 3-F | H | 5-$CH_3$ | $CH_3$ | 1 | |
| 10.45 | 3-$OCH_3$ | 3-F | H | 5-$CH_3$ | $CH_3$ | 1 | |
| 10.46 | 3-$OC_2H_5$ | 3-F | H | 5-$CH_3$ | $CH_3$ | 1 | |
| 10.47 | 4-F | 3-F | H | 5-$CH_3$ | $CH_3$ | 1 | |
| 10.48 | H | H | H | 2-Cyclopropyl | $CH_3$ | 1 | |
| 10.49 | 3-F | H | H | 2-Cyclopropyl | $CH_3$ | 1 | |
| 10.50 | 3-Cl | H | H | 2-Cyclopropyl | $CH_3$ | 1 | |
| 10.51 | 3-Br | H | H | 2-Cyclopropyl | $CH_3$ | 1 | |
| 10.52 | 3-$CF_3$ | H | H | 2-Cyclopropyl | $CH_3$ | 1 | |
| 10.53 | 3-$CH_3$ | H | H | 2-Cyclopropyl | $CH_3$ | 1 | |
| 10.54 | 3-$C_2H_5$ | H | H | 2-Cyclopropyl | $CH_3$ | 1 | |
| 10.55 | 3-$OCH_2$ | H | H | 2-Cyclopropyl | $CH_3$ | 1 | |
| 10.56 | H | H | H | 2-Cyclopropyl | Cyclopropyl | 1 | |
| 10.57 | H | 3-F | H | 2-Cyclopropyl | $CH_3$ | 1 | |
| 10.58 | 3-$C_2H_5$ | 3-F | H | 2-Cyclopropyl | $CH_3$ | 1 | |
| 10.59 | H | H | H | 2-$CH_3$ | Cyclopropyl | 1 | |
| 10.60 | 3-$C_2H_5$ | H | H | 2-$CH_3$ | Cyclopropyl | 1 | |

Tabelle 11

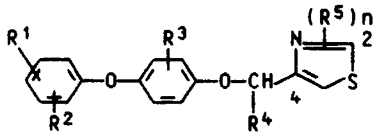

| Verbindung Nr. | $R^1$ | $R^2$ | $R^3$ | $R^4$ | $R^5$ | n | phys. Daten (Fp. °C) |
|---|---|---|---|---|---|---|---|
| 11.1 | H | H | H | H | - | 0 | |
| 11.2 | H | H | H | $CH_3$ | - | 0 | |
| 11.3 | 3-F | H | H | H | - | 0 | |
| 11.4 | 3-Cl | H | H | H | - | 0 | |
| 11.5 | 3-Br | H | H | H | - | 0 | |
| 11.6 | 3-$CF_3$ | H | H | H | - | 0 | |
| 11.7 | 3-$CH_3$ | H | H | H | - | 0 | |
| 11.8 | 3-$C_2H_5$ | H | H | H | - | 0 | |
| 11.9 | 3-$OCH_3$ | H | H | H | - | 0 | |
| 11.10 | 3-Cl | 4-F | H | H | - | 0 | |
| 11.11 | H | H | H | H | 2-$CH_3$ | 1 | 67-68 |
| 11.12 | H | H | H | $CH_3$ | 2-$CH_3$ | 1 | |
| 11.13 | 3-F | H | H | H | 2-$CH_3$ | 1 | 300-MHz $^1H$-NMR in $CDCl_3$ [ppm]: 2,72(s) |
| 11.14 | 3-Cl | H | H | H | 2-$CH_3$ | 1 | |
| 11.15 | 3-Br | H | H | H | 2-$CH_3$ | 1 | |
| 11.16 | 3-$CF_3$ | H | H | H | 2-$CH_3$ | 1 | 73-74 |
| 11.17 | 3-$CH_3$ | H | H | H | 2-$CH_3$ | 1 | |
| 11.18 | 3-$C_2H_5$ | H | H | H | 2-$CH_3$ | 1 | |
| 11.19 | 3-$OCH_3$ | H | H | H | 2-$CH_3$ | 1 | |
| 11.20 | 3-Cl | 4-F | H | H | 2-$CH_3$ | 1 | 96-99 |
| 11.21 | 3-F | H | 3-F | H | 2-$CH_3$ | 1 | |
| 11.22 | 3-Cl | H | 3-Cl | H | 2-$CH_3$ | 1 | |
| 11.23 | 3-$CF_3$ | H | 3-F | H | 2-$CH_3$ | 1 | |
| 11.24 | 3-$CH_3$ | H | 3-Cl | H | 2-$CH_3$ | 1 | |
| 11.25 | 3-$C_2H_5$ | H | 3-F | H | 2-$CH_3$ | 1 | |
| 11.26 | 3-$OCH_3$ | H | 3-Cl | H | 2-$CH_3$ | 1 | |
| 11.27 | 3-Cl | 4-F | 3-F | H | 2-$CH_3$ | 1 | |
| 11.28 | H | H | H | H | 2-Cyclopropyl | 1 | 300-MHz-$^1H$-NMR in $CDCl_3$ [ppm]: 5,08 (s) |
| 11.29 | H | H | H | $CH_3$ | 2-Cyclopropyl | 1 | |
| 11.30 | 3-F | H | H | H | 2-Cyclopropyl | 1 | |
| 11.31 | 3-Cl | H | H | H | 2-Cyclopropyl | 1 | |

Tabelle 11 (Fortsetzung)

| Verbindung Nr. | $R^1$ | $R^2$ | $R^3$ | $R^4$ | $R^5$ | n | phys. Daten (Fp. °C) |
|---|---|---|---|---|---|---|---|
| 11.32 | 3-Br | H | H | H | 2-Cyclopropyl | 1 | |
| 11.33 | 3-$CF_3$ | H | H | H | 2-Cyclopropyl | 1 | |
| 11.34 | 3-$CH_3$ | H | H | H | 2-Cyclopropyl | 1 | |
| 11.35 | 3-$C_2H_5$ | H | H | H | 2-Cyclopropyl | 1 | |
| 11.36 | 3-$OCH_3$ | H | H | H | 2-Cyclopropyl | 1 | |
| 11.37 | 3-Cl | 4-F | H | H | 2-Cyclopropyl | 1 | |
| 11.38 | 3-F | H | 3-F | H | 2-Cyclopropyl | 1 | |
| 11.39 | 3-Cl | H | 3-Cl | H | 2-Cyclopropyl | 1 | |
| 11.40 | 3-$CF_3$ | H | 3-F | H | 2-Cyclopropyl | 1 | |
| 11.41 | 3-$CH_3$ | H | 3-Cl | H | 2-Cyclopropyl | 1 | |
| 11.42 | 3-$C_2H_5$ | H | 3-F | H | 2-Cyclopropyl | 1 | |
| 11.43 | 3-$OCH_3$ | H | 3-Cl | H | 2-Cyclopropyl | 1 | |
| 11.44 | 3-Cl | 4-F | 3-F | H | 2-Cyclopropyl | 1 | |
| 11.45 | H | H | H | H | 2-Cl | 1 | |
| 11.46 | H | H | H | $CH_3$ | 2-Cl | 1 | |
| 11.47 | 3-F | H | H | H | 2-Cl | 1 | |
| 11.48 | 3-Cl | H | H | H | 2-Cl | 1 | |
| 11.49 | 3-Br | H | H | H | 2-Cl | 1 | |
| 11.50 | 3-$CF_3$ | H | H | H | 2-Cl | 1 | |
| 11.51 | 3-$CH_3$ | H | H | H | 2-Cl | 1 | |
| 11.52 | 3-$C_2H_5$ | H | H | H | 2-Cl | 1 | |
| 11.53 | 3-$OCH_3$ | H | H | H | 2-Cl | 1 | |
| 11.54 | 3-Cl | 4-F | H | H | 2-Cl | 1 | |
| 11.55 | H | H | H | H | 2-$OCH_3$ | 1 | |
| 11.56 | H | H | H | $CH_3$ | 2-$OCH_3$ | 1 | |
| 11.57 | H | H | H | H | 2-$OC_2H_5$ | 1 | |
| 11.58 | H | H | H | $CH_3$ | 2-$OC_2H_5$ | 1 | |
| 11.59 | 3-F | H | H | H | 2-$OC_2H_5$ | 1 | |
| 11.60 | 3-Cl | H | H | H | 2-$OC_2H_5$ | 1 | |
| 11.61 | 3-Br | H | H | H | 2-$OC_2H_5$ | 1 | |
| 11.62 | 3-$CF_3$ | H | H | H | 2-$OC_2H_5$ | 1 | |
| 11.63 | 3-$CH_3$ | H | H | H | 2-$OC_2H_5$ | 1 | |
| 11.64 | 3-$C_2H_5$ | H | H | H | 2-$OC_2H_5$ | 1 | |
| 11.65 | 3-$OCH_3$ | H | H | H | 2-$OC_2H_5$ | 1 | |
| 11.66 | 3-Cl | 4-F | H | H | 2-$OC_2H_5$ | 1 | |

Tabelle 12

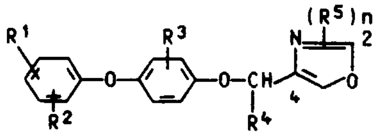

| Verbindung Nr. | $R^1$ | $R^2$ | $R^3$ | $R^4$ | $R^5$ | n | phys. Daten (Fp. °C) |
|---|---|---|---|---|---|---|---|
| 12.1 | H | H | H | H | - | 0 | |
| 12.2 | H | H | H | $CH_3$ | - | 0 | |
| 12.3 | 3-F | H | H | H | - | 0 | |
| 12.4 | 3-Cl | H | H | H | - | 0 | |
| 12.5 | 3-Br | H | H | H | - | 0 | |
| 12.6 | 3-$CF_3$ | H | H | H | - | 0 | |
| 12.7 | 3-$CH_3$ | H | H | H | - | 0 | |
| 12.8 | 3-$C_2H_5$ | H | H | H | - | 0 | |
| 12.9 | 3-$OCH_3$ | H | H | H | - | 0 | |
| 12.10 | 3-Cl | 4-F | H | H | - | 0 | |
| 12.11 | H | H | H | H | 2-$CH_3$ | 1 | |
| 12.12 | H | H | H | $CH_3$ | 2-$CH_3$ | 1 | |
| 12.13 | 3-F | H | H | H | 2-$CH_3$ | 1 | |
| 12.14 | 3-Cl | H | H | H | 2-$CH_3$ | 1 | |
| 12.15 | 3-Br | H | H | H | 2-$CH_3$ | 1 | |
| 12.16 | 3-$CF_3$ | H | H | H | 2-$CH_3$ | 1 | |
| 12.17 | 3-$CH_3$ | H | H | H | 2-$CH_3$ | 1 | |
| 12.18 | 3-$C_2H_5$ | H | H | H | 2-$CH_3$ | 1 | |
| 12.19 | 3-$OCH_3$ | H | H | H | 2-$CH_3$ | 1 | |
| 12.20 | 3-Cl | 4-F | H | H | 2-$CH_3$ | 1 | |
| 12.21 | H | H | 3-F | H | 2-$CH_3$ | 1 | |
| 12.22 | H | H | 3-Cl | H | 2-$CH_3$ | 1 | |
| 12.23 | 3-Cl | H | 3-F | H | 2-$CH_3$ | 1 | |
| 12.24 | 3-Cl | H | 3-Cl | H | 2-$CH_3$ | 1 | |
| 12.25 | 3-$CH_3$ | H | 3-F | H | 2-$CH_3$ | 1 | |
| 12.26 | 3-$CH_3$ | H | 3-Cl | H | 2-$CH_3$ | 1 | |
| 12.27 | H | H | H | H | 2-Cyclopropyl | 1 | |
| 12.28 | H | H | H | $CH_3$ | 2-Cyclopropyl | 1 | |
| 12.29 | 3-F | H | H | H | 2-Cyclopropyl | 1 | |
| 12.30 | 3-Cl | H | H | H | 2-Cyclopropyl | 1 | |
| 12.31 | 3-Br | H | H | H | 2-Cyclopropyl | 1 | |
| 12.32 | 3-$CF_3$ | H | H | H | 2-Cyclopropyl | 1 | |
| 12.33 | 3-$CH_3$ | H | H | H | 2-Cyclopropyl | 1 | |

Tabelle 12 (Fortsetzung)

| Verbindung Nr. | $R^1$ | $R^2$ | $R^3$ | $R^4$ | $R^5$ | n | phys. Daten (Fp. °C) |
|---|---|---|---|---|---|---|---|
| 12.34 | 3-$C_2H_5$ | H | H | H | 2-Cyclopropyl | 1 | |
| 12.35 | 3-$OCH_3$ | H | H | H | 2-Cyclopropyl | 1 | |
| 12.36 | 3-Cl | 4-F | H | H | 2-Cyclopropyl | 1 | |
| 12.37 | H | H | H | H | 2-$OCH_3$ | 1 | |
| 12.38 | H | H | H | H | 2-$OC_2H_5$ | 1 | |
| 12.39 | 3-F | H | H | H | 2-$OC_2H_5$ | 1 | |
| 12.40 | 3-Cl | H | H | H | 2-$OC_2H_5$ | 1 | |
| 12.41 | 3-Br | H | H | H | 2-$OC_2H_5$ | 1 | |
| 12.42 | 3-$CF_3$ | H | H | H | 2-$OC_2H_5$ | 1 | |
| 12.43 | 3-$CH_3$ | H | H | H | 2-$OC_2H_5$ | 1 | |
| 12.44 | 3-$C_2H_5$ | H | H | H | 2-$OC_2H_5$ | 1 | |
| 12.45 | 3-$OCH_3$ | H | H | H | 2-$OC_2H_5$ | 1 | |
| 12.46 | 3-Cl | 4-F | H | H | 2-$OC_2H_5$ | 1 | |

Tabelle 13

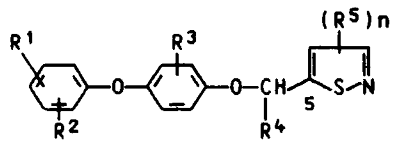

| Verbindung Nr. | $R^1$ | $R^2$ | $R^3$ | $R^4$ | $R^5$ | n | phys. Daten (Fp. °C) |
|---|---|---|---|---|---|---|---|
| 13.1 | H | H | H | H | - | 0 | |
| 13.2 | 3-F | H | H | H | - | 0 | |
| 13.3 | 3-Cl | H | H | H | - | 0 | |
| 13.4 | 3-Br | H | H | H | - | 0 | |
| 13.5 | $3-CF_3$ | H | H | H | - | 0 | |
| 13.6 | $3-CH_3$ | H | H | H | - | 0 | |
| 13.7 | $3-C_2H_5$ | H | H | H | - | 0 | |
| 13.8 | $3-OCH_3$ | H | H | H | - | 0 | |
| 13.9 | 3-Cl | 4-F | H | H | - | 0 | |
| 13.10 | H | H | H | H | $3-CH_3$ | 1 | 61-64 |
| 13.11 | 3-F | H | H | H | $3-CH_3$ | 1 | |
| 13.12 | 3-Cl | H | H | H | $3-CH_3$ | 1 | |
| 13.13 | 3-Br | H | H | H | $3-CH_3$ | 1 | |
| 13.14 | $3-CH_3$ | H | H | H | $3-CH_3$ | 1 | |
| 13.15 | $3-C_2H_5$ | H | H | H | $3-CH_3$ | 1 | |
| 13.16 | $3-OCH_3$ | H | H | H | $3-CH_3$ | 1 | |
| 13.17 | 3-Cl | 4-F | H | H | $3-CH_3$ | 1 | |
| 13.18 | $3-CF_3$ | H | H | H | 3-Cyclopropyl | 1 | |
| 13.19 | 3-F | H | H | H | 3-Cyclopropyl | 1 | |
| 13.20 | 3-Cl | H | H | H | 3-Cyclopropyl | 1 | |
| 13.21 | 3-Br | H | H | H | 3-Cyclopropyl | 1 | |
| 13.22 | $3-CF_3$ | H | H | H | 3-Cyclopropyl | 1 | |
| 13.23 | $3-CH_3$ | H | H | H | 3-Cyclopropyl | 1 | |
| 13.24 | $3-C_2H_5$ | H | H | H | 3-Cyclopropyl | 1 | |
| 13.25 | $3-OCH_3$ | H | H | H | 3-Cyclopropyl | 1 | |
| 13.26 | 3-Cl | 4-F | H | H | 3-Cyclopropyl | 1 | |
| 13.27 | H | H | H | $CH_3$ | $3-CH_3$ | 1 | |
| 13.28 | H | H | 3-F | H | - | 0 | |
| 13.29 | H | H | 3-F | H | $3-CH_3$ | 1 | |
| 13.30 | H | H | 3-F | H | 3-Cyclopropyl | 1 | |
| 13.31 | H | H | H | $CH_3$ | 3-Cyclopropyl | 1 | |
| 13.32 | H | H | H | H | $3-OC_2H_5$ | 1 | |
| 13.33 | 3-F | H | H | H | $3-OC_2H_5$ | 1 | |

Tabelle 13 (Fortsetzung)

| Verbindung Nr. | $R^1$ | $R^2$ | $R^3$ | $R^4$ | $R^5$ | n | phys. Daten (Fp. °C) |
|---|---|---|---|---|---|---|---|
| 13.34 | 3-Cl | H | H | H | $3\text{-}OC_2H_5$ | 1 | |
| 13.35 | 3-Br | H | H | H | $3\text{-}OC_2H_5$ | 1 | |
| 13.36 | $3\text{-}CF_3$ | H | H | H | $3\text{-}OC_2H_5$ | 1 | |
| 13.37 | $3\text{-}CH_3$ | H | H | H | $3\text{-}OC_2H_5$ | 1 | |
| 13.38 | $3\text{-}C_2H_5$ | H | H | H | $3\text{-}OC_2H_5$ | 1 | |
| 13.39 | 3-Cl | 4-F | H | H | $3\text{-}OC_2H_5$ | 1 | |

Tabelle 14

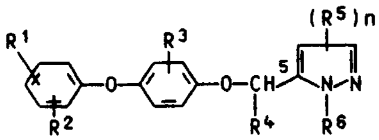

| Verbindung Nr. | $R^1$ | $R^2$ | $R^3$ | $R^4$ | $R^5$ | $R^6$ | n | phys. Daten (Fp. °C) |
|---|---|---|---|---|---|---|---|---|
| 14.1 | H | H | H | H | - | $CH_3$ | 0 | |
| 14.2 | H | H | H | $CH_3$ | 4-$CH_3$ | $CH_3$ | 1 | |
| 14.3 | 3-F | H | H | H | 4-$CH_3$ | $CH_3$ | 1 | |
| 14.4 | 3-Cl | H | H | H | 4-$CH_3$ | $CH_3$ | 1 | |
| 14.5 | 3-Br | H | H | H | 4-$CH_3$ | $CH_3$ | 1 | |
| 14.6 | 3-$CF_3$ | H | H | H | 4-$CH_3$ | $CH_3$ | 1 | |
| 14.7 | 3-$CH_3$ | H | H | H | 4-$CH_3$ | $CH_3$ | 1 | |
| 14.8 | 3-$C_2H_5$ | H | H | H | 4-$CH_3$ | $CH_3$ | 1 | |
| 14.9 | 3-$OCH_3$ | H | H | H | 4-$CH_3$ | $CH_3$ | 1 | |
| 14.10 | 3-$OC_2H_5$ | H | H | H | 4-$CH_3$ | $CH_3$ | 1 | |
| 14.11 | 4-F | H | H | H | 4-$CH_3$ | $CH_3$ | 1 | |
| 14.12 | 3-F | H | H | H | 3-$CH_3$ | $CH_3$ | 1 | |
| 14.13 | 3-Cl | H | H | H | 3-$CH_3$ | $CH_3$ | 1 | |
| 14.14 | 3-Br | H | H | H | 3-$CH_3$ | $CH_3$ | 1 | |
| 14.15 | 3-$CF_3$ | H | H | H | 3-$CH_3$ | $CH_3$ | 1 | |
| 14.16 | 3-$CH_3$ | H | H | H | 3-$CH_3$ | $CH_3$ | 1 | |
| 14.17 | 3-$C_2H_5$ | H | H | H | 3-$CH_3$ | $CH_3$ | 1 | |
| 14.18 | 3-$OCH_3$ | H | H | H | 3-$CH_3$ | $CH_3$ | 1 | |
| 14.19 | 3-$OC_2H_5$ | H | H | H | 5-$CH_3$ | $CH_3$ | 1 | |
| 14.20 | 4-F | H | H | H | 3-$CH_3$ | $CH_3$ | 1 | |
| 14.21 | 3-F | H | H | $CH_3$ | 4-$CH_3$ | $CH_3$ | 1 | |
| 14.22 | 3-Cl | H | H | $CH_3$ | 4-$CH_3$ | $CH_3$ | 1 | |
| 14.23 | 3-Br | H | H | $CH_3$ | 4-$CH_3$ | $CH_3$ | 1 | |
| 14.24 | 3-$CF_3$ | H | H | $CH_3$ | 4-$CH_3$ | $CH_3$ | 1 | |
| 14.25 | 3-$CH_3$ | H | H | $CH_3$ | 4-$CH_3$ | $CH_3$ | 1 | |
| 14.26 | 3-$C_2H_5$ | H | H | $CH_3$ | 4-$CH_3$ | $CH_3$ | 1 | |
| 14.27 | 3-$OCH_3$ | H | H | $CH_3$ | 4-$CH_3$ | $CH_3$ | 1 | |
| 14.28 | 3-$OC_2H_5$ | H | H | $CH_3$ | 4-$CH_3$ | $CH_3$ | 1 | |
| 14.29 | 4-F | H | H | $CH_3$ | 4-$CH_3$ | $CH_3$ | 1 | |
| 14.30 | 3-F | H | H | $CH_3$ | 3-$CH_3$ | $CH_3$ | 1 | |
| 14.31 | 3-Cl | H | H | $CH_3$ | 3-$CH_3$ | $CH_3$ | 1 | |
| 14.32 | 3-Br | H | H | $CH_3$ | 3-$CH_3$ | $CH_3$ | 1 | |
| 14.33 | 3-$CF_3$ | H | H | $CH_3$ | 3-$CH_3$ | $CH_3$ | 1 | |

Tabelle 14 (Fortsetzung)

| Verbindung Nr. | $R^1$ | $R^2$ | $R^3$ | $R^4$ | $R^5$ | $R^6$ | n | phys. Daten (Fp. °C) |
|---|---|---|---|---|---|---|---|---|
| 14.34 | 3-$CH_3$ | H | H | $CH_3$ | 3-$CH_3$ | $CH_3$ | 1 | |
| 14.35 | 3-$C_2H_5$ | H | H | $CH_3$ | 3-$CH_3$ | $CH_3$ | 1 | |
| 14.36 | 3-$OCH_3$ | H | H | $CH_3$ | 3-$CH_3$ | $CH_3$ | 1 | |
| 14.37 | 3-$OC_2H_5$ | H | H | $CH_3$ | 3-$CH_3$ | $CH_3$ | 1 | |
| 14.38 | 4-F | H | H | $CH_3$ | 3-$CH_3$ | $CH_3$ | 1 | |
| 14.39 | 3-F | H | 3-F | H | 4-$CH_3$ | $CH_3$ | 1 | |
| 14.40 | 3-Cl | H | 3-F | H | 4-$CH_3$ | $CH_3$ | 1 | |
| 14.41 | 3-Br | H | 3-F | H | 4-$CH_3$ | $CH_3$ | 1 | |
| 14.42 | 3-$CF_3$ | H | 3-F | H | 4-$CH_3$ | $CH_3$ | 1 | |
| 14.43 | 3-$CH_3$ | H | 3-F | H | 4-$CH_3$ | $CH_3$ | 1 | |
| 14.44 | 3-$C_2H_5$ | H | 3-F | H | 4-$CH_3$ | $CH_3$ | 1 | |
| 14.45 | 3-$OCH_3$ | H | 3-F | H | 4-$CH_3$ | $CH_3$ | 1 | |
| 14.46 | 3-$OC_2H_5$ | H | 3-F | H | 4-$CH_3$ | $CH_3$ | 1 | |
| 14.47 | 4-F | H | 3-F | H | 4-$CH_3$ | $CH_3$ | 1 | |
| 14.48 | H | H | H | H | 3-Cyclopropyl | $CH_3$ | 1 | 87-89 |
| 14.49 | 4-F | H | H | H | 3-Cyclopropyl | $CH_3$ | 1 | |
| 14.50 | 3-Cl | H | H | H | 3-Cyclopropyl | $CH_3$ | 1 | |
| 14.51 | 3-Br | H | H | H | 3-Cyclopropyl | $CH_3$ | 1 | |
| 14.52 | 3-$CF_3$ | H | H | H | 3-Cyclopropyl | $CH_3$ | 1 | 96-99 |
| 14.53 | 3-$OCH_3$ | H | H | H | 3-Cyclopropyl | $CH_3$ | 1 | |
| 14.54 | 3-$CH_3$ | H | H | H | 3-Cyclopropyl | $CH_3$ | 1 | |
| 14.55 | 3-$C_2H_5$ | H | H | H | 3-Cyclopropyl | $CH_3$ | 1 | |
| 14.56 | 3-Cl | 4-F | H | H | 3-Cyclopropyl | $CH_3$ | 1 | |
| 14.57 | H | H | 3-F | H | 3-Cyclopropyl | $CH_3$ | 1 | |
| 14.58 | H | H | H | H | 3-$CH_3$ | Cyclopropyl | 1 | |
| 14.59 | 3-F | H | H | H | 3-Cyclopropyl | $CH_3$ | 1 | 85-87 |

Tabelle 15

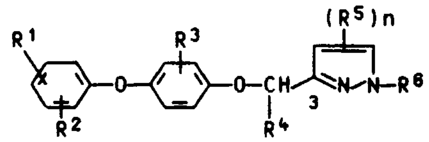

| Verbindung Nr. | $R^1$ | $R^2$ | $R^3$ | $R^4$ | $R^5$ | $R^6$ | n | phys. Daten (Fp. °C) |
|---|---|---|---|---|---|---|---|---|
| 15.1 | H | H | H | H | - | $CH_3$ | 0 | |
| 15.2 | H | H | H | $CH_3$ | 4-$CH_3$ | $CH_3$ | 1 | |
| 15.3 | 3-F | H | H | H | 4-$CH_3$ | $CH_3$ | 1 | |
| 15.4 | 3-Cl | H | H | H | 4-$CH_3$ | $CH_3$ | 1 | |
| 15.5 | 3-Br | H | H | H | 4-$CH_3$ | $CH_3$ | 1 | |
| 15.6 | 3-$CF_3$ | H | H | H | 4-$CH_3$ | $CH_3$ | 1 | |
| 15.7 | 3-$CH_3$ | H | H | H | 4-$CH_3$ | $CH_3$ | 1 | |
| 15.8 | 3-$C_2H_5$ | H | H | H | 4-$CH_3$ | $CH_3$ | 1 | |
| 15.9 | 3-$OCH_3$ | H | H | H | 4-$CH_3$ | $CH_3$ | 1 | |
| 15.10 | 3-$OC_2H_5$ | H | H | H | 4-$CH_3$ | $CH_3$ | 1 | |
| 15.11 | 4-F | H | H | H | 4-$CH_3$ | $CH_3$ | 1 | |
| 15.12 | 3-F | H | H | H | 4-$OCH_3$ | $CH_3$ | 1 | |
| 15.13 | 3-Cl | H | H | H | 4-$OCH_3$ | $CH_3$ | 1 | |
| 15.14 | 3-Br | H | H | H | 4-$OCH_3$ | $CH_3$ | 1 | |
| 15.15 | 3-$CF_3$ | H | H | H | 4-$OCH_3$ | $CH_3$ | 1 | |
| 15.16 | 3-$CH_3$ | H | H | H | 4-$OCH_3$ | $CH_3$ | 1 | |
| 15.17 | 3-$C_2H_5$ | H | H | H | 4-$OCH_3$ | $CH_3$ | 1 | |
| 15.18 | 3-$OCH_3$ | H | H | H | 4-$OCH_3$ | $CH_3$ | 1 | |
| 15.19 | 3-$OC_2H_5$ | H | H | H | 4-$OCH_3$ | $CH_3$ | 1 | |
| 15.20 | 4-F | H | H | H | 4-$OCH_3$ | $CH_3$ | 1 | |
| 15.21 | 3-F | H | H | $CH_3$ | 4-$CH_3$ | $CH_3$ | 1 | |
| 15.22 | 3-Cl | H | H | $CH_3$ | 4-$CH_3$ | $CH_3$ | 1 | |
| 15.23 | 3-Br | H | H | $CH_3$ | 4-$CH_3$ | $CH_3$ | 1 | |
| 15.24 | 3-$CF_3$ | H | H | $CH_3$ | 4-$CH_3$ | $CH_3$ | 1 | |
| 15.25 | 3-$CH_3$ | H | H | $CH_3$ | 4-$CH_3$ | $CH_3$ | 1 | |
| 15.26 | 3-$C_2H_5$ | H | H | $CH_3$ | 4-$CH_3$ | $CH_3$ | 1 | |
| 15.27 | 3-$OCH_3$ | H | H | $CH_3$ | 4-$CH_3$ | $CH_3$ | 1 | |
| 15.28 | 3-$OC_2H_5$ | H | H | $CH_3$ | 4-$CH_3$ | $CH_3$ | 1 | |
| 15.29 | 4-F | H | H | $CH_3$ | 4-$CH_3$ | $CH_3$ | 1 | |
| 15.30 | 3-F | H | H | $CH_3$ | 4-$OCH_3$ | $CH_3$ | 1 | |
| 15.31 | 3-Cl | H | H | $CH_3$ | 4-$OCH_3$ | $CH_3$ | 1 | |
| 15.32 | 3-Br | H | H | $CH_3$ | 4-$OCH_3$ | $CH_3$ | 1 | |
| 15.33 | 3-$CF_3$ | H | H | $CH_3$ | 4-$OCH_3$ | $CH_3$ | 1 | |

Tabelle 15 (Fortsetzung)

| Verbindung Nr. | $R^1$ | $R^2$ | $R^3$ | $R^4$ | $R^5$ | $R^6$ | n | phys. Daten (Fp. °C) |
|---|---|---|---|---|---|---|---|---|
| 15.34 | 3-$CH_3$ | H | H | $CH_3$ | 4-$OCH_3$ | $CH_3$ | 1 | |
| 15.35 | 3-$C_2H_5$ | H | H | $CH_3$ | 4-$OCH_3$ | $CH_3$ | 1 | |
| 15.36 | 3-$OCH_3$ | H | H | $CH_3$ | 4-$OCH_3$ | $CH_3$ | 1 | |
| 15.37 | 3-$OC_2H_5$ | H | H | $CH_3$ | 4-$OCH_3$ | $CH_3$ | 1 | |
| 15.38 | 4-F | H | H | $CH_3$ | 4-$OCH_3$ | $CH_3$ | 1 | |
| 15.39 | 3-F | H | 3-F | H | 5-$CH_3$ | $CH_3$ | 1 | |
| 15.40 | 3-Cl | H | 3-F | H | 5-$CH_3$ | $CH_3$ | 1 | |
| 15.41 | 3-Br | H | 3-F | H | 5-$CH_3$ | $CH_3$ | 1 | |
| 15.42 | 3-$CF_3$ | H | 3-F | H | 5-$CH_3$ | $CH_3$ | 1 | |
| 15.43 | 3-$CH_3$ | H | 3-F | H | 5-$CH_3$ | $CH_3$ | 1 | |
| 15.44 | 3-$C_2H_5$ | H | 3-F | H | 5-$CH_3$ | $CH_3$ | 1 | |
| 15.45 | 3-$OCH_3$ | H | 3-F | H | 5-$CH_3$ | $CH_3$ | 1 | |
| 15.46 | 3-$OC_2H_5$ | H | 3-F | H | 5-$CH_3$ | $CH_3$ | 1 | |
| 15.47 | 4-F | H | 3-F | H | 5-$CH_3$ | $CH_3$ | 1 | |
| 15.48 | H | H | H | H | 5-i-$C_3H_7$ | $CH_3$ | 1 | 300-MHz-$^1$H-NMR in $CDCl_3$ [ppm]: 4,98 (s) |
| 15.49 | H | H | H | H | 5-Cyclopropyl | $CH_3$ | 1 | 57-58 |
| 15.50 | 3-F | H | H | H | 5-Cyclopropyl | $CH_3$ | 1 | 46-48 |
| 15.51 | 3-Cl | H | H | H | 5-Cyclopropyl | $CH_3$ | 1 | |
| 15.52 | 3-Br | H | H | H | 5-Cyclopropyl | $CH_3$ | 1 | |
| 15.53 | 3-$CF_3$ | H | H | H | 5-Cyclopropyl | $CH_3$ | 1 | 57-58 |
| 15.54 | 3-$OCH_3$ | H | H | H | 5-Cyclopropyl | $CH_3$ | 1 | |
| 15.55 | 3-$CH_3$ | H | H | H | 5-Cyclopropyl | $CH_3$ | 1 | |
| 15.56 | 3-$C_2H_5$ | H | H | H | 5-Cyclopropyl | $CH_3$ | 1 | |
| 15.57 | 3-Cl | 4-F | H | H | 5-Cyclopropyl | $CH_3$ | 1 | |
| 15.58 | H | H | H | H | 5-$CH_3$ | Cyclo-propyl | 1 | |
| 15.59 | 3-Br | H | H | H | 5-$CH_3$ | Cyclo-propyl | 1 | |
| 15.60 | 3-$C_2H_5$ | H | H | H | 5-$CH_3$ | Cyclo-propyl | 1 | |

Tabelle 16

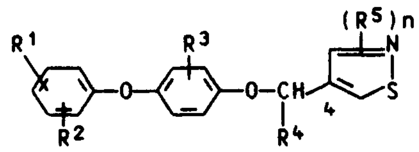

| Verbindung Nr. | $R^1$ | $R^2$ | $R^3$ | $R^4$ | $R^5$ | n | phys. Daten (Fp. °C) |
|---|---|---|---|---|---|---|---|
| 16.1 | H | H | H | H | 3-$CH_3$ | 1 | 57-60 |
| 16.2 | 3-F | H | H | H | 3-$CH_3$ | 1 | |
| 16.3 | 3-Cl | H | H | H | 3-$CH_3$ | 1 | |
| 16.4 | 3-Br | H | H | H | 3-$CH_3$ | 1 | |
| 16.5 | 3-$OCH_3$ | H | H | H | 3-$CH_3$ | 1 | |
| 16.6 | 3-$CF_3$ | H | H | H | 3-$CH_3$ | 1 | |
| 16.7 | 3-$CH_3$ | H | H | H | 3-$CH_3$ | 1 | |
| 16.8 | 3-$C_2H_5$ | H | H | H | 3-$CH_3$ | 1 | |

Tabelle 17

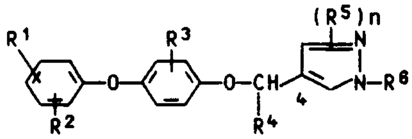

| Verbindung Nr. | $R^1$ | $R^3$ | $R^4$ | $R^5$ | $R^6$ | n | phys. Daten (Fp. °C) |
|---|---|---|---|---|---|---|---|
| 17.1 | H | H | H | - | $CH_3$ | 0 | 90-91 |
| 17.2 | H | H | H | - | Cyclopropyl | 0 | |
| 17.3 | 3-F | H | H | - | Cyclopropyl | 0 | |
| 17.4 | 3-Cl | H | H | - | Cyclopropyl | 0 | |
| 17.5 | 3-Br | H | H | - | Cyclopropyl | 0 | |
| 17.6 | 3-$CF_3$ | H | H | - | Cyclopropyl | 0 | |
| 17.7 | 3-$CH_3$ | H | H | - | Cyclopropyl | 0 | |
| 17.8 | 3-$C_2H_5$ | H | H | - | Cyclopropyl | 0 | |
| 17.9 | 3-$OCH_3$ | H | H | - | Cyclopropyl | 0 | |
| 17.10 | 3-$OC_2H_5$ | H | H | - | Cyclopropyl | 0 | |
| 17.11 | 4-F | H | H | - | Cyclopropyl | 0 | |
| 17.12 | 3-F | H | H | 3-$CH_3$ | Cyclopropyl | 1 | |
| 17.13 | 3-Cl | H | H | 3-$CH_3$ | Cyclopropyl | 1 | |
| 17.14 | 3-Br | H | H | 3-$CH_3$ | Cyclopropyl | 1 | |
| 17.15 | 3-$CF_3$ | H | H | 3-$CH_3$ | Cyclopropyl | 1 | |
| 17.16 | 3-$CH_3$ | H | H | 3-$CH_3$ | Cyclopropyl | 1 | |
| 17.17 | 3-$C_2H_5$ | H | H | 3-$CH_3$ | Cyclopropyl | 1 | |
| 17.18 | 3-$OCH_3$ | H | H | 3-$CH_3$ | Cyclopropyl | 1 | |
| 17.19 | 3-$OC_2H_5$ | H | H | 3-$CH_3$ | Cyclopropyl | 1 | |
| 17.20 | 4-F | H | H | 3-$CH_3$ | Cyclopropyl | 1 | |
| 17.21 | 3-F | H | H | 3-$CH_3$ | $CH_3$ | 1 | |
| 17.22 | 3-Cl | H | H | 3-$CH_3$ | $CH_3$ | 1 | |
| 17.23 | 3-Br | H | H | 3-$CH_3$ | $CH_3$ | 1 | |
| 17.24 | 3-$CF_3$ | H | H | 3-$CH_3$ | $CH_3$ | 1 | |
| 17.25 | 3-$CH_3$ | H | H | 3-$CH_3$ | $CH_3$ | 1 | |
| 17.26 | 3-$C_2H_5$ | H | H | 3-$CH_3$ | $CH_3$ | 1 | |
| 17.27 | 3-$OCH_3$ | H | H | 3-$CH_3$ | $CH_3$ | 1 | |
| 17.28 | 3-$OC_2H_5$ | H | H | 3-$CH_3$ | $CH_3$ | 1 | |
| 17.29 | 4-F | H | H | 3-$CH_3$ | $CH_3$ | 1 | |
| 17.30 | 3-F | H | $CH_3$ | 5-$CH_3$ | $CH_3$ | 1 | |
| 17.31 | 3-Cl | H | $CH_3$ | 5-$CH_3$ | $CH_3$ | 1 | |
| 17.32 | 3-Br | H | $CH_3$ | 5-$CH_3$ | $CH_3$ | 1 | |
| 17.33 | 3-$CF_3$ | H | $CH_3$ | 5-$CH_3$ | $CH_3$ | 1 | |

Tabelle 17 (Fortsetzung)

| Verbindung Nr. | $R^1$ | $R^3$ | $R^4$ | $R^5$ | $R^6$ | n | phys. Daten (Fp. °C) |
|---|---|---|---|---|---|---|---|
| 17.34 | 3-$CH_3$ | H | $CH_3$ | 5-$CH_3$ | $CH_3$ | 1 | |
| 17.35 | 3-$C_2H_5$ | H | $CH_3$ | 5-$CH_3$ | $CH_3$ | 1 | |
| 17.36 | 3-$OCH_3$ | H | $CH_3$ | 5-$CH_3$ | $CH_3$ | 1 | |
| 17.37 | 3-$OC_2H_5$ | H | $CH_3$ | 5-$CH_3$ | $CH_3$ | 1 | |
| 17.38 | 4-F | H | $CH_3$ | 5-$CH_3$ | $CH_3$ | 1 | |
| 17.39 | 3-F | 3-F | H | 5-$CH_3$ | $CH_3$ | 1 | |
| 17.40 | 3-Cl | 3-F | H | 5-$CH_3$ | $CH_3$ | 1 | |
| 17.41 | 3-Br | 3-F | H | 5-$CH_3$ | $CH_3$ | 1 | |
| 17.42 | 3-$CF_3$ | 3-F | H | 5-$CH_3$ | $CH_3$ | 1 | |
| 17.43 | 3-$CH_3$ | 3-F | H | 5-$CH_3$ | $CH_3$ | 1 | |
| 17.44 | 3-$C_2H_5$ | 3-F | H | 5-$CH_3$ | $CH_3$ | 1 | |
| 17.45 | 3-$OCH_3$ | 3-F | H | 5-$CH_3$ | $CH_3$ | 1 | |
| 17.46 | 3-$OC_2H_5$ | 3-F | H | 5-$CH_3$ | $CH_3$ | 1 | |
| 17.47 | 4-F | 3-F | H | 5-$CH_3$ | $CH_3$ | 1 | |

Tabelle 18

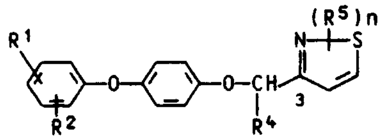

| Verbindung Nr. | $R^1$ | $R^2$ | $R^4$ | $R^5$ | n | phys. Daten (Fp. °C) |
|---|---|---|---|---|---|---|
| 18.1 | H | H | H | - | 0 | |
| 18.2 | H | H | H | 5-$CH_3$ | 1 | |
| 18.3 | 3-F | H | H | 5-$CH_3$ | 1 | |
| 18.4 | 3-Cl | H | H | 5-$CH_3$ | 1 | |
| 18.5 | 3-Br | H | H | 5-$CH_3$ | 1 | |
| 18.6 | 3-$CF_3$ | H | H | 5-$CH_3$ | 1 | |
| 18.7 | 3-$OCH_3$ | H | H | 5-$CH_3$ | 1 | |
| 18.8 | 3-$CH_3$ | H | H | 5-$CH_3$ | 1 | |
| 18.9 | 3-$C_2H_5$ | H | H | 5-$CH_3$ | 1 | |
| 18.10 | H | H | H | 5-Cyclopropyl | 1 | |
| 18.11 | 3-F | H | H | 5-Cyclopropyl | 1 | |
| 18.12 | 3-Cl | H | H | 5-Cyclopropyl | 1 | |
| 18.13 | 3-Br | H | H | 5-Cyclopropyl | 1 | |
| 18.14 | 3-$CF_3$ | H | H | 5-Cyclopropyl | 1 | |
| 18.15 | 3-$OCH_3$ | H | H | 5-Cyclopropyl | 1 | |
| 18.16 | 3-$CH_3$ | H | H | 5-Cyclopropyl | 1 | |
| 18.17 | 3-$C_2H_5$ | H | H | 5-Cyclopropyl | 1 | |
| 18.18 | 3-Cl | 4-F | H | 5-Cyclopropyl | 1 | |
| 18.19 | H | H | H | 5-$OC_2H_5$ | 1 | |
| 18.20 | H | H | $CH_3$ | 5-$CH_3$ | 1 | |
| 18.21 | H | H | $CH_3$ | 5-Cyclopropyl | 1 | |

Tabelle 19

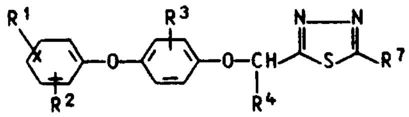

| Verbindung Nr. | $R^1$ | $R^3$ | $R^4$ | $R^7$ | phys. Daten (Fp. °C) |
|---|---|---|---|---|---|
| 19.1 | H | H | H | Cyclopropyl | |
| 19.2 | H | H | $CH_3$ | Cyclopropyl | |
| 19.3 | 3-F | H | H | Cyclopropyl | |
| 19.4 | 3-Cl | H | H | Cyclopropyl | |
| 19.5 | 3-Br | H | H | Cyclopropyl | |
| 19.6 | 3-$CF_3$ | H | H | Cyclopropyl | |
| 19.7 | 3-$CH_3$ | H | H | Cyclopropyl | |
| 19.8 | 3-$C_2H_5$ | H | H | Cyclopropyl | |
| 19.9 | 3-$OCH_3$ | H | H | Cyclopropyl | |
| 19.10 | 3-$OC_2H_5$ | H | H | Cyclopropyl | |
| 19.11 | 4-F | H | $CH_3$ | Cyclopropyl | |
| 19.12 | 3-F | H | $CH_3$ | Cyclopropyl | |
| 19.13 | 3-Cl | H | $CH_3$ | Cyclopropyl | |
| 19.14 | 3-Br | H | $CH_3$ | Cyclopropyl | |
| 19.15 | 3-$CF_3$ | H | $CH_3$ | Cyclopropyl | |
| 19.16 | 3-$CH_3$ | H | $CH_3$ | Cyclopropyl | |
| 19.17 | 3-$C_2H_5$ | H | $CH_3$ | Cyclopropyl | |
| 19.18 | 3-$OCH_3$ | H | $CH_3$ | Cyclopropyl | |
| 19.19 | 3-$OC_2H_5$ | H | $CH_3$ | Cyclopropyl | |
| 19.20 | 4-F | H | H | Cyclopropyl | |
| 19.21 | 3-F | 3-F | H | Cyclopropyl | |
| 19.22 | 3-Cl | 3-F | H | Cyclopropyl | |
| 19.23 | 3-Br | 3-F | H | Cyclopropyl | |
| 19.24 | 3-$CF_3$ | 3-F | H | Cyclopropyl | |
| 19.25 | 3-$CH_3$ | 3-F | H | Cyclopropyl | |
| 19.26 | 3-$C_2H_5$ | 3-F | H | Cyclopropyl | |
| 19.27 | 3-$OCH_3$ | 3-F | H | Cyclopropyl | |
| 19.28 | 3-$OC_2H_5$ | 3-F | H | Cyclopropyl | |
| 19.29 | 4-F | 3-F | H | Cyclopropyl | |
| 19.30 | 3-F | 3-F | $CH_3$ | Cyclopropyl | |
| 19.31 | 3-Cl | 3-F | $CH_3$ | Cyclopropyl | |
| 19.32 | 3-Br | 3-F | $CH_3$ | Cyclopropyl | |
| 19.33 | 3-$CF_3$ | 3-F | $CH_3$ | Cyclopropyl | |
| 19.34 | 3-$CH_3$ | 3-F | $CH_3$ | Cyclopropyl | |

Tabelle 19 (Fortsetzung)

| Verbindung Nr. | $R^1$ | $R^3$ | $R^4$ | $R^7$ | phys. Daten (Fp. °C) |
|---|---|---|---|---|---|
| 19.35 | 3-$C_2H_5$ | 3-F | $CH_3$ | Cyclopropyl | |
| 19.36 | 3-$OCH_3$ | 3-F | $CH_3$ | Cyclopropyl | |
| 19.37 | 3-$OC_2H_5$ | 3-F | $CH_3$ | Cyclopropyl | |
| 19.38 | 4-F | 3-F | $CH_3$ | Cyclopropyl | |
| 19.39 | H | 3-F | $CH_3$ | Cyclopropyl | |
| 19.40 | H | 3-F | H | Cyclopropyl | |

Tabelle 20

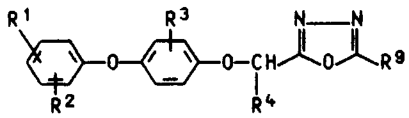

| Verbindung Nr. | $R^1$ | $R^2$ | $R^3$ | $R^4$ | $R^9$ | phys. Daten (Fp. °C) |
|---|---|---|---|---|---|---|
| 20.1 | H | H | H | H | H | |
| 20.2 | H | H | H | $CH_3$ | H | |
| 20.3 | 3-F | H | H | H | H | |
| 20.4 | 3-Cl | H | H | H | H | |
| 20.5 | 3-Br | H | H | H | H | |
| 20.6 | 3-$CF_3$ | H | H | H | H | |
| 20.7 | 3-$CH_3$ | H | H | H | H | |
| 20.8 | 3-$C_2H_5$ | H | H | H | H | |
| 20.9 | 3-$OCH_3$ | H | H | H | H | |
| 20.10 | 3-Cl | 4-F | H | H | H | |
| 20.11 | 3-F | H | 3-F | H | H | |
| 20.12 | 3-Cl | H | 3-F | H | H | |
| 20.13 | 3-$CF_3$ | H | 3-F | H | H | |
| 20.14 | 3-$CH_3$ | H | 3-F | H | H | |
| 20.15 | 3-$OCH_3$ | H | 3-F | H | H | |
| 20.16 | 3-Cl | 4-F | 3-F | H | H | |
| 20.17 | 3-F | H | 3-Cl | H | H | |
| 20.18 | 3-Cl | H | 3-Cl | H | H | |
| 20.19 | 3-Br | H | 3-Cl | H | H | |
| 20.20 | 3-$CF_3$ | H | 3-Cl | H | H | |
| 20.21 | 3-$CH_3$ | H | 3-Cl | H | H | |
| 20.22 | 3-$OCH_3$ | H | 3-Cl | H | H | |
| 20.23 | 3-Cl | 4-F | 3-Cl | H | H | |
| 20.24 | H | H | H | H | $CH_3$ | 78-79 |
| 20.25 | H | H | H | $CH_3$ | $CH_3$ | |
| 20.26 | 3-F | H | H | H | $CH_3$ | |
| 20.27 | 3-Cl | H | H | H | $CH_3$ | |
| 20.28 | 3-Br | H | H | H | $CH_3$ | |
| 20.29 | 3-$CF_3$ | H | H | H | $CH_3$ | |
| 20.30 | 3-$CH_3$ | H | H | H | $CH_3$ | 88-89 |
| 20.31 | 3-$C_2H_5$ | H | H | H | $CH_3$ | |
| 20.32 | 3-$OCH_3$ | H | H | H | $CH_3$ | |
| 20.33 | 3-Cl | 4-F | H | H | $CH_3$ | 81 |
| 20.34 | 3-F | H | 3-F | H | $CH_3$ | |

Tabelle 20 (Fortsetzung)

| Verbindung Nr. | $R^1$ | $R^2$ | $R^3$ | $R^4$ | $R^9$ | phys. Daten (Fp. °C) |
|---|---|---|---|---|---|---|
| 20.35 | 3-Cl | H | 3-F | H | $CH_3$ | |
| 20.36 | 3-$CF_3$ | H | 3-F | H | $CH_3$ | 300 MHz-$^1$H-NMR in $CDCl_3$ [ppm]:2.60(s) |
| 20.37 | 3-$CH_3$ | H | 3-F | H | $CH_3$ | |
| 20.38 | 3-$OCH_3$ | H | 3-F | H | $CH_3$ | |
| 20.39 | 3-Cl | 4-F | 3-F | H | $CH_3$ | |
| 20.40 | 3-F | H | 3-Cl | H | $CH_3$ | |
| 20.41 | 3-Cl | H | 3-Cl | H | $CH_3$ | |
| 20.42 | 3-Br | H | 3-Cl | H | $CH_3$ | |
| 20.43 | 3-$CF_3$ | H | 3-Cl | H | $CH_3$ | |
| 20.44 | 3-$CH_3$ | H | 3-Cl | H | $CH_3$ | |
| 20.45 | 3-$C_2H_5$ | H | 3-Cl | H | $CH_3$ | |
| 20.46 | 3-$OCH_3$ | H | 3-Cl | H | $CH_3$ | |
| 20.47 | 3-Cl | H | 3-Cl | H | $CH_3$ | |
| 20.48 | H | H | H | H | Cyclopropyl | 65-68 |
| 20.49 | H | H | H | $CH_3$ | Cyclopropyl | |
| 20.50 | 3-F | H | H | H | Cyclopropyl | 300-MHz-$^1$NMR in $CDCl_3$ [ppm]:5,17(s) |
| 20.51 | 3-Cl | H | H | H | Cyclopropyl | 300-MHz-$^1$NMR in $CDCl_3$ [ppm]:5,16(s) |
| 20.52 | 3-Br | H | H | H | Cyclopropyl | 61-64 |
| 20.53 | 3-$CF_3$ | H | H | H | Cyclopropyl | 300 MHz-$^1$H-NMR in $CDCl_3$ [ppm]:5.20(s) |
| 20.54 | 3-$CH_3$ | H | H | H | Cyclopropyl | 300 MHz-$^1$H-NMR in $CDCl_3$ [ppm]:2.36(s) |
| 20.55 | 3-$C_2H_5$ | H | H | H | Cyclopropyl | 57-58 |
| 20.56 | 3-$OCH_3$ | H | H | H | Cyclopropyl | 300-MHz-$^1$NMR in $CDCl_3$ [ppm]:3,73(s) |
| 20.57 | 3-Cl | 4-F | H | H | Cyclopropyl | 300 MHz-$^1$H-NMR in $CDCl_3$ [ppm]:5.20(s) |
| 20.58 | 3-F | H | 3-F | H | Cyclopropyl | |
| 20.59 | 3-Cl | H | 3-F | H | Cyclopropyl | |
| 20.60 | 3-Br | H | 3-F | H | Cyclopropyl | |
| 20.61 | 3-$CF_3$ | H | 3-F | H | Cyclopropyl | 300 MHz-$^1$H-NMR in $CDCl_3$ [ppm]:5.49(s) |
| 20.62 | 3-$CH_3$ | H | 3-F | H | Cyclopropyl | |
| 20.63 | 3-$C_2H_5$ | H | 3-F | H | Cyclopropyl | |
| 20.64 | 3-$OCH_3$ | H | 3-F | H | Cyclopropyl | |

Tabelle 20 (Fortsetzung)

| Verbindung Nr. | $R^1$ | $R^2$ | $R^3$ | $R^4$ | $R^9$ | phys. Daten (Fp. °C) |
|---|---|---|---|---|---|---|
| 20.65 | 3-Cl | 4-F | 3-F | H | Cyclopropyl | |
| 20.66 | 3-F | H | 3-Cl | H | Cyclopropyl | |
| 20.67 | 3-Cl | H | 3-Cl | H | Cyclopropyl | |
| 20.68 | 3-Br | H | 3-Cl | H | Cyclopropyl | |
| 20.69 | 3-$CF_3$ | H | 3-Cl | H | Cyclopropyl | |
| 20.70 | 3-$CH_3$ | H | 3-Cl | H | Cyclopropyl | |
| 20.71 | 3-$C_2H_5$ | H | 3-Cl | H | Cyclopropyl | |
| 20.72 | 3-$OCH_3$ | H | 3-Cl | H | Cyclopropyl | |
| 20.73 | 3-Cl | 4-F | 3-Cl | H | Cyclopropyl | |
| 20.74 | H | H | H | H | Cl | |
| 20.75 | H | H | H | H | Br | |
| 20.76 | 3-F | H | H | H | Cl | |
| 20.77 | 3-Cl | H | H | H | Cl | |
| 20.78 | 3-Br | H | H | H | Cl | |
| 20.79 | 3-$CF_3$ | H | H | H | Cl | |
| 20.80 | 3-$CH_3$ | H | H | H | Cl | |
| 20.81 | 3-$C_2H_5$ | H | H | H | Cl | |
| 20.82 | 3-$OCH_3$ | H | H | H | Cl | |
| 20.83 | 3-Cl | 4-F | H | H | Cl | |
| 20.84 | H | H | H | H | $OCH_3$ | |
| 20.85 | H | H | H | H | $OC_2H_5$ | |
| 20.86 | 3-F | H | H | H | $OC_2H_5$ | |
| 20.87 | 3-Cl | H | H | H | $OC_2H_5$ | |
| 20.88 | 3-Br | H | H | H | $OC_2H_5$ | |
| 20.89 | 3-$CF_3$ | H | H | H | $OC_2H_5$ | |
| 20.90 | 3-$CH_3$ | H | H | H | $OC_2H_5$ | |
| 20.91 | 3-$CH_3$ | H | H | H | $OC_2H_5$ | |
| 20.92 | 3-$C_2H_5$ | H | H | H | $OC_2H_5$ | |
| 20.93 | 3-$OCH_3$ | H | H | H | $OC_2H_5$ | |
| 20.94 | 3-Cl | 4-F | H | H | $OC_2H_5$ | |
| 20.95 | 3-F | H | 3-Cl | H | $OC_2H_5$ | |
| 20.96 | 3-F | H | 3-F | H | $OC_2H_5$ | |
| 20.97 | 3-Cl | H | 3-F | H | $OC_2H_5$ | |
| 20.98 | 3-$CF_3$ | H | 3-F | H | $OC_2H_5$ | |
| 20.99 | 3-$OCH_3$ | H | 3-Cl | H | $OC_2H_5$ | |
| 20.100 | H | H | H | H | $C_2H_5$ | 300 MHz $^1$H-NMR in $CDCl_3$ [ppm]: 1,39(t) |
| 20.101 | 4-F | H | H | H | Cyclopropyl | 55-56 |

Tabelle 21

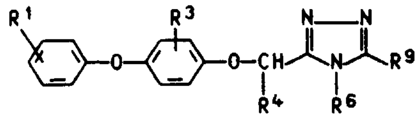

| Verbindung Nr. | $R^1$ | $R^3$ | $R^4$ | $R^9$ | $R^6$ | phys. Daten (Fp. °C) |
|---|---|---|---|---|---|---|
| 21.1 | H | H | H | H | $CH_3$ | |
| 21.2 | H | H | $CH_3$ | H | $CH_3$ | |
| 21.3 | 3-F | H | H | H | $CH_3$ | |
| 21.4 | 3-Cl | H | H | H | $CH_3$ | |
| 21.5 | 3-Br | H | H | H | $CH_3$ | |
| 21.6 | 3-$CF_3$ | H | H | H | $CH_3$ | |
| 21.7 | 3-$CH_3$ | H | H | H | $CH_3$ | |
| 21.8 | 3-$C_2H_5$ | H | H | H | $CH_3$ | |
| 21.9 | 3-$OCH_3$ | H | H | H | $CH_3$ | |
| 21.10 | 3-$OC_2H_5$ | H | H | H | $CH_3$ | |
| 21.11 | 4-F | H | H | H | $CH_3$ | |
| 21.12 | 3-F | H | $CH_3$ | H | $CH_3$ | |
| 21.13 | 3-Cl | H | $CH_3$ | H | $CH_3$ | |
| 21.14 | 3-Br | H | $CH_3$ | H | $CH_3$ | |
| 21.15 | 3-$CF_3$ | H | $CH_3$ | H | $CH_3$ | |
| 21.16 | 3-$CH_3$ | H | $CH_3$ | H | $CH_3$ | |
| 21.17 | 3-$C_2H_5$ | H | $CH_3$ | H | $CH_3$ | |
| 21.18 | 3-$OCH_3$ | H | $CH_3$ | H | $CH_3$ | |
| 21.19 | 3-$OC_2H_5$ | H | $CH_3$ | H | $CH_3$ | |
| 21.20 | 4-F | H | $CH_3$ | H | $CH_3$ | |
| 21.21 | 3-F | 3-F | H | Cyclopropyl | $CH_3$ | |
| 21.22 | 3-Cl | 3-F | H | Cyclopropyl | $CH_3$ | |
| 21.23 | 3-Br | 3-F | H | Cyclopropyl | $CH_3$ | |
| 21.24 | 3-$CF_3$ | 3-F | H | Cyclopropyl | $CH_3$ | |
| 21.25 | 3-$CH_3$ | 3-F | H | Cyclopropyl | $CH_3$ | |
| 21.26 | 3-$C_2H_5$ | 3-F | H | Cyclopropyl | $CH_3$ | |
| 21.27 | 3-$OCH_3$ | 3-F | H | Cyclopropyl | $CH_3$ | |
| 21.28 | 3-$OC_2H_5$ | 3-F | H | Cyclopropyl | $CH_3$ | |
| 21.29 | 4-F | 3-F | H | Cyclopropyl | $CH_3$ | |
| 21.30 | 3-F | H | $CH_3$ | Cyclopropyl | $CH_3$ | |
| 21.31 | 3-Cl | H | $CH_3$ | Cyclopropyl | $CH_3$ | |
| 21.32 | 3-Br | H | $CH_3$ | Cyclopropyl | $CH_3$ | |
| 21.33 | 3-$CF_3$ | H | $CH_3$ | Cyclopropyl | $CH_3$ | |
| 21.34 | 3-$CH_3$ | H | $CH_3$ | Cyclopropyl | $CH_3$ | |

Tabelle 21 (Fortsetzung)

| Verbindung Nr. | $R^1$ | $R^3$ | $R^4$ | $R^9$ | $R^6$ | phys. Daten (Fp. °C) |
|---|---|---|---|---|---|---|
| 21.35 | 3-$C_2H_5$ | H | $CH_3$ | Cyclopropyl | $CH_3$ | |
| 21.36 | 3-$OCH_3$ | H | $CH_3$ | Cyclopropyl | $CH_3$ | |
| 21.37 | 3-$OC_2H_5$ | H | $CH_3$ | Cyclopropyl | $CH_3$ | |
| 21.38 | 4-F | H | $CH_3$ | Cyclopropyl | $CH_3$ | |
| 21.39 | 3-F | H | H | $CH_3$ | $CH_3$ | |
| 21.40 | 3-Cl | H | H | $CH_3$ | $CH_3$ | |
| 21.41 | 3-Br | H | H | $CH_3$ | $CH_3$ | |
| 21.42 | 3-$CF_3$ | H | H | $CH_3$ | $CH_3$ | |
| 21.43 | 3-$CH_3$ | H | H | $CH_3$ | $CH_3$ | |
| 21.44 | 3-$C_2H_5$ | H | H | $CH_3$ | $CH_3$ | |
| 21.45 | 3-$OCH_3$ | H | H | $CH_3$ | $CH_3$ | |
| 21.46 | 3-$OC_3H_5$ | H | H | $CH_3$ | $CH_3$ | |
| 21.47 | 4-F | H | H | $CH_3$ | $CH_3$ | |

Tabelle 22

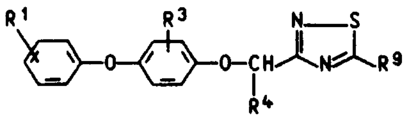

| Verbindung Nr. | $R^1$ | $R^3$ | $R^4$ | $R^9$ | phys. Daten (Fp. °C) |
|---|---|---|---|---|---|
| 22.1 | H | H | H | H | |
| 22.2 | H | H | $CH_3$ | H | |
| 22.3 | 3-F | H | H | H | |
| 22.4 | 3-Cl | H | H | H | |
| 22.5 | 3-Br | H | H | H | |
| 22.6 | 3-$CF_3$ | H | H | H | |
| 22.7 | 3-$CH_3$ | H | H | H | |
| 22.8 | 3-$C_2H_5$ | H | H | H | |
| 22.9 | 3-$OCH_3$ | H | H | H | |
| 22.10 | 3-$OC_2H_5$ | H | H | H | |
| 22.11 | 4-F | H | H | H | |
| 22.12 | 3-F | H | H | Cyclopropyl | |
| 22.13 | 3-Cl | H | H | Cyclopropyl | |
| 22.14 | 3-Br | H | H | Cyclopropyl | |
| 22.15 | 3-$CF_3$ | H | H | Cyclopropyl | |
| 22.16 | 3-$CH_3$ | H | H | Cyclopropyl | |
| 22.17 | 3-$C_2H_5$ | H | H | Cyclopropyl | |
| 22.18 | 3-$OCH_3$ | H | H | Cyclopropyl | |
| 22.19 | 3-$OC_2H_5$ | H | H | Cyclopropyl | |
| 22.20 | 4-F | H | H | $CH_3$ | |
| 22.21 | 3-F | H | H | $CH_3$ | |
| 22.22 | 3-Cl | H | H | $CH_3$ | |
| 22.23 | 3-Br | H | H | $CH_3$ | |
| 22.24 | 3-$CF_3$ | H | H | $CH_3$ | |
| 22.25 | 3-$CH_3$ | H | H | $CH_3$ | |
| 22.26 | 3-$C_2H_5$ | H | H | $CH_3$ | |
| 22.27 | 3-$OCH_3$ | H | H | $CH_3$ | |
| 22.28 | 3-$OC_2H_5$ | H | H | $CH_3$ | |
| 22.29 | 4-F | H | H | $CH_3$ | |
| 22.30 | 3-F | 3-F | H | $CH_3$ | |
| 22.31 | 3-Cl | 3-F | H | $CH_3$ | |
| 22.32 | 3-Br | 3-F | H | $CH_3$ | |
| 22.33 | 3-$CF_3$ | 3-F | H | $CH_3$ | |
| 22.34 | 3-$CH_3$ | 3-F | H | $CH_3$ | |

Tabelle 22 (Fortsetzung)

| Verbindung Nr. | $R^1$ | $R^3$ | $R^4$ | $R^9$ | phys. Daten (Fp. °C) |
|---|---|---|---|---|---|
| 22.35 | $3-C_2H_5$ | 3-F | H | $CH_3$ | |
| 22.36 | $3-OCH_3$ | 3-F | H | $CH_3$ | |
| 22.37 | $3-OC_2H_5$ | 3-F | H | $CH_3$ | |
| 22.38 | 4-F | 3-F | H | $CH_3$ | |
| 22.39 | 3-F | H | $CH_3$ | Cyclopropyl | |
| 22.40 | 3-Cl | H | $CH_3$ | Cyclopropyl | |
| 22.41 | 3-Br | H | $CH_3$ | Cyclopropyl | |
| 22.42 | $3-CF_3$ | H | $CH_3$ | Cyclopropyl | |
| 22.43 | $3-CH_3$ | H | $CH_3$ | Cyclopropyl | |
| 22.44 | $3-C_2H_5$ | H | $CH_3$ | Cyclopropyl | |
| 22.45 | $3-OCH_3$ | H | $CH_3$ | Cyclopropyl | |
| 22.46 | $3-OC_2H_5$ | H | $CH_3$ | Cyclopropyl | |
| 22.47 | 4-F | H | $CH_3$ | Cyclopropyl | |

Tabelle 23

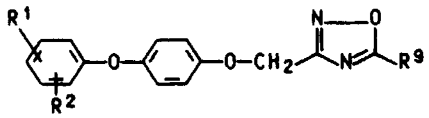

| Verbindung Nr. | $R^1$ | $R^2$ | $R^9$ | phys. Daten (Fp. °C) |
|---|---|---|---|---|
| 23.1 | H | H | H | |
| 23.2 | H | H | $CH_3$ | |
| 23.3 | 3-F | H | $CH_3$ | |
| 23.4 | 3-Cl | H | $CH_3$ | |
| 23.5 | 3-Br | H | $CH_3$ | |
| 23.6 | 3-$OCH_3$ | H | $CH_3$ | |
| 23.7 | 3-$CF_3$ | H | $CH_3$ | |
| 23.8 | 3-$CH_3$ | H | $CH_3$ | |
| 23.9 | 3-$C_2H_5$ | H | $CH_3$ | |
| 23.10 | 3-Cl | 4-F | $CH_3$ | |
| 23.11 | H | H | Cyclopropyl | |
| 23.12 | 3-F | H | Cyclopropyl | |
| 23.13 | 3-Cl | H | Cyclopropyl | |
| 23.14 | 3-Br | H | Cyclopropyl | |
| 23.15 | 3-$OCH_3$ | H | Cyclopropyl | |
| 23.16 | 3-$CF_3$ | H | Cyclopropyl | |
| 23.17 | 3-$CH_3$ | H | Cyclopropyl | |
| 23.18 | 3-$C_2H_5$ | H | Cyclopropyl | |
| 23.19 | H | H | $OC_2H_5$ | |
| 23.20 | 3-F | H | $OC_2H_5$ | |
| 23.21 | 3-Br | H | $OC_2H_5$ | |
| 23.22 | 3-$C_2H_5$ | H | $OC_2H_5$ | |

Tabelle 24

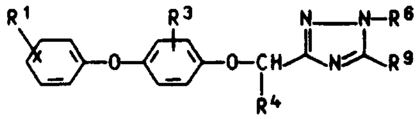

| Verbindung Nr. | $R^1$ | $R^3$ | $R^4$ | $R^9$ | $R^6$ | phys. Daten (Fp. $^0C$) |
|---|---|---|---|---|---|---|
| 24.1 | H | H | H | H | $CH_3$ | |
| 24.2 | H | H | $CH_3$ | H | $CH_3$ | |
| 24.3 | 3-F | H | H | H | $CH_3$ | |
| 24.4 | 3-Cl | H | H | H | $CH_3$ | |
| 24.5 | 3-Br | H | H | H | $CH_3$ | |
| 24.6 | 3-$CF_3$ | H | H | H | $CH_3$ | |
| 24.7 | 3-$CH_3$ | H | H | H | $CH_3$ | |
| 24.8 | 3-$C_2H_5$ | H | H | H | $CH_3$ | |
| 24.9 | 3-$OCH_3$ | H | H | H | $CH_3$ | |
| 24.10 | 3-$OC_2H_5$ | H | H | H | $CH_3$ | |
| 24.11 | 4-F | H | H | H | $CH_3$ | |
| 24.12 | 3-F | H | H | Cyclopropyl | $CH_3$ | |
| 24.13 | 3-Cl | H | H | Cyclopropyl | $CH_3$ | |
| 24.14 | 3-Br | H | H | Cyclopropyl | $CH_3$ | |
| 24.15 | 3-$CF_3$ | H | H | Cyclopropyl | $CH_3$ | |
| 24.16 | 3-$CH_3$ | H | H | Cyclopropyl | $CH_3$ | |
| 24.17 | 3-$C_2H_5$ | H | H | Cyclopropyl | $CH_3$ | |
| 24.18 | 3-$OCH_3$ | H | H | Cyclopropyl | $CH_3$ | |
| 24.19 | 3-$OC_2H_5$ | H | H | Cyclopropyl | $CH_3$ | |
| 24.20 | 4-F | H | H | Cyclopropyl | $CH_3$ | |
| 24.21 | 3-F | H | $CH_3$ | Cyclopropyl | $CH_3$ | |
| 24.22 | 3-Cl | H | $CH_3$ | Cyclopropyl | $CH_3$ | |
| 24.23 | 3-Br | H | $CH_3$ | Cyclopropyl | $CH_3$ | |
| 24.24 | 3-$CF_3$ | H | $CH_3$ | Cyclopropyl | $CH_3$ | |
| 24.25 | 3-$CH_3$ | H | $CH_3$ | Cyclopropyl | $CH_3$ | |
| 24.26 | 3-$C_2H_5$ | H | $CH_3$ | Cyclopropyl | $CH_3$ | |
| 24.27 | 3-$OCH_3$ | H | $CH_3$ | Cyclopropyl | $CH_3$ | |
| 24.28 | 3-$OC_2H_5$ | H | $CH_3$ | Cyclopropyl | $CH_3$ | |
| 24.29 | 4-F | H | $CH_3$ | Cyclopropyl | $CH_3$ | |
| 24.30 | 3-F | 3-F | H | Cyclopropyl | $CH_3$ | |
| 24.31 | 3-Cl | 3-F | H | Cyclopropyl | $CH_3$ | |
| 24.32 | 3-Br | 3-F | H | Cyclopropyl | $CH_3$ | |
| 24.33 | 3-$CF_3$ | 3-F | H | Cyclopropyl | $CH_3$ | |
| 24.34 | 3-$CH_3$ | 3-F | H | Cyclopropyl | $CH_3$ | |

Tabelle 24 (Fortsetzung)

| Verbindung Nr. | $R^1$ | $R^3$ | $R^4$ | $R^9$ | $R^6$ | phys. Daten (Fp. °C) |
|---|---|---|---|---|---|---|
| 24.35 | 3-$C_2H_5$ | 3-F | H | Cyclopropyl | $CH_3$ | |
| 24.36 | 3-$OCH_3$ | 3-F | H | Cyclopropyl | $CH_3$ | |
| 24.37 | 3-$OC_2H_5$ | 3-F | H | Cyclopropyl | $CH_3$ | |
| 24.38 | 4-F | 3-F | H | Cyclopropyl | $CH_3$ | |
| 24.39 | 3-F | H | H | $CH_3$ | $CH_3$ | |
| 24.40 | 3-Cl | H | H | $CH_3$ | $CH_3$ | |
| 24.41 | 3-Br | H | H | $CH_3$ | $CH_3$ | |
| 24.42 | 3-$CF_3$ | H | H | $CH_3$ | $CH_3$ | |
| 24.43 | 3-$CH_3$ | H | H | $CH_3$ | $CH_3$ | |
| 24.44 | 3-$C_2H_5$ | H | H | $CH_3$ | $CH_3$ | |
| 24.45 | 3-$OCH_3$ | H | H | $CH_3$ | $CH_3$ | |
| 24.46 | 3-$OC_2H_5$ | H | H | $CH_3$ | $CH_3$ | |
| 24.47 | 4-F | H | H | $CH_3$ | $CH_3$ | |

Tabelle 25

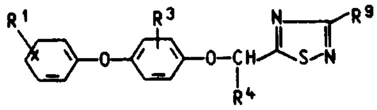

| Verbindung Nr. | $R^1$ | $R^3$ | $R^4$ | $R^9$ | phys. Daten (Fp. °C) |
|---|---|---|---|---|---|
| 25.1 | H | H | H | H | |
| 25.2 | H | H | $CH_3$ | H | |
| 25.3 | 3-F | H | H | H | |
| 25.4 | 3-Cl | H | H | H | |
| 25.5 | 3-Br | H | H | H | |
| 25.6 | 3-$CF_3$ | H | H | H | |
| 25.7 | 3-$CH_3$ | H | H | H | |
| 25.8 | 3-$C_2H_5$ | H | H | H | |
| 25.9 | 3-$OCH_3$ | H | H | H | |
| 25.10 | 3-$OC_2H_5$ | H | H | H | |
| 25.11 | 4-F | H | H | H | |
| 25.12 | 3-F | H | H | $CH_3$ | |
| 25.13 | 3-Cl | H | H | $CH_3$ | |
| 25.14 | 3-Br | H | H | $CH_3$ | |
| 25.15 | 3-$CF_3$ | H | H | $CH_3$ | |
| 25.16 | 3-$CH_3$ | H | H | $CH_3$ | |
| 25.17 | 3-$C_2H_5$ | H | H | $CH_3$ | |
| 25.18 | 3-$OCH_3$ | H | H | $CH_3$ | |
| 25.19 | 3-$OC_2H_5$ | H | H | $CH_3$ | |
| 25.20 | 4-F | H | H | $CH_3$ | |
| 25.21 | 3-F | H | H | Cyclopropyl | |
| 25.22 | 3-Cl | H | H | Cyclopropyl | |
| 25.23 | 3-Br | H | H | Cyclopropyl | |
| 25.24 | 3-$CF_3$ | H | H | Cyclopropyl | |
| 25.25 | 3-$CH_3$ | H | H | Cyclopropyl | |
| 25.26 | 3-$C_2H_5$ | H | H | Cyclopropyl | |
| 25.27 | 3-$OCH_3$ | H | H | Cyclopropyl | |
| 25.28 | 3-$OC_2H_5$ | H | H | Cyclopropyl | |
| 25.29 | 4-F | H | H | Cyclopropyl | |
| 25.30 | 3-F | 3-F | H | $CH_3$ | |
| 25.31 | 3-Cl | 3-F | H | $CH_3$ | |
| 25.32 | 3-Br | 3-F | H | $CH_3$ | |
| 25.33 | 3-$CF_3$ | 3-F | H | $CH_3$ | |
| 25.34 | 3-$CH_3$ | 3-F | H | $CH_3$ | |

Tabelle 25 (Fortsetzung)

| Verbindung Nr. | $R^1$ | $R^3$ | $R^4$ | $R^9$ | phys. Daten (Fp. °C) |
|---|---|---|---|---|---|
| 25.35 | 3-$C_2H_5$ | 3-F | H | $CH_3$ | |
| 25.36 | 3-$OCH_3$ | 3-F | H | $CH_3$ | |
| 25.37 | 3-$OC_2H_5$ | 3-F | H | $CH_3$ | |
| 25.38 | 4-F | 3-F | H | $CH_3$ | |
| 25.39 | 3-F | H | $CH_3$ | $CH_3$ | |
| 25.40 | 3-Cl | H | $CH_3$ | $CH_3$ | |
| 25.41 | 3-Br | H | $CH_3$ | $CH_3$ | |
| 25.42 | 3-$CF_3$ | H | $CH_3$ | $CH_3$ | |
| 25.43 | 3-$CH_3$ | H | $CH_3$ | $CH_3$ | |
| 25.44 | 3-$C_2H_5$ | H | $CH_3$ | $CH_3$ | |
| 25.45 | 3-$OCH_3$ | H | $CH_3$ | $CH_3$ | |
| 25.46 | 3-$OC_2H_5$ | H | $CH_3$ | $CH_3$ | |
| 25.47 | 4-F | H | $CH_3$ | $CH_3$ | |

Tabelle 26

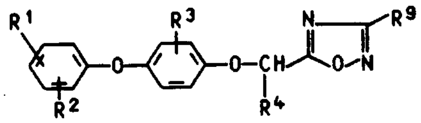

| Verbindung Nr. | $R^1$ | $R^2$ | $R^3$ | $R^4$ | $R^9$ | phys. Daten (Fp. °C) |
|---|---|---|---|---|---|---|
| 26.1 | H | H | H | H | H | |
| 26.2 | 3-F | H | H | H | H | |
| 26.3 | 3-Cl | H | H | H | H | |
| 26.4 | 3-Br | H | H | H | H | |
| 26.5 | 3-$CF_3$ | H | H | H | H | |
| 26.6 | 3-$OCH_3$ | H | H | H | H | |
| 26.7 | 3-$C_2H_5$ | H | H | H | H | |
| 26.8 | 3-$CH_3$ | H | H | H | H | |
| 26.9 | H | H | H | H | $CH_3$ | |
| 26.10 | 3-F | H | H | H | $CH_3$ | |
| 26.11 | 3-Cl | H | H | H | $CH_3$ | |
| 26.12 | 3-Br | H | H | H | $CH_3$ | |
| 26.13 | 3-$CF_3$ | H | H | H | $CH_3$ | |
| 26.14 | 3-$OCH_3$ | H | H | H | $CH_3$ | |
| 26.15 | 3-$C_2H_5$ | H | H | H | $CH_3$ | |
| 26.16 | 3-$CH_3$ | H | H | H | $CH_3$ | |
| 26.17 | 3-Cl | 4-F | H | H | $CH_3$ | |
| 26.18 | H | H | H | H | $OC_2H_5$ | 70-72 |
| 26.19 | H | H | H | H | $OCH_3$ | 93-94 |
| 26.20 | H | H | H | H | Cyclopropyl | 99 |
| 26.21 | 3-F | H | H | H | Cyclopropyl | 101 |
| 26.22 | 3-Cl | H | H | H | Cyclopropyl | 82 |
| 26.23 | 3-Br | H | H | H | Cyclopropyl | 83 |
| 26.24 | 3-$CF_3$ | H | H | H | Cyclopropyl | 102 |
| 26.25 | 3-$OCH_3$ | H | H | H | Cyclopropyl | 66 |
| 26.26 | 3-$CH_3$ | H | H | H | Cyclopropyl | 76 |
| 26.27 | 3-$C_2H_5$ | H | H | H | Cyclopropyl | |
| 26.28 | 3-Cl | 4-F | H | H | Cyclopropyl | |
| 26.32 | H | H | 3-F | H | Cyclopropyl | |
| 26.33 | H | H | 3-F | H | $CH_3$ | |
| 26.34 | H | H | 3-F | H | $OC_2H_5$ | |

Tabelle 26 (Fortsetzung)

| Verbindung Nr. | $R^1$ | $R^2$ | $R^3$ | $R^4$ | $R^9$ | phys. Daten (Fp. °C) |
|---|---|---|---|---|---|---|
| 26.35 | H | H | H | H | iso-$C_3H_7$ | |
| 26.36 | 3-F | H | 3-F | H | Cyclopropyl | |
| 26.37 | 3-CF | H | 3-Cl | H | Cyclopropyl | |
| 26.38 | 3-Cl | H | 3-F | H | Cyclopropyl | |
| 26.39 | 3-Cl | H | 3-Cl | H | Cyclopropyl | |
| 26.40 | 3-Br | H | 3-F | H | Cyclopropyl | |
| 26.41 | 3-Br | H | 3-Cl | H | Cyclopropyl | |
| 26.42 | 3-$CF_3$ | H | 3-F | H | Cyclopropyl | 300 MHz-$^1$H-NMR in $CDCl_3$ [ppm]:5.49(s) |
| 26.43 | 3-$CF_3$ | H | 3-Cl | H | Cyclopropyl | |
| 26.44 | 3-$CH_3$ | H | 3-F | H | Cyclopropyl | |
| 26.45 | 3-$CH_3$ | H | 3-Cl | H | Cyclopropyl | |
| 26.46 | 3-$C_2H_5$ | H | 3-F | H | Cyclopropyl | |
| 26.47 | 3-$C_2H_5$ | H | 3-Cl | H | Cyclopropyl | |
| 26.48 | 3-$OCH_3$ | H | 3-F | H | Cyclopropyl | |
| 26.49 | 3-$OCH_3$ | H | 3-Cl | H | Cyclopropyl | |
| 26.50 | 3-Cl | 4-F | 3-F | H | Cyclopropyl | |
| 26.51 | 3-Cl | 4-F | 3-Cl | H | Cyclopropyl | |
| 26.52 | H | 4-F | H | H | Cyclopropyl | |
| 26.53 | H | 4-F | 3-F | H | Cyclopropyl | |
| 26.54 | H | 4-F | 3-Cl | H | Cyclopropyl | |

Tabelle 27

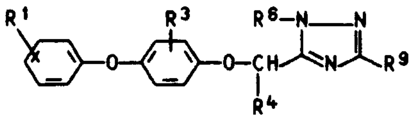

| Verbindung Nr. | $R^1$ | $R^3$ | $R^4$ | $R^9$ | $R^6$ | phys. Daten (Fp. °C) |
|---|---|---|---|---|---|---|
| 27.1 | H | H | H | H | $CH_3$ | |
| 27.2 | H | H | $CH_3$ | H | $CH_3$ | |
| 27.3 | 3-F | H | H | H | $CH_3$ | |
| 27.4 | 3-Cl | H | H | H | $CH_3$ | |
| 27.5 | 3-Br | H | H | H | $CH_3$ | |
| 27.6 | 3-$CF_3$ | H | H | H | $CH_3$ | |
| 27.7 | 3-$CH_3$ | H | H | H | $CH_3$ | |
| 27.8 | 3-$C_2H_5$ | H | H | H | $CH_3$ | |
| 27.9 | 3-$OCH_3$ | H | H | H | $CH_3$ | |
| 27.10 | 3-$OC_2H_5$ | H | H | H | $CH_3$ | |
| 27.11 | 4-F | H | H | H | $CH_3$ | |
| 27.12 | 3-F | H | H | $CH_3$ | $CH_3$ | 82-84 |
| 27.13 | 3-Cl | H | H | $CH_3$ | $CH_3$ | |
| 27.14 | 3-Br | H | H | $CH_3$ | $CH_3$ | |
| 27.15 | 3-$CF_3$ | H | H | $CH_3$ | $CH_3$ | |
| 27.16 | 3-$CH_3$ | H | H | $CH_3$ | $CH_3$ | |
| 27.17 | 3-$C_2H_5$ | H | H | $CH_3$ | $CH_3$ | 79-80 |
| 27.18 | 3-$OCH_3$ | H | H | $CH_3$ | $CH_3$ | |
| 27.19 | 3-H | H | H | $CH_3$ | $CH_3$ | 109-110 |
| 27.20 | 4-F | H | H | $CH_3$ | $CH_3$ | |
| 27.21 | 3-F | H | H | Cyclopropyl | $CH_3$ | 64-66 |
| 27.22 | 3-Cl | H | H | Cyclopropyl | $CH_3$ | 82-85 |
| 27.23 | 3-Br | H | H | Cyclopropyl | $CH_3$ | 85-88 |
| 27.24 | 3-$CF_3$ | H | H | Cyclopropyl | $CH_3$ | 77-79 |
| 27.25 | 3-$CH_3$ | H | H | Cyclopropyl | $CH_3$ | |
| 27.26 | 3-$C_2H_5$ | H | H | Cyclopropyl | $CH_3$ | 59-61 |
| 27.27 | 3-$OCH_3$ | H | H | Cyclopropyl | $CH_3$ | |
| 27.28 | 3-H | H | H | Cyclopropyl | $CH_3$ | 80-82 |
| 27.29 | 4-F | H | H | Cyclopropyl | $CH_3$ | |
| 27.30 | 3-F | H | $CH_3$ | Cyclopropyl | $CH_3$ | |
| 27.31 | 3-Cl | H | $CH_3$ | Cyclopropyl | $CH_3$ | |
| 27.32 | 3-Br | H | $CH_3$ | Cyclopropyl | $CH_3$ | |
| 27.33 | 3-$CF_3$ | H | $CH_3$ | Cyclopropyl | $CH_3$ | |
| 27.34 | 3-$CH_3$ | H | $CH_3$ | Cyclopropyl | $CH_3$ | |

Tabelle 27 (Fortsetzung)

| Verbindung Nr. | $R^1$ | $R^3$ | $R^4$ | $R^9$ | $R^6$ | phys. Daten (Fp. °C) |
|---|---|---|---|---|---|---|
| 27.35 | 3-$C_2H_5$ | H | $CH_3$ | Cyclopropyl | $CH_3$ | |
| 27.36 | 3-$OCH_3$ | H | $CH_3$ | Cyclopropyl | $CH_3$ | |
| 27.37 | 3-$OC_2H_5$ | H | $CH_3$ | Cyclopropyl | $CH_3$ | |
| 27.38 | 4-F | H | $CH_3$ | Cyclopropyl | $CH_3$ | |
| 27.39 | 3-F | 3-F | H | Cyclopropyl | $CH_3$ | |
| 27.40 | 3-Cl | 3-F | H | Cyclopropyl | $CH_3$ | |
| 27.41 | 3-Br | 3-F | H | Cyclopropyl | $CH_3$ | |
| 27.42 | 3-$CF_3$ | 3-F | H | Cyclopropyl | $CH_3$ | |
| 27.43 | 3-$CH_3$ | 3-F | H | Cyclopropyl | $CH_3$ | |
| 27.44 | 3-$C_2H_5$ | 3-F | H | Cyclopropyl | $CH_3$ | |
| 27.45 | 3-$OCH_3$ | 3-F | H | Cyclopropyl | $CH_3$ | |
| 27.46 | 3-$OC_2H_5$ | 3-F | H | Cyclopropyl | $CH_3$ | |
| 27.47 | 4-F | 3-F | H | Cyclopropyl | $CH_3$ | |

Tabelle 28

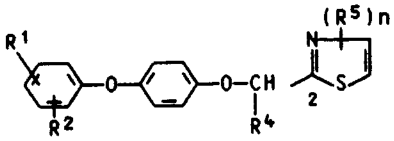

| Verbindung Nr. | $R^1$ | $R^2$ | $R^4$ | $R^5$ | n | phys. Daten (Fp. °C) |
|---|---|---|---|---|---|---|
| 28.1 | H | H | H | - | 0 | |
| 28.2 | H | H | $CH_3$ | - | 0 | |
| 28.3 | 3-F | H | H | - | 0 | |
| 28.4 | 3-Cl | H | $CH_3$ | - | 0 | |
| 28.5 | 3-Br | H | H | - | 0 | |
| 28.6 | 3-$CF_3$ | H | $CH_3$ | - | 0 | |
| 28.7 | 3-$CH_3$ | H | H | - | 0 | |
| 28.8 | 3-$C_2H_5$ | H | $CH_3$ | - | 0 | |
| 28.9 | 3-$OCH_3$ | H | H | - | 0 | |
| 28.10 | 3-Cl | 4-F | $CH_3$ | - | 0 | |
| 28.11 | H | H | H | 5-$CH_3$ | 1 | |
| 28.12 | H | H | $CH_3$ | 5-$CH_3$ | 1 | |
| 28.13 | 3-F | H | H | 5-$CH_3$ | 1 | |
| 28.14 | 3-F | H | $CH_3$ | 5-$CH_3$ | 1 | |
| 28.15 | 3-Cl | H | H | 5-$CH_3$ | 1 | |
| 28.16 | 3-Cl | H | $CH_3$ | 5-$CH_3$ | 1 | |
| 28.17 | 3-Br | H | H | 5-$CH_3$ | 1 | |
| 28.18 | 3-Br | H | $CH_3$ | 5-$CH_3$ | 1 | |
| 28.19 | 3-$CF_3$ | H | H | 5-$CH_3$ | 1 | |
| 28.20 | 3-$CF_3$ | H | $CH_3$ | 5-$CH_3$ | 1 | |
| 28.21 | 3-$CH_3$ | H | H | 5-$CH_3$ | 1 | |
| 28.22 | 3-$CH_3$ | H | $CH_3$ | 5-$CH_3$ | 1 | |
| 28.23 | 3-$C_2H_5$ | H | H | 5-$CH_3$ | 1 | |
| 28.24 | 3-$C_2H_5$ | H | $CH_3$ | 5-$CH_3$ | 1 | |
| 28.25 | 3-$OCH_3$ | H | H | 5-$CH_3$ | 1 | |
| 28.26 | 3-$OCH_3$ | H | $CH_3$ | 5-$CH_3$ | 1 | |
| 28.27 | 3-Cl | 4-F | H | 5-$CH_3$ | 1 | |
| 28.28 | 3-Cl | 4-F | $CH_3$ | 5-$CH_3$ | 1 | |
| 28.29 | H | H | H | 5-Cyclopropyl | 1 | |
| 28.30 | H | H | $CH_3$ | 5-Cyclopropyl | 1 | |
| 28.31 | 3-F | H | H | 5-Cyclopropyl | 1 | |
| 28.32 | 3-Cl | H | $CH_3$ | 5-Cyclopropyl | 1 | |
| 28.33 | 3-Br | H | H | 5-Cyclopropyl | 1 | |
| 28.34 | 3-$CF_3$ | H | $CH_3$ | 5-Cyclopropyl | 1 | |

Tabelle 28 (Fortsetzung)

| Verbindung Nr. | $R^1$ | $R^2$ | $R^4$ | $R^5$ | n | phys. Daten (Fp. °C) |
|---|---|---|---|---|---|---|
| 28.35 | 3-$CH_3$ | H | H | 5-Cyclopropyl | 1 | |
| 28.36 | 3-$C_2H_5$ | H | $CH_3$ | 5-Cyclopropyl | 1 | |
| 28.37 | 3-$OCH_3$ | H | H | 5-Cyclopropyl | 1 | |
| 28.38 | 3-Cl | 4-F | $CH_3$ | 5-Cyclopropyl | 1 | |
| 28.39 | H | H | H | 5-$OCH_3$ | 1 | |
| 28.40 | H | H | $CH_3$ | 5-$OCH_3$ | 1 | |
| 28.41 | H | H | H | 5-$OC_2H_5$ | 1 | |
| 28.42 | H | H | $CH_3$ | 5-$OC_2H_5$ | 1 | |
| 28.43 | 3-F | H | H | 5-$OC_2H_5$ | 1 | |
| 28.44 | 3-Cl | H | $CH_3$ | 5-$OC_2H_5$ | 1 | |
| 28.45 | 3-Br | H | H | 5-$OC_2H_5$ | 1 | |
| 28.46 | 3-$CF_3$ | H | $CH_3$ | 5-$OC_2H_5$ | 1 | |
| 28.47 | 3-$CH_3$ | H | H | 5-$OC_2H_5$ | 1 | |
| 28.48 | 3-$C_2H_5$ | H | $CH_3$ | 5-$OC_2H_5$ | 1 | |
| 28.49 | 3-$OCH_3$ | H | H | 5-$OC_2H_5$ | 1 | |
| 28.50 | 3-Cl | H | H | 5-$OC_2H_5$ | 1 | |
| 28.51 | H | H | H | 5-Cl | 1 | |
| 28.52 | H | H | $CH_3$ | 5-Cl | 1 | |
| 28.53 | 3-F | H | H | 5-Cl | 1 | |
| 28.54 | 3-Cl | H | $CH_3$ | 5-Cl | 1 | |
| 28.55 | 3-Br | H | H | 5-Cl | 1 | |
| 28.56 | 3-$CF_3$ | H | $CH_3$ | 5-Cl | 1 | |
| 28.57 | 3-$CH_3$ | H | H | 5-Cl | 1 | |
| 28.58 | 3-$C_2H_5$ | H | $CH_3$ | 5-Cl | 1 | |
| 28.59 | 3-$OCH_3$ | H | H | 5-Cl | 1 | |
| 28.60 | 3-Cl | 4-F | $CH_3$ | 5-Cl | 1 | |
| 28.61 | H | H | H | 4-$CH_3$ | 1 | |
| 28.62 | H | H | $CH_3$ | 4-$CH_3$ | 1 | |
| 28.63 | 3-F | H | H | 4-$CH_3$ | 1 | |
| 28.64 | 3-Cl | H | $CH_3$ | 4-$CH_3$ | 1 | |
| 28.65 | 3-Br | H | H | 4-$CH_3$ | 1 | |
| 28.66 | 3-$CF_3$ | H | $CH_3$ | 4-$CH_3$ | 1 | |
| 28.67 | 3-$CH_3$ | H | H | 4-$CH_3$ | 1 | |
| 28.68 | 3-$C_2H_5$ | H | $CH_3$ | 4-$CH_3$ | 1 | |
| 28.69 | 3-$OCH_3$ | H | H | 4-$CH_3$ | 1 | |
| 28.70 | 3-Cl | 4-F | $CH_3$ | 4-$CH_3$ | 1 | |
| 28.71 | H | H | H | 4-Cyclopropyl | 1 | |

Tabelle 28 (Fortsetzung)

| Verbindung Nr. | $R^1$ | $R^2$ | $R^4$ | $R^5$ | n | phys. Daten (Fp. °C) |
|---|---|---|---|---|---|---|
| 28.72 | H | H | $CH_3$ | 4-Cyclopropyl | 1 | |
| 28.73 | 3-F | H | H | 4-Cyclopropyl | 1 | |
| 28.74 | 3-Cl | H | $CH_3$ | 4-Cyclopropyl | 1 | |
| 28.75 | 3-Br | H | H | 4-Cyclopropyl | 1 | |
| 28.76 | 3-$CF_3$ | H | $CH_3$ | 4-Cyclopropyl | 1 | |
| 28.77 | 3-$CH_3$ | H | H | 4-Cyclopropyl | 1 | |
| 28.78 | 3-$C_2H_5$ | H | $CH_3$ | 4-Cyclopropyl | 1 | |
| 28.79 | 3-$OCH_3$ | H | H | 4-Cyclopropyl | 1 | |
| 28.80 | 3-Cl | 4-F | $CH_3$ | 4-Cyclopropyl | 1 | |
| 28.81 | H | H | H | 4-$OCH_3$ | 1 | |
| 28.82 | H | H | H | 4-$OC_2H_5$ | 1 | |
| 28.83 | H | H | $CH_3$ | 4-$OC_2H_5$ | 1 | |
| 28.84 | 3-F | H | H | 4-$OC_2H_5$ | 1 | |
| 28.85 | 3-Cl | H | $CH_3$ | 4-$OC_2H_5$ | 1 | |
| 28.86 | 3-Br | H | H | 4-$OC_2H_5$ | 1 | |
| 28.87 | 3-$CF_3$ | H | $CH_3$ | 4-$OC_2H_5$ | 1 | |
| 28.88 | 3-$CH_3$ | H | H | 4-$OC_2H_5$ | 1 | |
| 28.89 | 3-$C_2H_5$ | H | $CH_3$ | 4-$OC_2H_5$ | 1 | |
| 28.90 | 3-$OCH_3$ | H | H | 4-$OC_2H_5$ | 1 | |
| 28.91 | 3-Cl | 4-F | $CH_3$ | 4-$OC_2H_5$ | 1 | |
| 28.92 | H | H | H | 4-Cl | 1 | |
| 28.93 | H | H | $CH_3$ | 4-Cl | 1 | |
| 28.94 | 3-F | H | H | 4-Cl | 1 | |
| 28.95 | 3-Cl | H | $CH_3$ | 4-Cl | 1 | |
| 28.96 | 3-Br | H | H | 4-Cl | 1 | |
| 28.97 | 3-$CF_3$ | H | $CH_3$ | 4-Cl | 1 | |
| 28.98 | 3-$CH_3$ | H | H | 4-Cl | 1 | |
| 28.99 | 3-$C_2H_5$ | H | $CH_3$ | 4-Cl | 1 | |
| 28.100 | 3-$OCH_3$ | H | H | 4-Cl | 1 | |
| 28.101 | 3-Cl | 4-F | $CH_3$ | 4-Cl | 1 | |

Tabelle 29

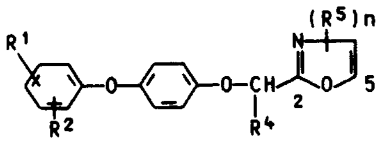

| Verbindung Nr. | $R^1$ | $R^2$ | $R^4$ | $R^5$ | n | phys. Daten (Fp. °C) |
|---|---|---|---|---|---|---|
| 29.1 | H | H | H | - | 0 | |
| 29.2 | H | H | $CH_3$ | - | 0 | |
| 29.3 | 3-F | H | H | - | 0 | |
| 29.4 | 3-Cl | H | H | - | 0 | |
| 29.5 | 3-Br | H | H | - | 0 | |
| 29.6 | 3-$CF_3$ | H | H | - | 0 | |
| 29.7 | 3-$CH_3$ | H | H | - | 0 | |
| 29.8 | 3-$C_2H_5$ | H | H | - | 0 | |
| 29.9 | 3-$OCH_3$ | H | H | - | 0 | |
| 29.10 | 3-Cl | 4-F | H | - | 0 | |
| 29.11 | H | H | H | 5-$CH_3$ | 1 | |
| 29.12 | 3-F | H | H | 5-$CH_3$ | 1 | |
| 29.13 | 3-Cl | H | H | 5-$CH_3$ | 1 | |
| 29.14 | 3-Br | H | H | 5-$CH_3$ | 1 | |
| 29.15 | 3-$CF_3$ | H | H | 5-$CH_3$ | 1 | |
| 29.16 | 3-$CH_3$ | H | H | 5-$CH_3$ | 1 | |
| 29.17 | 3-$C_2H_5$ | H | H | 5-$CH_3$ | 1 | |
| 29.18 | 3-$OCH_3$ | H | H | - | 0 | |
| 29.19 | 3-Cl | 4-F | H | - | 0 | |
| 29.20 | H | H | H | 5-Cyclopropyl | 1 | |
| 29.21 | 3-F | H | H | 5-Cyclopropyl | 1 | |
| 29.22 | 3-Cl | H | H | 5-Cyclopropyl | 1 | |
| 29.23 | 3-CN | H | H | 5-Cyclopropyl | 1 | |
| 29.24 | 3-Br | H | H | 5-Cyclopropyl | 1 | |
| 29.25 | 3-$CF_3$ | H | H | 5-Cyclopropyl | 1 | |
| 29.26 | 3-$CH_3$ | H | H | 5-Cyclopropyl | 1 | |
| 29.27 | 3-$C_2H_5$ | H | H | 5-Cyclopropyl | 1 | |
| 29.28 | 3-$OCH_3$ | H | H | 5-Cyclopropyl | 1 | |
| 29.29 | 3-Cl | 4-F | H | 5-Cyclopropyl | 1 | |
| 29.30 | H | H | H | 4-$CH_3$ | 1 | |
| 29.31 | 3-F | H | H | 4-$CH_3$ | 1 | |
| 29.32 | 3-Cl | H | H | 4-$CH_3$ | 1 | |
| 29.33 | 3-Br | H | H | 4-$CH_3$ | 1 | |
| 29.34 | 3-$CF_3$ | H | H | 4-$CH_3$ | 1 | |

Tabelle 29 (Fortsetzung)

| Verbindung Nr. | $R^1$ | $R^2$ | $R^4$ | $R^5$ | n | phys. Daten (Fp. °C) |
|---|---|---|---|---|---|---|
| 29.35 | 3-$CH_3$ | H | H | 4-$CH_3$ | 1 | |
| 29.36 | 3-$C_2H_5$ | H | H | 4-$CH_3$ | 1 | |
| 29.37 | 3-$OCH_3$ | H | H | 4-$CH_3$ | 1 | |
| 29.38 | 3-Cl | 4-F | H | 4-$CH_3$ | 1 | |
| 29.39 | H | H | H | 4-Cyclopropyl | 1 | |
| 29.40 | 3-F | H | H | 4-Cyclopropyl | 1 | |
| 29.41 | 3-Cl | H | H | 4-Cyclopropyl | 1 | |
| 29.42 | 3-Br | H | H | 4-Cyclopropyl | 1 | |
| 29.43 | 3-$CF_3$ | H | H | 4-Cyclopropyl | 1 | |
| 29.44 | 3-$CH_3$ | H | H | 4-Cyclopropyl | 1 | |
| 29.45 | 3-$C_2H_5$ | H | H | 4-Cyclopropyl | 1 | |
| 29.46 | 3-$OCH_3$ | H | H | 4-Cyclopropyl | 1 | |
| 29.47 | 3-Cl | 4-F | H | 4-Cyclopropyl | 1 | |
| 29.48 | H | H | H | 4-$OCH_3$ | 1 | |
| 29.49 | H | H | H | 4-$OC_2H_5$ | 1 | |
| 29.50 | 3-F | H | H | 4-$OC_2H_5$ | 1 | |
| 29.51 | 3-Cl | H | H | 4-$OC_2H_5$ | 1 | |
| 29.52 | 3-Br | H | H | 4-$OC_2H_5$ | 1 | |
| 29.53 | 3-$CF_3$ | H | H | 4-$OC_2H_5$ | 1 | |
| 29.54 | 3-$CH_3$ | H | H | 4-$OC_2H_5$ | 1 | |
| 29.55 | 3-$C_2H_5$ | H | H | 4-$OC_2H_5$ | 1 | |
| 29.56 | 3-$OCH_3$ | H | H | 4-$OC_2H_5$ | 1 | |
| 29.57 | 3-Cl | 4-F | H | 4-$OC_2H_5$ | 1 | |
| 29.58 | H | H | H | 5-$OCH_3$ | 1 | |
| 29.59 | H | H | H | 5-$OC_2H_5$ | 1 | |
| 29.60 | 3-F | H | H | 5-$OC_2H_5$ | 1 | |
| 29.61 | 3-Cl | H | H | 5-$OC_2H_5$ | 1 | |
| 29.62 | 3-Br | H | H | 5-$OC_2H_5$ | 1 | |
| 29.63 | 3-$CF_3$ | H | H | 5-$OC_2H_5$ | 1 | |
| 29.64 | 3-$CH_3$ | H | H | 5-$OC_2H_5$ | 1 | |
| 29.65 | 3-$C_2H_5$ | H | H | 5-$OC_2H_5$ | 1 | |
| 29.66 | 3-$OCH_3$ | H | H | 5-$OC_2H_5$ | 1 | |
| 29.67 | 3-Cl | 4-F | H | 5-$OC_2H_5$ | 1 | |
| 29.68 | H | H | H | 5-Cl | 1 | |
| 29.69 | 3-F | H | H | 5-Cl | 1 | |
| 29.70 | 3-Cl | H | H | 5-Cl | 1 | |
| 29.71 | 3-$CF_3$ | H | H | 5-Cl | 1 | |

Tabelle 29 (Fortsetzung)

| Verbindung Nr. | $R^1$ | $R^2$ | $R^4$ | $R^5$ | n | phys. Daten (Fp. °C) |
|---|---|---|---|---|---|---|
| 29.72 | 3-$CH_3$ | H | H | 5-Cl | 1 | |
| 29.73 | 3-$OCH_3$ | H | H | 5-Cl | 1 | |
| 29.74 | 3-Cl | H | H | 5-Cl | 1 | |

Tabelle 30

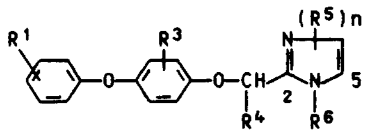

| Verbindung Nr. | $R^1$ | $R^3$ | $R^4$ | $R^5$ | $R^6$ | n | phys. Daten (Fp. °C) |
|---|---|---|---|---|---|---|---|
| 30.1 | H | H | H | - | $CH_3$ | 0 | 84-86 |
| 30.2 | H | H | H | - | $CH_3$ | 0 | |
| 30.3 | 3-F | H | H | - | $CH_3$ | 0 | 97-99 |
| 30.4 | 3-Cl | H | H | - | $CH_3$ | 0 | |
| 30.5 | 3-Br | H | H | - | $CH_3$ | 0 | |
| 30.6 | $3-CF_3$ | H | H | - | $CH_3$ | 0 | |
| 30.7 | $3-CH_3$ | H | H | - | $CH_3$ | 0 | |
| 30.8 | $3-C_2H_5$ | H | H | - | $CH_3$ | 0 | 300 MHz-$^1$H-NMR in $CDCl_3$ [ppm]: 3,78(s) |
| 30.9 | $3-OCH_3$ | H | H | - | $CH_3$ | 0 | |
| 30.10 | $3-OC_2H_5$ | H | H | - | $CH_3$ | 0 | |
| 30.11 | 4-F | H | H | $5-CH_3$ | $CH_3$ | 1 | |
| 30.12 | 3-F | H | H | $5-CH_3$ | $CH_3$ | 1 | |
| 30.13 | 3-Cl | H | H | $5-CH_3$ | $CH_3$ | 1 | |
| 30.14 | 3-Br | H | H | $5-CH_3$ | $CH_3$ | 1 | |
| 30.15 | $3-CF_3$ | H | H | $5-CH_3$ | $CH_3$ | 1 | |
| 30.16 | $3-CH_3$ | H | H | $5-CH_3$ | $CH_3$ | 1 | |
| 30.17 | $3-C_2H_5$ | H | H | $5-CH_3$ | $CH_3$ | 1 | |
| 30.18 | $3-OCH_3$ | H | H | $5-CH_3$ | $CH_3$ | 1 | |
| 30.19 | $3-OC_2H_5$ | H | H | $5-CH_3$ | $CH_3$ | 1 | |
| 30.20 | 4-F | H | H | $5-CH_3$ | $CH_3$ | 1 | |
| 30.21 | 3-F | H | $CH_3$ | $5-CH_3$ | $CH_3$ | 1 | |
| 30.22 | 3-Cl | H | $CH_3$ | $5-CH_3$ | $CH_3$ | 1 | |
| 30.23 | 3-Br | H | $CH_3$ | $5-CH_3$ | $CH_3$ | 1 | |
| 30.24 | $3-CF_3$ | H | $CH_3$ | $5-CH_3$ | $CH_3$ | 1 | |
| 30.25 | $3-CH_3$ | H | $CH_3$ | $5-CH_3$ | $CH_3$ | 1 | |
| 30.26 | $3-C_2H_5$ | H | $CH_3$ | $5-CH_3$ | $CH_3$ | 1 | |
| 20.27 | $3-OCH_3$ | H | $CH_3$ | $5-CH_3$ | $CH_3$ | 1 | |
| 30.28 | $3-OC_2H_5$ | H | $CH_3$ | $5-CH_3$ | $CH_3$ | 1 | |
| 30.29 | 4-F | H | $CH_3$ | $5-CH_3$ | $CH_3$ | 1 | |
| 30.30 | 3-F | H | $CH_3$ | - | $CH_3$ | 0 | |
| 30.31 | 3-Cl | H | $CH_3$ | - | $CH_3$ | 0 | |
| 30.32 | 3-Br | H | $CH_3$ | - | $CH_3$ | 0 | |
| 30.33 | $3-CF_3$ | H | $CH_3$ | - | $CH_3$ | 0 | |
| 30.34 | $3-CH_3$ | H | $CH_3$ | - | $CH_3$ | 0 | |

Tabelle 30 (Fortsetzung)

| Verbindung Nr. | $R^1$ | $R^3$ | $R^4$ | $R^5$ | $R^6$ | n | phys. Daten (Fp. °C) |
|---|---|---|---|---|---|---|---|
| 30.35 | 3-$C_2H_5$ | H | $CH_3$ | - | $CH_3$ | 0 | |
| 30.36 | 3-$OCH_3$ | H | $CH_3$ | - | $CH_3$ | 0 | |
| 30.37 | 3-$OC_2H_5$ | H | $CH_3$ | - | $CH_3$ | 0 | |
| 30.38 | 4-F | H | $CH_3$ | - | $CH_3$ | 0 | |
| 30.39 | 3-F | 3-F | H | - | $CH_3$ | 0 | |
| 30.40 | 3-Cl | 3-F | H | - | $CH_3$ | 0 | |
| 30.41 | 3-Br | 3-F | H | - | $CH_3$ | 0 | |
| 30.42 | 3-$CF_3$ | 3-F | H | - | $CH_3$ | 0 | |
| 30.43 | 3-$CH_3$ | 3-F | H | - | $CH_3$ | 0 | |
| 30.44 | 3-$C_2H_5$ | 3-F | H | - | $CH_3$ | 0 | |
| 30.45 | 3-$OCH_3$ | 3-F | H | - | $CH_3$ | 0 | |
| 30.46 | 3-$OC_2H_5$ | 3-F | H | - | $CH_3$ | 0 | |
| 30.47 | 4-F | 3-F | H | - | $CH_3$ | 0 | |

Tabelle 31

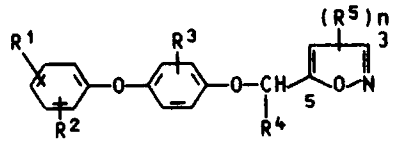

n = 1

| Verbindung Nr. | $R^1$ | $R^2$ | $R^3$ | $R^4$ | $R^5$ in 3-Stellung | physikalische Daten (Fp. °C) |
|---|---|---|---|---|---|---|
| 31.1 | H | H | H | H | $OC_2H_5$ | 54-58 |
| 31.2 | 3-F | H | H | H | $OC_2H_5$ | 42-43 |
| 31.3 | 3-Cl | H | H | H | $OC_2H_5$ | 57-58 |
| 31.4 | 3-Br | H | H | H | $OC_2H_5$ | 68-71 |
| 31.5 | $3-CF_3$ | H | H | H | $OC_2H_5$ | 51-53 |
| 31.6 | $3-OCH_3$ | H | H | H | $OC_2H_5$ | |
| 31.7 | $3-CH_3$ | H | H | H | $OC_2H_5$ | 43-44 |
| 31.8 | $3-C_2H_5$ | H | H | H | $OC_2H_5$ | 300 MHz-$^1$H-NMR in $CDCl_3$[ppm]:5,98(s) |
| 31.9 | 3-Cl | 4-F | H | H | $OC_2H_5$ | 55-56 |
| 31.10 | H | H | H | $CH_3$ | $OC_2H_5$ | 300 MHz-$^1$H-NMR in $CDCl_3$[ppm]:5,78(s) |
| 31.11 | 3-F | H | H | $CH_3$ | $OC_2H_5$ | |
| 31.12 | 3-Cl | H | H | $CH_3$ | $OC_2H_5$ | |
| 31.13 | 3-Br | H | H | $CH_3$ | $OC_2H_5$ | |
| 31.14 | $3-CF_3$ | H | H | $CH_3$ | $OC_2H_5$ | |
| 31.15 | $3-OCH_3$ | H | H | $CH_3$ | $OC_2H_5$ | |
| 31.16 | $3-CH_3$ | H | H | $CH_3$ | $OC_2H_5$ | |
| 31.17 | $3-C_2H_5$ | H | H | $CH_3$ | $OC_2H_5$ | |
| 31.18 | H | H | 3-F | H | $OC_2H_5$ | |
| 31.19 | H | H | 3-Cl | H | $OC_2H_5$ | |
| 31.20 | H | H | H | H | $OCH_3$ | 62-63 |
| 31.21 | H | H | H | H | Cl | |
| 31.22 | H | H | 3-F | $CH_3$ | $OC_2H_5$ | |
| 31.23 | H | H | 3-Cl | $CH_3$ | $OC_2H_5$ | |
| 31.24 | H | H | H | $CH_3$ | $OCH_3$ | 300 MHz-$^1$H-NMR in $CDCl_3$[ppm]:5,82(s) |
| 31.25 | H | H | H | H | $CH_3$ | 48-51 |
| 31.26 | 4-F | H | H | H | $CH_3$ | |
| 31.27 | H | H | H | H | iso-$C_3H_7$ | 200 MHz-$^1$H-NMR in $CDCl_3$ [ppm]: 1,26(6H); 3,04(1H); 5,05(2H); 6,2(1H); 6,8-7,35(9H) |

Tabelle 31 (Fortsetzung)

| Verbindung Nr. | $R^1$ | $R^2$ | $R^3$ | $R^4$ | $R^5$ in 3-Stellung | physikalische Daten (Fp. °C) |
|---|---|---|---|---|---|---|
| 31.28 | H | H | H | H | tert.-$C_4H_9$ | 200-MHz-$^1$H-NMR in $CDCl_3$ [ppm]: 1,35 (9H); 5,1(2H); 6,3(1H); 6,9-7,4(9H) |
| 31.29 | H | H | H | H | Ethyl | 300-MHz-$^1$H-NMR in $CDCl_3$ [ppm]: 1,25(3H); 2,9(2H); 5,1(2H); 6,2(1H); 6,8-7,1(7H); 7,2-7,4(2H) |
| 31.30 | H | H | H | H | Phenyl | 100 |
| 31.31 | H | H | H | H | Cyclohexyl | 94-95 |
| 31.32 | H | H | H | H | iso-$C_3H_7$ | 300-MHz-$^1$H-NMR in $CDCl_3$ [ppm]: 1,28(6H); 3,03(1H); 5,05(2H); 6,2(1H); 6,8-7,1 (7H); 7,2-7,35(2H) |
| 31.33 | H | H | H | H | (2,2-Dimethylvinyl |  |
| 31.34 | 4-$CF_3$ | H | H | H | $CH_3$ |  |
| 31.35 | 4-$CF_3$ | 2-Cl | H | H | $CH_3$ | 57-62 |
| 31.36 | 4-$OCF_3$ | H | H | H | $CH_3$ |  |
| 31.37 | 4-$OCH_3$ | H | H | H | $CH_3$ |  |
| 31.38 | 4-iso-$C_3H_7$ | H | H | H | $CH_3$ |  |
| 31.39 | 4-F | 2-Cl | H | H | $CH_3$ | 60-62 |
| 31.40 | H | H | H | H | $CH_2$-O-$CH_3$ | 300-MHz-$^1$H-NMR in $CDCl_3$ [ppm]: 3,38 (s) |
| 31.41 | H | H | H | $CH_3$ | iso-$C_3H_7$ | 300-MHz-$^1$H-NMR in $CDCl_3$ [ppm]: 1,66 (3H) |
| 31.42 | H | H | 2-F | H | iso-$C_3H_7$ |  |
| 31.43 | 4-F | 3-F | 3-F | H | iso-$C_3H_7$ |  |
| 31.44 | H | H | H | H | p-Chlorphenyl |  |
| 31.45 | 4-$NO_2$ | H | H | H | (2,2-Dimethyl)vinyl |  |
| 31.46 | 4-CN | H | H | $CH_3$ | iso-$C_3H_7$ |  |

Tabelle 31 (Fortsetzung)

| Verbindung Nr. | $R^1$ | $R^2$ | $R^3$ | $R^4$ | $R^5$ in 3-Stellung | physikalische Daten (Fp. °C) |
|---|---|---|---|---|---|---|
| 31.47 | H | H | H | H | $CF_3$ | 40-42 |
| 31.48 | 4-F | 3-Cl | H | H | $CH_3$ | 86-87 |
| 31.49 | 4-Cl | 3-Cl | H | H | $CH_3$ | |
| 31.50 | 4-F | 3-F | H | H | $CH_3$ | 87-89 |
| 31.51 | H | H | 3-F | H | $CH_3$ | 300-MHz-$^1$H-NMR in $CDCl_3$ [ppm]: 2,34 (3H) |
| 31.52 | H | H | 3-Cl | H | $CH_3$ | |
| 31.53 | H | 3-F | H | H | $CH_3$ | 71-72 |
| 31.54 | H | 3-Cl | H | H | $CH_3$ | |
| 31.55 | H | 3-Br | H | H | $CH_3$ | 76-77 |
| 31.56 | H | 3-$OCH_3$ | H | H | $CH_3$ | 59-61 |
| 31.57 | H | 3-$C_2H_5$ | H | H | $CH_3$ | 68-70 |
| 31.58 | H | 3-$CF_3$ | H | H | $CH_3$ | 76-77 |
| 31.59 | H | 3-F | 3-Cl | H | $CH_3$ | |
| 31.60 | H | 3-Cl | 3-Cl | H | $CH_3$ | 87-90 |
| 31.61 | H | 3-Br | 3-Cl | H | $CH_3$ | 90-92 |
| 31.62 | H | 3-$OCH_3$ | 3-Cl | H | $CH_3$ | |
| 31.63 | H | 3-$C_2H_5$ | 3-Cl | H | $CH_3$ | |
| 31.64 | H | 3-$CF_3$ | 3-Cl | H | $CH_3$ | |
| 31.65 | H | 3-Cl | 3-Cl | H | $CH_3$ | |
| 31.66 | H | 3-F | 3-F | H | $CH_3$ | |
| 31.67 | H | 3-Cl | 3-F | H | $CH_3$ | 300-MHz-$^1$H-NMR in $CDCl_3$ [ppm]: 2,32 (3H) |
| 31.68 | H | 3-Br | 3-F | H | $CH_3$ | |
| 31.69 | H | 3-$OCH_3$ | 3-F | H | $CH_3$ | |
| 31.70 | H | 3-$C_2H_5$ | 3-F | H | $CH_3$ | |
| 31.71 | H | 3-$CF_3$ | 3-F | H | $CH_3$ | |
| 31.72 | 4-F | 3-Cl | 3-F | H | $CH_3$ | |
| 31.73 | 4-F | 3-Cl | H | $CH_3$ | $CH_3$ | |
| 31.74 | H | H | H | H | $CH_3$ | |
| 31.75 | H | H | 3-F | $CH_3$ | $CH_3$ | |
| 31.76 | H | 3-F | H | $CH_3$ | $CH_3$ | |
| 31.77 | H | H | H | H | Cyclopropyl | 85-88 |
| 31.78 | 4-F | H | H | H | Cyclopropyl | |
| 31.79 | 4-F | 3-Cl | H | H | Cyclopropyl | 71-72 |
| 31.80 | 4-Cl | 3-Cl | H | H | Cyclopropyl | |
| 31.81 | H | 3-F | H | H | Cyclopropyl | 73-74 |

Tabelle 31 (Fortsetzung)

| Verbindung Nr. | $R^1$ | $R^2$ | $R^3$ | $R^4$ | $R^5$ in 3-Stellung | physikalische Daten (Fp. °C) |
|---|---|---|---|---|---|---|
| 31.82 | H | 3-Cl | H | H | Cyclopropyl | 72-73 |
| 31.83 | H | 3-Br | H | H | Cyclopropyl | 41-42 |
| 31.84 | H | 3-$OCH_3$ | H | H | Cyclopropyl | 42-44 |
| 31.85 | H | 3-$C_2H_5$ | H | H | Cyclopropyl | 41-43 |
| 31.86 | H | 3-$CF_3$ | H | H | Cyclopropyl | 96-97 |
| 31.87 | H | H | 3-F | H | Cyclopropyl | 64-66 |
| 31.88 | 4-F | 3-F | H | H | Cyclopropyl | |
| 31.89 | H | H | 3-Cl | H | Cyclopropyl | |
| 31.90 | H | 3-F | 3-F | H | Cyclopropyl | |
| 31.91 | F | 3-Cl | 3-F | H | Cyclopropyl | |
| 31.92 | H | 3-$CH_3$ | 3-F | H | Cyclopropyl | |
| 31.93 | H | 3-$OCH_3$ | 3-F | H | Cyclopropyl | |
| 31.94 | H | 3-$C_2H_5$ | 3-F | H | Cyclopropyl | |
| 31.95 | H | 3-$CF_3$ | 3-F | H | Cyclopropyl | 300 MHz-$^1$H-NMR in $CDCl_3$ [ppm]: 5,38 (2H) |
| 31.96 | 4-F | 3-Cl | 3-F | H | Cyclopropyl | |
| 31.97 | H | H | H | $CH_3$ | Cyclopropyl | |
| 31.98 | 4-F | H | H | $CH_3$ | Cyclopropyl | |
| 31.99 | H | 3-F | H | $CH_3$ | Cyclopropyl | |
| 31.100 | H | 3-Cl | H | $CH_3$ | Cyclopropyl | |
| 31.101 | H | 3-Br | H | $CH_3$ | Cyclopropyl | |
| 31.102 | H | 3-$OCH_3$ | H | $CH_3$ | Cyclopropyl | |
| 31.103 | H | 3-$C_2H_5$ | H | $CH_3$ | Cyclopropyl | |
| 31.104 | H | 3-$CF_3$ | H | $CH_3$ | Cyclopropyl | |
| 31.105 | 4-Cl | 3-Cl | H | $CH_3$ | Cyclopropyl | |
| 31.106 | H | 3-$CH_3$ | H | H | Cyclopropyl | 58-59 |
| 31.107 | H | 3-$CH_3$ | 3-F | H | Cyclopropyl | |
| 31.108 | H | 3-$CH_3$ | H | H | $CH_3$ | 77-78 |
| 31.109 | H | 3-tert.-$C_4H_9$ | H | H | $CH_3$ | 89-90 |
| 31.110 | 4-Cl | H | H | H | Cyclopropyl | |
| 31.111 | 4-Br | H | H | H | Cyclopropyl | |
| 31.112 | 4-$CH_3$ | H | H | H | Cyclopropyl | |
| 31.113 | 4-$OCH_3$ | H | H | H | Cyclopropyl | |
| 31.114 | 4-$C_2H_5$ | H | H | H | Cyclopropyl | |
| 31.115 | 2-F | H | H | H | Cyclopropyl | |
| 31.116 | 2-Cl | H | H | H | Cyclopropyl | |

Tabelle 31 (Fortsetzung)

| Verbindung Nr. | $R^1$ | $R^2$ | $R^3$ | $R^4$ | $R^5$ in 3-Stellung | physikalische Daten (Fp. °C) |
|---|---|---|---|---|---|---|
| 31.117 | 2-Br | H | H | H | Cyclopropyl | |
| 31.118 | 2-$CH_2$ | H | H | H | Cyclopropyl | |
| 31.119 | 3-Cl | 2-Cl | H | H | Cyclopropyl | |
| 31.120 | 3-Cl | 5-Cl | H | H | Cyclopropyl | 300-MHz-$^1$H-NMR in $CDCl_3$ [ppm]: 5,11 (s) |
| 31.121 | H | H | H | H | Cyclobutyl | 300-MHz-$^1$H-NMR in $CDCl_3$ [ppm]: 5,11 (s) |
| 31.122 | 3-Br | H | H | H | Cyclobutyl | 53-56 |

Tabelle 32

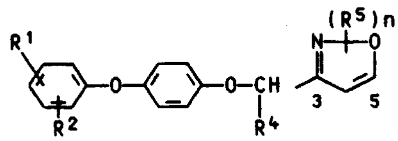

| Verbindung Nr. | $R^1$ | $R^2$ | $R^4$ | $R^5$ | n | phys. Daten (Fp. °C) |
|---|---|---|---|---|---|---|
| 32.1 | H | H | H | 5-$OC_2H_5$ | 1 | |
| 32.2 | 3-F | H | H | 5-$OC_2H_5$ | 1 | |
| 32.3 | 3-Cl | H | H | 5-$OC_2H_5$ | 1 | |
| 32.4 | 3-Br | H | H | 5-$OC_2H_5$ | 1 | |
| 32.5 | 3-$OCH_3$ | H | H | 5-$OC_2H_5$ | 1 | |
| 32.6 | 3-$CF_3$ | H | H | 5-$OC_2H_5$ | 1 | |
| 32.7 | 3-$CH_3$ | H | H | 5-$OC_2H_5$ | 1 | |
| 32.8 | 3-$C_2H_5$ | H | H | 5-$OC_2H_5$ | 1 | |
| 32.9 | 3-Cl | 4-F | H | 5-$OC_2H_5$ | 1 | |
| 32.10 | H | H | $CH_3$ | 5-$OC_2H_5$ | 1 | |
| 32.11 | 3-F | H | $CH_3$ | 5-$OC_2H_5$ | 1 | |
| 32.12 | 3-$CH_3$ | H | $CH_3$ | 5-$OC_2H_5$ | 1 | |
| 32.13 | 3-$OCH_3$ | H | $CH_3$ | 5-$OC_2H_5$ | 1 | |
| 32.14 | 3-$CF_3$ | H | $CH_3$ | 5-$OC_2H_5$ | 1 | |
| 32.15 | H | H | H | 5-Cl | 1 | |
| 32.16 | H | H | $CH_3$ | 5-Cl | 1 | |
| 32.17 | H | H | H | 5-Cyclopropyl | 1 | 80-84 |
| 32.18 | 3-F | H | H | 5-Cyclopropyl | 1 | 300 MHz-$^1$H-NMR in $CDCl_3$ [ppm]: 5,06 (s) |
| 32.19 | 3-Cl | H | H | 5-Cyclopropyl | 1 | 48-52 |
| 32.20 | 3-Br | H | H | 5-Cyclopropyl | 1 | |
| 32.21 | 3-$CH_3$ | H | H | 5-Cyclopropyl | 1 | |
| 32.22 | 3-$C_2H_5$ | H | H | 5-Cyclopropyl | 1 | |
| 32.23 | 3-$OCH_3$ | H | H | 5-Cyclopropyl | 1 | |
| 32.24 | 3-$CF_3$ | H | H | 5-Cyclopropyl | 1 | |
| 32.25 | 4-F | H | H | 5-Cyclopropyl | 1 | |
| 32.26 | 4-Cl | H | H | 5-Cyclopropyl | 1 | |
| 32.27 | 4-Br | H | H | 5-Cyclopropyl | 1 | |
| 32.28 | 4-$CH_3$ | H | H | 5-Cyclopropyl | 1 | |
| 32.29 | 4-$C_2H_5$ | H | H | 5-Cyclopropyl | 1 | |
| 32.30 | 4-$OCH_3$ | H | H | 5-Cyclopropyl | 1 | |
| 32.31 | 4-Cl | 3-Cl | H | 5-Cyclopropyl | 1 | |
| 32.32 | 4-Cl | 2-Cl | H | 5-Cyclopropyl | 1 | |

Tabelle 33

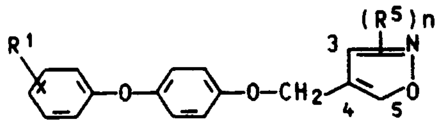

| Verbindung Nr. | $R^1$ | $R^5$ | n | phys. Daten (Fp. °C) |
|---|---|---|---|---|
| 33.1 | H | 3-$OC_2H_5$ | 1 | |
| 33.2 | 3-F | 3-$OC_2H_5$ | 1 | |
| 33.3 | H | 5-$OC_2H_5$ | 1 | |
| 33.4 | 3-F | 5-$OC_2H_5$ | 1 | |
| 33.5 | H | 3-iso-$C_3H_7$, 5-$CH_3$ | 2 | 78-82 |
| 33.6 | H | 3-$CH_3$, 5-Br | | |

Anwendungsbeispiele

Die Konzentrationen, bei denen die untersuchten Verbindungen eine 100 %ige Mortalität bzw. Hemmung bewirken, sind die jeweiligen Minimalkonzentrationen. Bei jeder Konzentration wurden mindestens 2 Versuche angesetzt.

Beispiel A

Dysdercus intermedius (Baumwollwanze), Zuchtversuch

Petrischalen von 10 cm Durchmesser werden mit 1 ml der acetonischen Wirkstofflösung ausgekleidet. Nach Verdunsten des Lösungsmittels besetzt man die Schalen mit je 20 Larven des vorletzten Stadiums. Nach 24 Stunden überführt man die überlebenden Tiere in 1 l-Gläser, die 200 g sterilen Quarzsand (Korngröße 0 bis 3 mm) enthalten. Dieser Sand wurde vor Versuchsbeginn mit 25 ml der wäßrigen Wirkstoffaufbereitung angegossen. Als Futter bietet man gequollene Baumwollsamen, die wöchentlich gewechselt werden. Ebenfalls wöchentlich feuchtet man den Sand mit reinem Wasser an.

Die Versuchstemperatur beträgt 25 bis 27 ° C. Die Beobachtungszeit erstreckt sich bis zum Schlüpfen der Eier in den Kontrollen.

In diesem Versuch lag die mortale Dosis der Verbindung Nr. 31.25 bei 10 ppm.

Beispiel B

Ovizide Wirkung bei Dysdercus intermedius (Baumwollwanze)

Stecketiketten mit einem ca. 8 mm breiten Klebestreifen werden 24 Stunden vor dem Versuchsbeginn in ein mit Baumwollwanzen-Eiern gefülltes Sammelgefäß eingetaucht. Die bei diesem Vorgang auf der Steckkarte haften gebliebenen Eier werden für ca. 5 Sekunden in die wäßrige Wirkstoffaufbereitung eingetaucht und auf einem Filterpapier abtropfen lassen, ohne daß die Eier das Filterpapier berühren. Anschließend werden die Stecketiketten mit dem Klebstreifen nach oben in Plastikpaletten gestellt, die eine mit Wasser durchfeuchtete halbe Watterolle enthalten, und mit einer Glasplatte abgedeckt. Nach dem Schlupf der Larven im Kontrollversuch (nach ca. 8 Tagen) wird bonitiert.

In diesem Versuch lag die mortale Dosis der Verbindung Nr. 31.25 bei 0,002 %.

Beispiel C

Wirkung auf die Eiablage von Epilachna varivestis (Mexikanischer Bohnenkäfer)

Gruppen von je 20 Eiern des Mexikanischen Bohnenkäfers werden mit ihrer Blattunterlage für 3 Sekunden in die wäßrige Wirkstoffaufbereitung getaucht. Anschließend plaziert man sie auf einem angefeuchteten Filterpapier in Kunststoff-Käfige. Registriert wird der Schlupf lebensfähiger Larven, was an der Annahme des gebotenen Futters zu erkennen ist.

In diesem Versuch lag die mortale Dosis der Verbindung Nr. 31.25 bei 0,02 ppm.

Beispiel D

Entwicklungshemmung von Heliothis virescens auf behandeltem Nährboden

100 g des Standard-Nährbodens für Heliothis werden in 250 ml-Becher gefüllt und warm mit der wäßrigen Wirkstoffaufbereitung sorgfältig gemischt. Nach Erkalten belegt man jedes Gefäß mit 10 Raupen im dritten Larvenstadium (1,5 bis 1,8 cm) und lagert sie bei 23 ° C. Beurteilt wird Verpuppung und Schlupf der Falter.

Bei diesem Versuch lag die mortale Dosis der Verbindung Nr. 31.25 bei 1 ppm.

## Patentansprüche

**1.** (p-Phenoxy-phenoxy)-methyl-fünfringheteroaromaten der allgemeinen Formel I

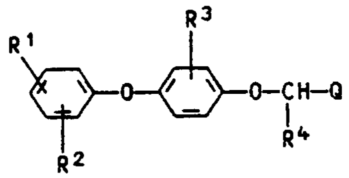

(I),

in der die Substituenten folgende Bedeutung haben:

| | |
|---|---|
| $R^1$, $R^2$, $R^3$ | Wasserstoff, Halogen, $C_1$-$C_8$-Alkyl, $C_1$-$C_8$-Alkoxy, $C_1$-$C_4$-Halogenalkyl, $C_1$-$C_4$-Halogenalkoxy, $C_3$-$C_{10}$-Cycloalkyl, Nitro oder Cyano, |
| $R^4$ | Wasserstoff oder $C_1$-$C_4$-Alkyl, |
| Q | ein Fünfringhetarylrest der Formeln II.1 bis II.33 |

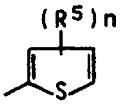 (II.1)

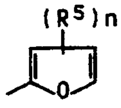 (II.2)

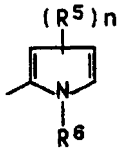 (II.3)

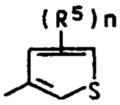 (II.4)

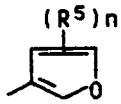 (II.5)

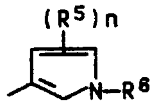 (II.6)

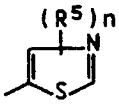 (II.7)

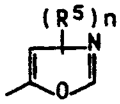 (II.8)

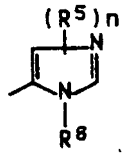 (II.9)

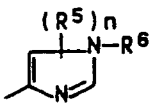 (II.10)

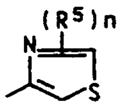 (II.11)

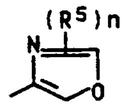 (II.12)

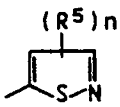 (II.13)

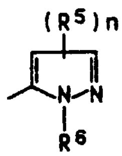 (II.14)

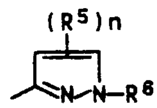 (II.15)

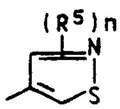 (II.16)

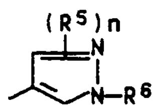 (II.17)

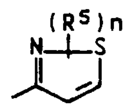 (II.18)

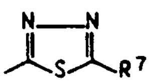 (II.19)

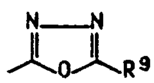 (II.20)

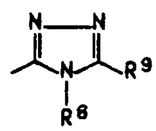 (II.21)

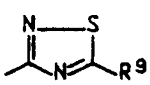 (II.22)

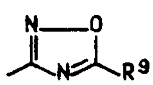 (II.23)

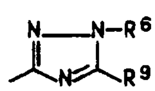 (II.24)

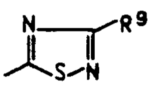 (II.25)

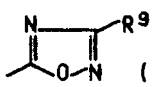 (II.26)

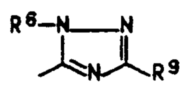 (II.27)

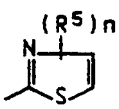 (II.28)

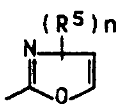 (II.29)

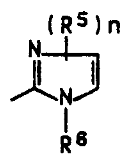 (II.30)

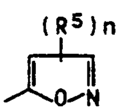 (II.31)

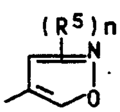 (II.32)

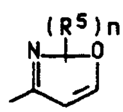 (II.33)

in denen

$R^5$ Halogen, $C_1$-$C_8$-Alkyl, $C_2$-$C_8$-Alkenyl, $C_1$-$C_4$-Halogenalkyl, $C_1$-$C_8$-Alkoxy, $C_2$-$C_8$-Alkoxyalkyl, $C_3$-$C_{10}$-Cycloalkyl, Aryl, $C_7$-$C_{20}$-Arylalkyl oder im Arylteil ein- bis dreifach durch Halogen, $C_1$-$C_8$-Alkyl, $C_1$-$C_8$-Alkoxy, $C_1$-$C_4$-Halogenalkyl oder $C_1$-$C_4$-Halogenalkoxy oder Cyano oder ein- bis zweifach durch Nitro substituiertes Aryl oder $C_7$-$C_{20}$-Arylalkyl,

$R^6$ Wasserstoff, $C_1$-$C_8$-Alkyl, $C_3$-$C_{10}$-Cycloalkyl oder Aryl, welches gegebenenfalls ein- bis dreifach durch Halogen, $C_1$-$C_6$-Alkyl, $C_1$-$C_8$-Alkoxy, $C_1$-$C_4$-Halogenalkyl oder $C_1$-$C_3$-Halogenalkoxy substituiert ist,

$R^7$ Halogen oder $C_3$-$C_{10}$-Cycloalkyl,

$R^9$ Wasserstoff oder $R^5$ und

n 0, 1, gegebenenfalls 2 oder 3

bedeuten, mit der Maßgabe, daß $R^4$ nicht für Methyl steht, wenn Q der Fünfringhetarylrest II.23 oder II.26 ist.

**2.** Verfahren zur Herstellung der (p-Phenoxy-phenoxy)-methyl-fünfringheteroaromaten der allgemeinen Formel I gemäß Anspruch 1, dadurch gekennzeichnet, daß man in an sich bekannter Weise

a) (p-Phenoxy)-phenole III

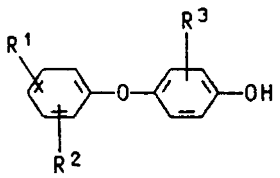

(III)

mit einem Fünfringhetarylderivat der Formel IV

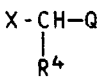

(IV),

in der X eine Abgangsgruppe bedeutet, in Gegenwart einer Base oder

b) ein Phenolatanion von III

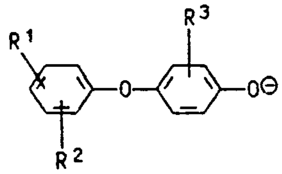

mit einem Fünfringhetarylderivat IV umsetzt.

**3.** Schädlingsbekämpfungsmittel, enthaltend einen (p-Phenoxy-phenoxy)-methyl-fünfringheteroaromaten gemäß Anspruch 1 neben hierfür üblichen Trägerstoffen.

**4.** Schädlingsbekämpfungsmittel nach Anspruch 3, enthaltend 0,1 bis 95 Gew.% eines (p-Phenoxy-phenoxy)-methyl-fünfringheteroaromaten der Formel I.

**5.** Verfahren zur Bekämpfung von Schädlingen, dadurch gekennzeichnet, daß man die Schädlinge und/oder die von Schädlingen freizuhaltenden Flächen und/oder Räume mit einer für Schädlinge wirksamen Menge eines (p-Phenoxy-phenoxy)-methyl-fünfringheteroaromaten der Formel I gemäß Anspruch 1 behandelt.

## Claims

**1.** A (p-phenoxyphenoxy)methyl-five-membered hetaryl compound of the formula I

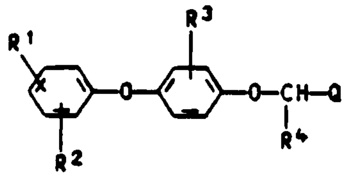

(I)

where

$R^1$, $R^2$ and $R^3$ are each hydrogen, halogen, $C_1$-$C_8$-alkyl, $C_1$-$C_8$-alkoxy, $C_1$-$C_4$-haloalkyl, $C_1$-$C_4$-haloalkoxy, $C_3$-$C_{10}$-cycloalkyl, nitro or cyano,

$R^4$ is hydrogen or $C_1$-$C_4$-alkyl,

Q is a five-membered hetaryl radical of the formulae II.1 to II.33

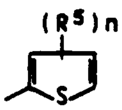 (II.1)

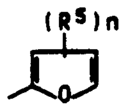 (II.2)

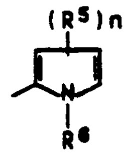 (II.3)

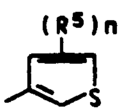 (II.4)

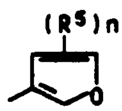 (II.5)

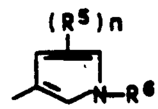 (II.6)

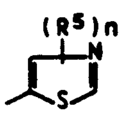 (II.7)

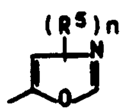 (II.8)

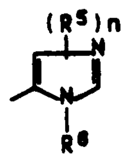 (II.9)

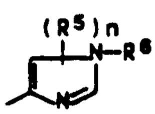 (II.10)

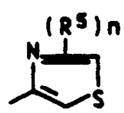 (II.11)

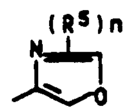 (II.12)

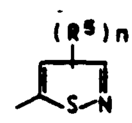 (II.13) 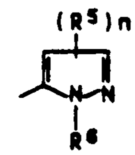 (II.14) 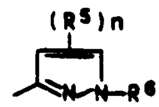 (II.15)

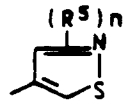 (II.16) 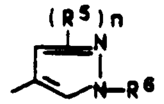 (II.17) 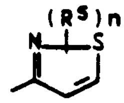 (II.18)

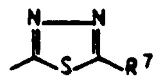 (II.19) 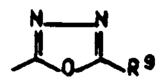 (II.20) 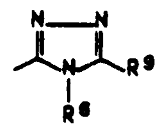 (II.21)

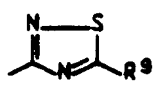 (II.22) 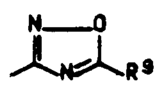 (II.23) 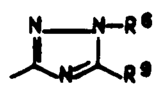 (II.24)

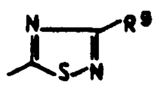 (II.25) 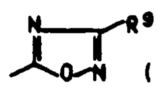 (II.26) 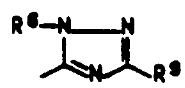 (II.27)

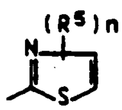 (II.28) 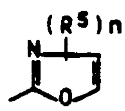 (II.29) 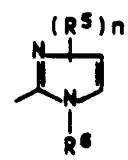 (II.30)

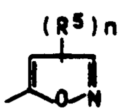 (II.31) 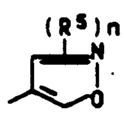 (II.32) 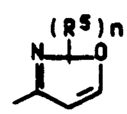 (II.33)

where
$R^5$ is halogen, $C_1$-$C_8$-alkyl, $C_2$-$C_8$-alkenyl, $C_1$-$C_4$-haloalkyl, $C_1$-$C_8$-alkoxy, $C_2$-$C_8$-alkoxyalkyl, $C_3$-$C_{10}$-cycloalkyl, aryl or $C_7$-$C_{20}$-arylalkyl, the aryl and $C_7$-$C_{20}$-arylalkyl being unsubstituted or monosubstituted, disubstituted or trisubstituted in the aryl moiety by halogen, $C_1$-$C_8$-alkyl, $C_1$-$C_8$-alkoxy, $C_1$-$C_4$-haloalkyl or $C_1$-$C_4$-haloalkoxy or cyano or monsubstituted or disubstituted by nitro,
$R^6$ is hydrogen, $C_1$-$C_8$-alkyl, $C_3$-$C_{10}$-cycloalkyl or aryl, which is unsubstituted, monosubstituted, disubstituted or trisubstituted by halogen, $C_1$-$C_6$-alkyl, $C_1$-$C_8$-alkoxy, $C_1$-$C_4$-haloalkyl or $C_1$-$C_3$-haloalkoxy,
$R^7$ is halogen or $C_3$-$C_{10}$-cycloalkyl,
$R^9$ is hydrogen or $R^5$, and
n is 0, 1, or, where relevant, 2 or 3,
with the proviso that $R^4$ is not methyl if Q is the five-membered hetaryl radical II.23 or II.26.

**2.** A process for preparing a (p-phenoxyphenoxy)-methyl-five-membered hetaryl compound of the formula I as claimed in claim 1, which comprises reacting, in a conventional manner,
a) a (p-phenoxy)phenol III

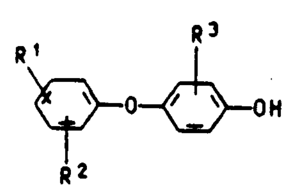 (III)

with a five-membered hetaryl derivative of the formula IV

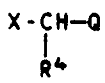

(IV),

where X is a leaving group, in the presence of a base or
b) a phenolate anion of III

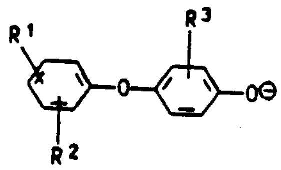

with a five-membered hetaryl derivative IV.

3. A pesticide containing a (p-phenoxyphenoxy)methyl-five-membered hetaryl compound as claimed in claim 1 together with conventional carriers therefor.

4. A pesticide as claimed in claim 3, containing from 0.1 to 95 % by weight of a (p-phenoxyphenoxy)-methyl-five-membered hetaryl compound of the formula I.

5. A method for controlling pests, wherein the pests, or the areas or spaces to be kept free from pests are treated with a pesticidally effective amount of a (p-phenoxyphenoxy)methyl-five-membered hetaryl compound of the formula I as claimed in claim 1.

**Revendications**

1. Composé (p-phénoxy-phénoxyl)-méthyl-hétéro-aromatiques à noyau pentagonal de la formule générale I

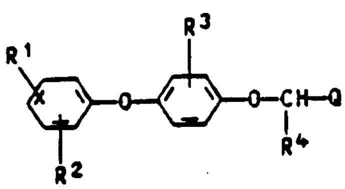

(I),

dans laquelle les substituants ont les significations suivantes :
$R^1$, $R^2$, $R^3$, hydrogène, halogène, alkyle en C 1-C 8, alcoxy en C 1-C 8, halogénalkyle en C 1-C 4, halogénalcoxy en C 1-C 4, cycloalkyle en C 3-C 10, nitro ou cyano
$R^4$, hydrogène ou alkyle en C 1-C 4,
Q, un reste hétaryle à noyau pentagonal des formules II.1 à II.33

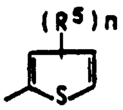 (II.1)

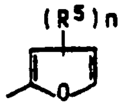 (II.2)

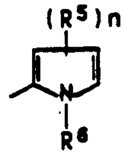 (II.3)

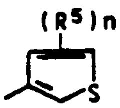 (II.4)

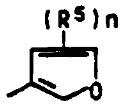 (II.5)

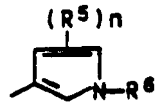 (II.6)

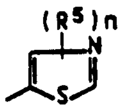 (II.7)

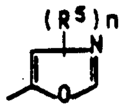 (II.8)

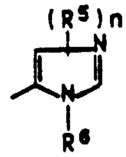 (II.9)

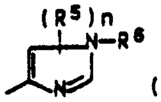 (II.10) 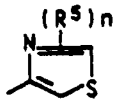 (II.11) 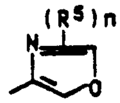 (II.12)

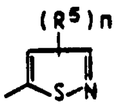 (II.13) 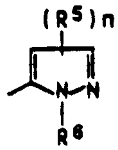 (II.14) 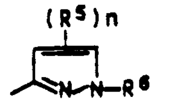 (II.15)

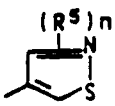 (II.16) 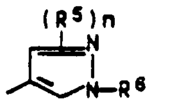 (II.17) 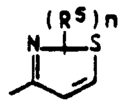 (II.18)

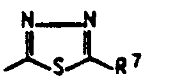 (II.19) 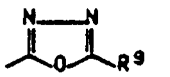 (II.20) 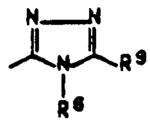 (II.21)

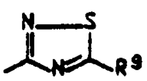 (II.22) 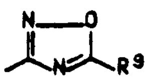 (II.23) 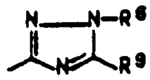 (II.24)

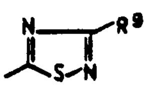 (II.25) 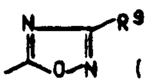 (II.26) 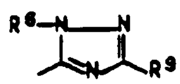 (II.27)

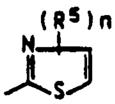 (II.28) 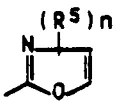 (II.29) 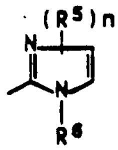 (II.30)

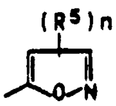 (II.31) 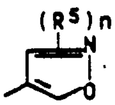 (II.32) 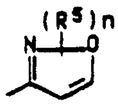 (II.33)

dans lequel

$R^5$ signifie halogène, alkyle en C 1-C 8, alcényle en C 2-C 8, halogénalkyle en C 1-C 4, alcoxy en C 1-C 8, alcoxyalkyle en C 2-C 8, cycloalkyle en C 3-C 10, aryle, arylalkyle en C 7-C 20 ou aryle substitué dans la partie aryle, une à trois fois par halogène, alkyle en C 1-C 8, alcoxy en C 1-C 8 halogène-alkyle en C 1-C 4 ou halogenalcoxy en C 1-C 4 ou cyano ou une à deux fois par nitro, ou arylalkyle en C 7-C 20,

$R^6$, hydrogène, alkyle en C 1-C 8, cycloalkyle en C 3-C 10 ou aryle qui est éventuellement substitué une à trois fois par halogène, alkyle en C 1-C 6, alcoxy en C 1-C 8, halogenalkyle en C 1-C 4 ou halogenalcoxy en C 1-C 3

$R^7$, halogène ou cycloalkyle en C 3-C 10

$R^9$, hydrogène ou $R^5$ et

n, 0,1, eventuellement 2 ou 3

sous réserve que $R^4$ n'est pas mis pour méthyle lorsque Q est le reste hétaryle à noyau pentagonal II.23 ou II.26.

2. Procédé de préparation de composé (p-phénoxy-phénoxyl)-méthyl-hétéro-aromatique à noyau pentagonal de la formule générale I, caractérisé par le fait que l'on fait réagir, de manière comme en soi,
a) du (p-phénoxyl)-phénol III

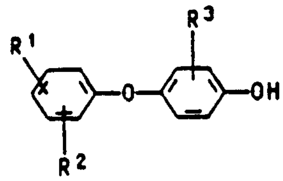

(III)

avec un dérivé d'hétaryle à noyau pentagonal de formule IV

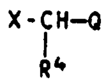

(IV),

dans laquelle X représente un groupe éliminable, en présence d'une base, ou
b) un anion phénolate de III

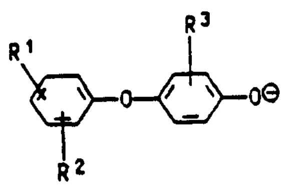

avec un dérivé d'hétaryle à noyau pentagonal IV.

3. Pesticide, contenant un composé (p-phénoxy-phénoxyl)-méthyl-hétéro-aromatique à noyau pentagonal de la formule générale I avec des véhicules usuels pour cela.

4. Pesticide selon la revendication 3 contenant 0,1 à 95 % en poids d'un composé (p-phénoxy-phénoxyl)-méthyl-hétéroaromatique à noyau pentagonal de la formule générale I.

5. Procédé de lutte contre les parasites, caractérisé par le fait que l'on traite les parasites et/ou les surfaces et/ou l'espace à maintenir libre de parasites, avec une quantité efficace contre les parasites d'un composé (p-phénoxy-phénoxyl)-méthyl-hétéro-aromatique à noyau pentagonal de la formule générale I.